# EUROPÄISCHE PATENTSCHRIFT

(11) **EP 2 432 851 B1**
(45) Veröffentlichungstag und Bekanntmachung des Hinweises auf die Patenterteilung: **02.10.2013**
(21) Anmeldenummer: 10713863.8
(22) Anmeldetag: 19.04.2010
(51) Int. Cl.: C09K 19/32, C09K 19/34, C09K 19/04, C09K 19/54

(54) **FLÜSSIGKRISTALLANZEIGE**
LIQUID CRYSTAL DISPLAY
DISPOSITIF D'AFFICHAGE À BASE DE CRISTAUX LIQUIDES

(30) Priorität: 22.05.2009 DE 102009022309
(43) Veröffentlichungstag der Anmeldung: 28.03.2012
(73) Patentinhaber: Merck Patent GmbH, 64293 Darmstadt (DE)
(72) Erfinder: TAUGERBECK, Andreas, 64285 Darmstadt (DE); HAHN, Alexander, 64584 Biebesheim (DE); GOETZ, Achim, 64665 Alsbach-Hähnlein (DE)
(86) Internationale Anmeldenummer: PCT/EP2010/002374
(87) Internationale Veröffentlichungsnummer: WO 2010/133278

(56) Entgegenhaltungen:
- EP-A1- 0 397 418
- EP-A1- 1 837 324
- EP-A1- 1 944 287
- WO-A1-2005/054406
- WO-A1-2009/030322
- WO-A1-2009/030352
- DE-A1-102006 057 250
- DE-A1-102008 036 248
- JP-A- 63 233 952
- US-A- 5 648 021
- US-A- 5 674 576
- US-A1- 2002 110 650
- US-A1- 2003 203 128
- US-A1- 2005 255 258
- PEARSON D ET AL: "Phosphoric Acid Systems, Part 7. The Halogenation of Nitration of Aryl Compounds in Trialkyl Phosphates" SYNTHESIS, GEORG THIEME VERLAG, STUTTGART, DE, 1. September 1976 (1976-09-01), Seiten 621-623, XP008122907 ISSN: 0039-7881
- CORNFORTH J.W. ET AL.: "144. Experiments on the Synthesis of Substansces related to the Sterols. The Preparation of 2:7-Dihydroxyphenanthrene and Certain Derivatives. Further Observations on the Reduction of 1-gamma-Ketobutyl-2-naphthol." JOURNAL OF THE CHEMICAL SOCIETY, 27. Juni 1942 (1942-06-27), Seiten 684-689, XP008122950 CHEMICAL SOCIETY ISSN: 0368-1769

## Beschreibung

Die vorliegende Erfindung betrifft polymerisierbare Verbindungen, Verfahren und Zwischenprodukte zu ihrer Herstellung, und ihre Verwendung für optische, elektrooptische und elektronische Zwecke, insbesondere in Flüssigkristall (FK)-Medien und FK-Anzeigen, vor allem in FK-Anzeigen des PS- (polymer stabilized) oder PSA- (polymer sustained alignment) Typs.

Die derzeit verwendeten Flüssigkristallanzeigen (FK-Anzeigen) sind meist solche des TN-Typs ("twisted nematic"). Diese weisen allerdings den Nachteil einer starken Blickwinkelabhängigkeit des Kontrastes auf. Daneben sind sogenannte VA-Anzeigen ("vertically aligned") bekannt, die einen breiteren Blickwinkel aufweisen. Die FK-Zelle einer VA-Anzeige enthält eine Schicht eines FK-Mediums zwischen zwei transparenten Elektroden, wobei das FK-Medium üblicherweise einen negativen Wert der dielektrischen (DK-) Anisotropie aufweist. Die Moleküle der FK-Schicht sind im ausgeschalteten Zustand senkrecht zu den Elektrodenflächen (homöotrop) oder gekippt homöotrop ("tilted") orientiert. Bei Anlegen einer elektrischen Spannung an die beiden Elektroden findet eine Umorientierung der FK-Moleküle parallel zu den Elektrodenflächen statt. Weiterhin sind OCB-Anzeigen ("optically compensated bend") bekannt, die auf einem Doppelbrechungseffekt beruhen und eine FK-Schicht mit einer sogenannten "bend"-Orientierung und üblicherweise positiver (DK-) Anisotropie aufweisen. Bei Anlegen einer elektrischen Spannung findet eine Umorientierung der FK-Moleküle senkrecht zu den Elektrodenflächen statt. Darüber hinaus enthalten OCB-Anzeigen normalerweise einen oder mehrere doppelbrechende optische Retardationsfilme, um unerwünschte Lichtdurchlässigkeit der "bend"-Zelle im dunklen Zustand zu vermeiden. OCB-Anzeigen besitzen gegenüber TN-Anzeigen einen weiteren Blickwinkel und kürzere Schaltzeiten. Weiterhin sind sogenannte IPS-Anzeigen ("In-Plane Switching") bekannt, die eine FK-Schicht zwischen zwei Substraten enthalten, wobei die beiden Elektroden nur auf einem der beiden Substrate angeordnet sind, und vorzugsweise ineinander verzahnte, kammförmige Strukturen aufweisen. Dadurch wird bei Anlegen einer Spannung an die Elektroden ein elektrisches Feld zwsichen ihnen erzeugt, welches eine signifikante Komponente parallel zur FK-Schicht aufweist. Dies bewirkt eine Umorientierung der FK-Moleküle in der Schichtebene. Des weiteren wurden sogenannte FFS-Anzeigen ("Fringe-Field-Switching") vorgeschlagen (siehe u.a. S.H. Jung et al., Jpn. J. Appl. Phys., Band 43, No. 3, 2004, 1028), die ebenfalls zwei Elektroden auf dem gleichen Substrat beinhalten, wovon jedoch im Gegensatz zu IPS-Anzeigen nur eine als kammförmig strukturierte Elektrode ausgebildet ist, und die andere Elektrode unstrukturiert ist. Dadurch wird ein starkes sogenanntes "fringe field" erzeugt, also ein starkes elektrisches Feld nahe am Rand der Elektroden und in der gesamten Zelle ein elektrisches Feld, welches sowohl eine starke vertikale als auch eine starke horizontale Komponente aufweist. Sowohl IPS-Anzeigen als auch FFS-Anzeigen weisen eine geringe Blickwinkelabhängigkeit des Kontrastes auf.

In VA-Anzeigen des neueren Typs ist die einheitliche Ausrichtung der FK-Moleküle auf mehrere kleinere Domänen innerhalb der FK-Zelle beschränkt. Zwischen diesen Domänen, auch als Tilt-Domänen (engl. "tilt domains") bezeichnet, können Disklinationen existieren. VA-Anzeigen mit Tilt-Domänen weisen, verglichen mit herkömmlichen VA-Anzeigen, eine größere Blickwinkelunabhängigkeit des Kontrastes und der Graustufen auf. Außerdem sind solche Anzeigen einfacher herzustellen, da eine zusätzliche Behandlung der Elektrodenoberfläche zur einheitlichen Orientierung der Moleküle im eingeschalteten Zustand, wie z.B. durch Reiben, nicht mehr notwendig ist. Stattdessen wird die Vorzugsrichtung des Kipp- oder Tiltwinkels (engl. "pretilt") durch eine spezielle Ausgestaltung der Elektroden kontrolliert. In den sogenannten MVA-Anzeigen ("multidomain vertical alignment") wird dies üblicherweise dadurch erreicht, dass die Elektroden Erhebungen oder Vorsprünge (engl. "protrusions") aufweisen, die einen lokalen pretilt verursachen. Als Folge werden die FK-Moleküle beim Anlegen einer Spannung in verschiedenen, definierten Regionen der Zelle in unterschiedliche Richtungen parallel zu den Elektrodenflächen orientiert. Dadurch wird ein "kontrolliertes" Schalten erreicht und das Entstehen störender Disklinationslinien vermieden. Diese Anordnung verbessert zwar den Blickwinkel der Anzeige, führt aber zu einer Verringerung ihrer Lichtdurchlässigkeit. Ein Weiterentwicklung von MVA verwendet Protrusions nur auf einer Elektroden-Seite, die gegenüberliegende Elektrode weist hingegen Schlitze (engl. "slits") auf, was die Lichtdurchlässigkeit verbessert. Die geschlitzten Elektroden erzeugen beim Anlegen einer Spannung ein inhomogenes elektrisches Feld in der FK-Zelle, so dass weiterhin ein kontrolliertes Schalten erreicht wird. Zur weiteren Verbesserung der Lichtdurchlässigkeit können die Abstände zwischen den slits und protrusions vergrößert werden, was jedoch wiederum zu einer Verlängerung der Schaltzeiten führt. Beim sogenannten PVA (Patterned VA) kommt man ganz ohne Protrusions aus, indem man beide Elektroden auf den gegenüberliegenden Seiten durch Schlitze strukturiert, was zu einem erhöhten Kontrast und verbesserter Lichtdurchlässigkeit führt, aber technologisch schwierig ist und das Display empfindlicher gegen mechanische Einflüsse macht (Klopfen, engl. "tapping", etc.). Für viele Anwendungen, wie beispielsweise Monitore und vor allem TV-Bildschirme, ist jedoch eine Verkürzung der Schaltzeiten sowie eine Verbesserung des Kontrastes und der Luminanz (Transmission) der Anzeige gefragt.

Eine Weiterentwicklung stellen die sogenannten PS-Anzeigen (polymer stabilized) dar, die auch unter dem Begriff "PSA" (polymer sustained alignment) bekannt sind. Darin wird dem FK-Medium eine geringe Menge (zum Beispiel 0.3 Gew.%, typischerweise <1 Gew.%) einer polymerisierbaren Verbindung zugesetzt, welche nach Einfüllen in die FK-Zelle bei angelegter elektrischer Spannung zwischen den Elektroden in situ polymerisiert bzw. vernetzt wird, üblicherweise durch UV-Photopolymerisation. Als besonders geeignet hat sich der Zusatz von polymerisierbaren mesogenen oder flüssigkristallinen Verbindungen, auch als reaktive Mesogene oder "RM"s bezeichnet, zur FK-Mischung erwiesen.

Mittlerweile wird das PSA-Prizip in diversen klassischen FK-Anzeigen angenwendet. So sind beispielsweise PSA-VA-, PSA-OCB-, PS-IPS/FFS- und PS-TN-Anzeigen bekannt. Die Polymerisation der polymerisierbaren Verbindung(en) erfolgt bei PSA-VA- und PSA-OCB-Anzeigen vorzugsweise bei angelegter elektrischer Spannung, bei PSA-IPS-Anzeigen mit oder ohne, vorzugsweise ohne, angelegte elektrische Spannung. Wie man in Testzellen nachweisen kann, führt das PSA-Verfahren zu einem pretilt in der Zelle. Bei PSA-OCB-Anzeigen kann man daher erreichen, dass die Bend-Struktur stabilisiert wird, so dass man ohne Offset-Spannung auskommt oder diese reduzieren kann. Im Falle von PSA-VA-Anzeigen wirkt sich dieser pretilt positiv auf die Schaltzeiten aus. Für PSA-VA-Anzeigen kann ein Standard-MVA- bzw. -PVA Pixel- und Elektroden-Layout verwendet werden. Darüber hinaus kann man aber beispielsweise mit nur einer strukturierten Elektrodenseite und ohne Protrusions auskommen, was die Herstellung wesentlich vereinfacht und gleichzeitig zu einem sehr guten Kontrast bei sehr guter Lichtdurchlässigkeit führt. PSA-VA-Anzeigen sind beispielsweise in JP 10-036847 A, EP 1 170 626 A2, US 6,861,107, US 7,169,449, US 2004/0191428 A1, US 2006/0066793 A1 und US 2006/0103804 A1 beschrieben. PSA-OCB-Anzeigen sind beispielsweise in T.-J- Chen et al., Jpn. J. Appl. Phys. 45, 2006, 2702-2704 und S. H. Kim, L.-C- Chien, Jpn. J. Appl. Phys. 43, 2004, 7643-7647 beschrieben. PS-IPS-Anzeigen sind zum Beispiel in US 6,177,972 und Appl. Phys. Lett. 1999, 75(21), 3264 beschrieben. PS-TN-Anzeigen sind zum Beispiel in Optics Express 2004, 12(7), 1221 beschrieben.

PSA-Anzeigen können ebenso wie die oben beschriebenen konventionallen FK-Anzeigen als Aktivmatrix- oder Passivmatrix-Anzeigen betrieben werden. Bei Aktivmatrix-Anzeigen erfolgt die Ansteuerung einzelner Bildpunkte üblicherweise durch integrierte, nicht-lineare aktive Elemente wie beispielsweise Transistoren (z.B. Dünnfilmtransistoren, engl. "thin film transistor" bzw. "TFT"), bei Passivmatrix-Anzeigen üblicherweise nach dem Multiplex-Verfahren, wie aus dem Stand der Technik bekannt.

Insbesondere für Monitor- und vor allem TV-Anwendungen ist nach wie vor die Optimierung der Schaltzeiten, wie aber auch des Kontrastes und der Luminanz (also auch Transmission) der FK-Anzeige gefragt. Hier kann das PSA-Verfahren entscheidende Vorteile bringen. Insbesondere beim PSA-VA kann man ohne nennenswerte Einbußen sonstiger Parameter eine Verkürzung der Schaltzeiten erreichen, die mit einem in Testzellen messbaren pretilt korrelieren.

Im Stand der Technik werden beispielsweise polymerisierbare Verbindungen der Formel **1** verwendet 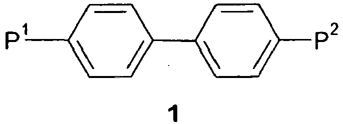 worin P eine polymerisierbare Gruppe, üblicherweise eine Acrylat- oder Methacrylategruppe bedeutet, wie beispielsweise in US 7,169,449 beschrieben.

Es ergibt sich jedoch das Problem, dass nicht alle Kombinationen bestehend aus FK-Mischung (nachfolgend auch als "FK-Hostmischung" bezeichnet) + polymerisierbarer Komponente (typischerweise RMs) für PSA-Anzeigen geeignet sind, weil sich zum Beispiel kein oder kein ausreichender Tilt einstellt, oder weil zum Beispiel die sogenannte "Voltage Holding Ratio" (VHR oder HR) für TFT-Disptayanwendungen unzureichend ist. Zudem hat sich gezeigt, dass bei Verwendung in PSA-Anzeigen die aus dem Stand der Technik bekannten FK-Mischungen und RMs noch einige Nachteile aufweisen. So eignet sich nicht jedes bekannte, in FK-Mischungen lösliche RM zur Verwendung in PSA-Anzeigen. Ausserdem ist es oft schwierig, neben der direkten Messung des pretilts in der PSA-Anzeige ein geeignetes Auswahlkriterium für das RM zu finden. Noch kleiner wird die Auswahl geeigneter RMs, wenn eine Polymerisation mittels UV-Licht ohne den Zusatz von Photoinitiatoren gewünscht ist, was für bestimmte Anwendungen von Vorteil sein kann.

Darüber hinaus sollte die gewählte Kombination FK-Hostmischung/RM eine möglichst geringe Rotationsviskosität sowie möglichst gute elektrische Eigenschaften aufweisen, insbesondere sollte sie eine möglichst hohe VHR besitzen. Bei PSA-Anzeigen ist vor allem eine hohe VHR nach Bestrahlung mit UV-Licht erforderlich, da die UV-Belichtung ein notwendiger Teil des Herstellungsprozesses der Anzeige ist, aber auch als normale Belastung im Betrieb der fertigen Anzeige auftritt.

Insbesondere wäre es wünschenswert, neue Materialien für PSA-Anzeigen zur Verfügung zu haben, die einen besonders kleinen pretilt-Winkel erzeugen. Hierbei sind Materialien bevorzugt, die während der Polymerisation bei gleicher Belichtungszeit einen niedrigeren pretilt-Winkel erzeugen als die bisher bekannten Materialien, und/oder durch deren Verwendung der mit den bekannten Materialien erzielbare (höhere) pretilt-Winkel bereits nach kürzerer Belichtungszeit erreicht werden kann. Dadurch könnten die Produktionszeit (engl. "tact time") der Anzeige verkürzt und die Kosten des Produktionsprozesses verringert werden.

Ein weiteres Problem bei der Herstellung von PSA-Anzeigen ist das Vorhandensein bzw. die Entfernung von Restmengen unpolymerisierter RMs insbesondere nach dem Polymerisationsschritt zur Erzeugung des pretilt-Winkels in der Anzeige. Beispielsweise können solche nicht abreagierten RMs die Eigenschaften der Anzeige nachteilig beeinflussen, indem sie z.B. nach Fertigstellung der Anzeige während des Betriebes unkontrolliert polymerisieren.

So zeigen die aus dem Stand der Technik bekannten PSA-Anzeigen oft den unerwünschten Effekt des sogenannten "image sticking" oder "image burn", d.h. dass das in der FK-Anzeige durch vorübergehende Ansteuerung einzelner Bildpunkte (pixel) erzeugte Bild auch nach Abschalten des elektrischen Feldes in diesen Bildpunkten, oder nach Ansteuerung anderer Bildpunkte, noch sichtbar bleibt.

Dieses "image sticking" kann einerseits auftreten, wenn FK-Hostmischungen mit einem geringen VHR verwendet wird. In diesen kann die UV-Komponente des Tageslichtes oder der Hintergrundbeleuchtung unerwünschte Zerfallsreaktionen der FK-Moleküle und dadurch die Erzeugung von ionischen oder radikalischen Verunreinigungen auslösen. Diese können sich insbesondere an den Elektroden bzw. den Orientierungsschichten anreichern und dort die effektive angelegte Spannung reduzieren. Dieser Effekt ist auch bei herkömmlichen FK-Anzeigen ohne Polymerkomponente zu beobachten.

In PSA-Anzeigen wird darüber hinaus oft ein zusätzlicher "image sticking"-Effekt beobachtet, der durch die Gegenwart von unpolymerisierten RMs hervorgerufen wird. Dabei wird durch UV-Licht aus der Umgebung oder von der Hintergrundbeleuchtung eine unkontrollierte Polymerisation der Rest-RMs ausgelöst. In den geschalteten Displaybereichen wird dadurch nach mehreren Ansteuerungszyklen der Tiltwinkel verändert. Als Resultat kann eine Transmissionsänderung in den geschalteten Bereichen auftreten, während sie in den ungeschalteten Bereichen unverändert bleibt.

Es ist deshalb wünschenswert, dass die Polymerisation der RMs bei der Herstellung der PSA-Anzeige möglichst vollständig abläuft und die Anwesenheit von unpolymerisierten RMs in der Anzeige möglichst ausgeschlossen oder auf ein Minimum reduziert wird. Hierzu werden Materialien benötigt, die eine möglichst effektive und vollständige Polymerisation ermöglichen. Zudem wäre ein kontrolliertes Abreagieren dieser Restmengen wünschenswert. Dies wäre einfacher, wenn das RM schneller und effektiver polymerisiert als die bisher bekannten Materialien.

Es besteht somit immer noch ein großer Bedarf an PSA-Anzeigen, insbesondere vom VA- und OCB-Typ, sowie FK-Medien und polymerisierbaren Verbindungen zur Verwendung in solchen Anzeigen, welche die oben beschriebenen Nachteile nicht oder nur in geringem Maße zeigen und verbesserte Eigenschaften besitzen. Insbesondere besteht ein großer Bedarf nach PSA-Anzeigen, sowie Materialien zur Verwendung in PSA-Anzeigen, die einen hohen spezifischen Widerstand bei gleichzeitig großem Arbeitstemperaturbereich, kurze Schaltzeiten auch bei tiefen Temperaturen und niedriger Schwellenspannung, einen niedrigen pretilt-Winkel, eine Vielzahl von Graustufen, einen hohen Kontrast und einen weiten Blickwinkel ermöglichen, sowie hohe Werte der "voltage holding ratio" (VHR) nach UV-Belastung aufweisen.

Der Erfindung liegt die Aufgabe zugrunde, neue geeignete Materialien, insbesondere RMs und diese enthaltende FK-Medien, für die Verwendung in PSA-Anzeigen bereitzustellen, die die oben angegebenen Nachteile nicht oder in geringerem Maße aufweisen, möglichst schnell und vollständig polymerisieren, eine möglichst schnelle Einstellung eines niedrigen pretilt-Winkels ermöglichen, das Auftreten von "image sticking" in der Anzeige verringern oder vermeiden, und vorzugsweise gleichzeitig sehr hohe spezifische Widerstände, niedrige Schwellenspannungen und niedrige Schaltzeiten ermöglichen.

Weitere Aufgabe der Erfindung ist die Bereitstellung von neuen RMs, insbesondere für optische, elektrooptische und elektronische Anwendungen, sowie von geeigneten Verfahren und Zwischenprodukten zu ihrer Herstellung.

Insbesondere liegt der Erfindung die Aufgabe zugrunde, polymerisierbare Verbindungen bereitzustellen, die nach Photopolymerisation einen größeren maximalen Pretilt erzeugen, was zu einem schnelleren Erreichen des gewünschten Pretilts und so zu deutlich verkürzten Zeiten bei der Herstellung der FK-Anzeige führt

Diese Aufgabe wurde erfindungsgemäß gelöst durch die Bereitstellung von Materialien, Verfahren und Anzeigen wie in der vorliegenden Anmeldung beschrieben. Insbesondere wurde überraschend gefunden, dass die oben beschriebenen Aufgaben teilweise oder vollständig gelöst werden können, indem man erfindungsgemäße PSA-Anzeigen verwendet, die eine polymerisierte Verbindung mit einem Strukturelement der Formel **2** enthalten, 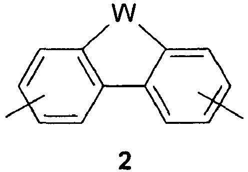 worin Weine Carbylbrücke oder ein Heteroatom bedeutet. Gegenüber den aus dem Stand der Technik bekannten Verbindungen der oben genannten Formel **1** wird durch Einführung eines Brückenelementes in das Biarylgerüst die Beweglichkeit um die zentrale Bindung eingeschränkt und so ein starres und sterisch anspruchsvolleres Grundgerüst erhalten.

Die Verwendung solcher polymerisierbarer Verbindungen in erfindungsgemäßen FK-Medien und PSA-Anzeigen führt zu einem besonders schnellen Erreichen des gewünschten Pretilts und zu deutlich verkürzten Zeiten bei der Herstellung der Anzeige. Dies konnte in Verbindung mit einem FK-Medium mittels belichtungszeitabhängiger Pretilt-Messungen in VA-Tilt-Messzellen nachgewiesen werden. Insbesondere konnte ein Pretilt ohne den Zusatz von Photoinitiator erreicht werden

Da die polymerisierbaren Verbindungen in den erfindungsgemäßen Anzeigen eine deutlich höhere Polymerisationsgeschwindigkeit zeigen, verbleiben auch weniger unreagierte Restmengen in der Zelle, wodurch deren elektrooptische Eigenschaften verbessert werden, und das kontrollierte Abreagieren dieser Restmengen einfacher wird.

Polymerisierbare Verbindungen mit einem Strukturelement der Formel **2** wurden im Stand der Technik bereits für andere Zwecke beschrieben. So offenbart die US 6,824,709 polymerisierbare Verbindungen mit einer zentralen Fluorengruppe (d.h. der Formel **2** worin W = CH₂) zur Verwendung in anisotropen Polymerfilmen wie beispielsweise Orientierungsschichten, Retardationsfilmen oder Polarisatoren.

Die US 5,674,576 offenbart FK-Anzeigen des PSCT-Typs ("polymer stabilized cholesteric texture") oder PDLC-Typs ("polymer dispersed liquid crystal"), enthaltend ein chiral nematisches oder cholesterisches FK-Medium (d.h. mit helikaler Verdrillung der FK-Moleküle) sowie ein Polymer. Das Polymer ist erhältlich durch Polymerisation eines Fluorendiacrylats oder -dimethacrylats, oder dessen Derivat mit einer O- oder NH-Brücke anstelle der CH₂-Brücke. Die Anzeige wird durch Anlegen einer Spannung zwischen verschiedenen Zuständen geschaltet, in denen das chirale FK-Medium entweder Lichtstreuung, Transparenz oder Selektivreflektion von farbigem Licht zeigt. Die US 5,674,576 offenbart jedoch keine Anzeigen des PSA-Typs wie in der vorliegenden Erfindung beschrieben, worin die Polymerisation der polymerisierbaren Komponente unter Anlegen einer Spannung erfolgt und dadurch ein pretilt-Wnkel im FK-Medium erzeugt wird. Zudem enthalten die in der US 5,674,576 beschriebenen FK-Anzeigen größere Mengen an polymerisierbaren Verbindungen, vorzugsweise von 0.5 bis 8 Gew.%, um sichtbare Streueffekte oder Reflektion zu ermöglichen. Dagegen sind solch große RM-Mengen und die dadurch hervorgerufenen Streueffekte oder Selektivreflektion in den PSA-Anzeigen der vorliegenden Erfindung unerwünscht Auch die in den Beispielen der US 5,674,576 beschriebenen langen Polymerisationszeiten von 150-180 Minuten sind für eine Herstellung von FK-Anzeigen im industriellen Maßstab nicht geeignet.

D1 (EP 0 397 418 A1) offenbart die Verbindung 2,7-Dibrom-9,10-dihydrophenanthren.

D2 (Pearson et al., Synthesis 1976, 09, 621-623) offenbart die Verbindung 2,7-Dijod-9,10-dihydrophenanthren.

D3 (EP 1 837 324 A1) offenbart die Verbindungen 2,7-Dichlor-9,10-dihydrophenanthren und 2,7-Dibrom-9,10-dihydrophenanthren.

D4 (Conforth et al., J. Chem. Soc. 1942, 684-689) offenbart die Verbindung 2,7-Dihydroxy-9,10-dihydrophenanthren.

D5 (JP 63-233952 A) offenbart die Verbindung 2,7-Dijod-9,10-dihydrophenanthren.

D6 (WO 2009/030322 A1) offenbart polymerisierbare Verbindungen einer generischen Formel I sowie deren Verwendung in FK-Medien für PSA-Anzeigen.

D7 (DE 10 2008 036248 A1) offenbart direaktive polymerisierbare Verbindungen mit nur einer Abstandsgruppe.

D10 (US 2005/255258 A1) offenbart nichtreaktive flüssigkristalline Fluorenverbindungen und deren Verwendung in FK-Medien und FK-Anzeigen. D10 offenbart jedoch keine polymerisierbaren Fluorenverbindungen.

D11 (WO 2009/030352 A1) offenbart Fluorenderivate und deren Verwendung in FK-Medien und FK-Polymerfilmen mit negativer optischer Dispersion.

D12 (US 2002/0110650 A1) offenbart Phenylacetylenverbindungen, welche auch polymerisierbar sein können und zwei Dibenzothiophengruppen enthalten können, und deren Verwendung in FK-Medien, FK-Anzeigen und FK-Polymeren.

D13 (WO 2005/054406 A1) offenbart polymerisierbare Verbindungen, welche auch eine Carbazolgruppe enthalten können, sowie deren Verwendung in FK-Medien, FK-Anzeigen, FK-Polymeren und FK-Netzwerken.

D15 (US 5,648,021) offenbart nichtreaktive flüssigkristalline Dihydrophenanthrenderivate und deren Verwendung in FK-Medien und FK-Anzeigen.

D14 (EP 1 944 287 A1) offenbart polymerisierbare flüssigkristalline Verbindungen, welche auch eine Phenanthrengruppe enthalten können, und deren Verwendung in FK-Polymeren.

D16 (DE 10 2005 057 250 A1) offenbart nichtreaktive flüssigkristalline Benzochromen-derivate und deren Verwendung in FK-Medien und FK-Anzeigen.

Die Verwendung von polymerisierbaren Fluorenderivaten in niedermolekularen FK-Medien für PSA-Anzeigen zur schnellen Einstellung eines Tiltwinkels durch in situ-Polymerisation im elektrischen Feld wurde im Stand der Technk jedoch bisher weder beschrieben noch vorgeschlagen.

Gegenstand der Erfindung ist somit die Verwendung von Verbindungen der Formel I 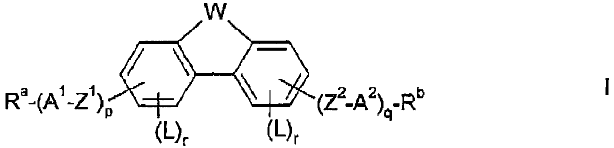 worin die einzelnen Reste folgende Bedeutung besitzen
- W: -C(R^{c}R^{d})-, -CH₂CH₂-, -CH₂-O-, -O-, -CO-, -CO-O-, -S- oder - N(R^{c})-,
- R^{c}, R^{d}: jeweils unabhängig voneinander P-Sp-, H, F, Cl, Br, I, -CN, - NO₂, -NCO, -NCS, -OCN, -SCN, SF₅, geradkettiges oder verzweigtes Alkyl mit 1 bis 25 C-Atomen, worin auch eine oder mehrere nicht benachbarte CH₂-Gruppen Jeweils unabhängig voneinander durch Arylen, -C(R⁰)=C(R⁰⁰)-, -C=C-, -N(R⁰)-, -O-, - S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, Br, I, CN oder P-Sp- ersetzt sein können, oder Aryl oder Heteroaryl, vorzugsweise mit 2 bis 25 C-Atomen, welches auch zwei oder mehr anellierte Ringe enthalten kann und welches optional durch L ein- oder mehrfach substituiert ist,
- R^{a}, R^{b}: jeweils unabhängig voneinander eine der für R^{c} angegebenen Bedeutungen, wobei mindestens einer der Reste R^{a} und R^{b} eine Gruppe P-Sp- bedeutet oder enthält,
- P: bei jedem Auftreten gleich oder verschieden eine polymerisierbare Gruppe,
- Sp: bei jedem Auftreten gleich oder verschieden eine Abstandsgruppe oder eine Einfachbindung,
- A¹ und A²: jeweils unabhängig voneinander 1,4-Phenylen, Naphthalin-1,4-diyl oder Naphthalin-2,6-diyl, wobei in diesen Gruppen auch eine oder mehrere CH-Gruppen durch N ersetzt sein können, Cyclohexan-1,4-diyl, worin auch eine oder mehrere nicht-benachbarte CH₂-Gruppen durch O und/oder S ersetzt sein können, 1,4-Cyclohexenylen, Bicyclo[1.1.1]pentan-1,3-diyl, Bicyclo[2.2.2]octan-1,4-diyl, Spiro[3.3]heptan-2,6-diyl, Piperidin-1,4-diyl, Decahydronaphthalin-2,6-diyl, 1,2,3,4-Tetrahydronaphthalin-2,8-diyl, Indan-2,5-diyl, Octahydro-4,7-methano-indan-2,5-diyl, Phenanthren-2,7-diyl oder Anthracen-2,7-diyl, wobei alle diese Gruppen unsubstituiert oder durch L ein- oder mehrfach substituiert sein können,
- L: P, P-Sp-, OH, CH₂OH, F, Cl, Br, I, -CN, -NO₂, -NCO, -NCS, -OCN, -SCN, -C(=O)N(R^{x})₂, -C(=O)Y¹, -C(=O)R^{x}, -N(R^{x})₂, optional substituiertes Silyl, optional substituiertes Aryl mit 6 bis 20 C Atomen, geradkettiges oder verzweigtes Alkyl oder Alkoxy 1 bis 25 C-Atomen, oder geradkettiges oder verzweigtes Alkenyl, Alkinyl, Alkylcarbonyl, Alkoxycarbonyl, Alkylcarbonyloxy oder Alkoxycarbonyloxy mit 2 bis 25 C-Atomen, worin in allen diesen Gruppen auch ein oder mehrere H-Atome durch F, Cl, P oder P-Sp- ersetzt sein können,
- Y¹: Halogen,
- R^{x}: P, P-Sp-, H, Halogen, geradkettiges, verzweigtes oder cyclisches Alkyl mit 1 bis 25 C-Atomen, worin auch eine oder mehrere nicht benachbarte CH₂-Gruppen durch -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl oder P-Sp- ersetzt sein können,
- Z¹, Z²: jeweils unabhängig voneinander-O-, -S-, -CO-, -CO-O-, -OCO-, -O-CO-O-, -OCH₂-, -CH₂O-, -SCH₂-, -CH₂S-, -CF₂O-, -OCF₂-, - CF₂S-, -SCF₂-, -(CH₂)ₙ-, -CF₂CH₂-, -CH₂CF₂-, -(CF₂)ₙ-, -CH=CH-, -CF=CF-, -CH=CF-, -CF=CH-, -C=C-, -CH=CH-COO-, -OCO-CH=CH-, -CH₂-CH₂-COO-, -OCO-CH₂-CH₂-, -C(R⁰R⁰⁰)-, - C(R^{y}R²)-, oder eine Einfachbindung,
- R⁰, R⁰⁰: jeweils unabhängig voneinander und bei jedem Auftreten gleich oder verschieden H oder Alkyl mit 1 bis 12 C-Atomen,
- R^{y}, R^{z}: jeweils unabhängig voneinander H, F, CH₃ oder CF₃,
- n: bei jedem Auftreten gleich oder verschieden 1, 2, 3 oder 4,
- p, q: jeweils unabhängig voneinander 0, 1, 2 oder 3,
- r: bei jedem Auftreten gleich oder verschieden 0, 1, 2 oder 3,
in FK-Anzeigen des PS-(polymer stabilized) oder PSA-(polymer sustained alignment) Typs.

Ein weiterer Gegenstand der Erfindung ist ein FK-Medium enthaltend eine oder mehrere polymerisierbare Verbindungen der Formel I wie in Anspruch 12, 13, 14, 15 oder 16 definiert und eine oder mehrere zusätzliche Verbindungen, welche auch mesogen, flüssigkristallin und/oder polymerisierbar sein können.

Ein weiterer Gegenstand der Erfindung ist ein FK-Medium enthaltend ein Polymer erhältlich durch Polymerisation einer oder mehrerer Verbindungen der Formel I wie in Anspruch 12, 13, 14, 15 oder 16 definiert und eine oder mehrere zusätzliche Verbindungen, welche auch mesogen, flüssigkristallin und/oder polymerisierbar sein können.

Ein weiterer Gegenstand der Erfindung ist ein FK-Medium enthaltend
- eine polymerisierbare Komponente A), enthaltend eine oder mehrere polymerisierbare Verbindungen der Formel I wie in Anspruch 12, 13, 14, 15 oder 16 definiert, sowie
- eine flüssigkristalline Komponente B), im Folgenden auch als "FK-Hostmischung" bezeichnet, enthaltend eine oder mehrere, vorzugsweise zwei oder mehr niedermolekulare (monomere und unpolymerisierbare) Verbindungen wie vor- und nachstehend beschrieben.

Ein weiterer Gegenstand der Erfindung ist ein Verfahren zur Herstellung eines FK-Mediums wie vor- und nachstehend beschrieben, indem man eine oder mehrere niedermolekulare flüssigkristalline Verbindungen, oder eine FK-Hostmischung wie vor- und nachstehend beschrieben, mit einer oder mehreren polymerisierbaren Verbindungen der Formel I wie in Anspruch 12, 13, 14, 15 oder 16 definiert oderderen Unterformeln, und gegebenenfalls mit weiteren flüssigkristallinen Verbindungen und/oder Additiven, mischt.

Ein weiterer Gegenstand der Erfindung ist die Verwendung von Verbindungen der Formel I und erfindungsgemäßen FK-Medien in PS- und PSA-Anzeigen, insbesondere die Verwendung in PS- und PSA-Anzeigen enthaltend ein FK-Medium, zur Erzeugung eines Tiltwinkels im FK-Medium durch in situ-Polymerisation der Verbindung(en) der Formel I in der PSA-Anzeige unter Anlegen eines elektrischen oder magnetischen Feldes.

Ein weiterer Gegenstand der Erfindung ist eine FK-Anzeige enthaltend eine oder mehrere Verbindungen der Formel I oder ein erfindungsgemäßes FK-Medium, insbesondere eine PS- oder PSA-Anzeige, besonders bevorzugt eine PSA-VA-, PSA-OCB-, PS-IPS-, PS-FFS- oder PS-TN-Anzeige.

Ein weiterer Gegenstand der Erfindung ist eine FK-Anzeige des PS- oder PSA-Typs, enthaltend eine FK-Zelle mit zwei Substraten, wobei mindestens ein Substrat lichtdurchlässig ist und mindestens ein Substrat eine oder zwei Elektroden aufweist, sowie einer zwischen den Substraten befindlichen Schicht eines FK-Mediums enthaltend eine polymerisierte Komponente und eine niedermolekulare Komponente, wobei die polymerisierte Komponente erhältlich ist durch Polymerisation einer oder mehrerer polymerisierbarer Verbindungen zwischen den Substraten der FK-Zelle im FK-Medium unter Anlegen einer elektrischen Spannung an die Elektroden, wobei mindestens eine der polymerisierbaren Verbindungen aus Formel I ausgewählt ist.

Ein weiterer Gegenstand der Erfindung ist ein Verfahren zur Herstellung einer FK-Anzeige wie vor- und nachstehend beschrieben, indem man ein FK-Medium, enthaltend eine oder mehrere niedermolekulare flüssigkristalline Verbindungen oder eine FK-Hostmischung wie vor- und nachstehend beschrieben sowie eine oder mehrere polymerisierbare Verbindungen der Formel I oder deren Unterformeln, in eine FK-Zelle mit zwei Substraten und zwei Elektroden wie vor- und nachstehend beschrieben füllt, und die polymerisierbaren Verbindungen unter Anlegen einer elektrischen Spannung an die Elektroden polymerisiert.

Die erfindungsgemäßen PS- und PSA-Anzeigen weisen zwei Elektroden, vorzugsweise in Form von transparenten Schichten, auf, wobei diese auf einem oder beiden der Substrate aufgebracht sind, die die FK-Zelle bilden. Dabei ist entweder jeweils eine Elektrode auf je einem der beiden Substrate aufgebracht, wie zum Beispiel in erfindungsgemäßen PSA-VA-, PSA-OCB- oder PSA-TN-Anzeigen, oder beide Elektroden sind auf nur einem der beiden Substrate aufgebracht, während das andere Substrat keine Elektrode aufweist, wie zum Beispiel in erfindungsgemäßen PSA-IPS- oder PSA-FFS-Anzeigen.

Weiterer Gegenstand der Erfindung sind neue Verfahren zur Herstellung von Verbindungen der Formel I wie in Anspruch 12, 13, 14, 15 oder 16 defniert, sowie in diesen Verfahren verwendete oder daraus erhaltene neue Zwischenprodukte.

Weiterer Gegenstand der Erfindung sind neue Verbindungen der Formel I wie in Anspruch 12, 13, 14, 15 oder 16 definiert.

Vor- und nachstehend gelten folgende Bedeutungen:
Der Begriff "PSA" wird, falls nicht anders angegeben, stellvertretend für PS-Anzeigen und PSA-Anzeigen verwendet.

Die Begriffe "Tilt" und "Tiltwinkel" beziehen sich auf eine gekippte oder geneigte Orientierung der FK-Moleküle eines FK-Mediums relativ zu den Oberflächen der Zelle in einer FK-Anzeige (hier vorzugsweise einer PS- oder PSA-Anzeige). Der Tiltwinkel bezeichnet dabei den durchschnittlichen Winkel (<90°) zwischen den Moleküllängsachsen der FK-Moleküle (FK-Direktor) und der Oberfläche der planparallelen Trägerplatten, welche die FK-Zelle bilden. Ein niedriger Wert des Tiltwinkels (d.h. eine große Abweichung vom 90°-Winkel) entspricht dabei einem großen Tilt. Eine geeignete Methode zur Messung des Tiltwinkels findet sich in den Beispielen. Soweit nicht anders angegeben, beziehen sich vor- und nachstehend offenbarte Werte des Tiltwinkels auf diese Messmethode.

Der Begriff "mesogene Gruppe" ist dem Fachmann bekannt und in der Literatur beschrieben, und bedeutet eine Gruppe, die durch die Anisotropie ihrer anziehenden und abstoßenden Wechselwirkungen wesentlich dazu beiträgt, in niedermolekularen oder polymeren Substanzen eine Flüssigkristall(FK-)Phase hervorzurufen. Verbindungen enthaltend mesogene Gruppen (mesogene Verbindungen) müssen nicht unbedingt selbst eine FK-Phase aufweisen. Es ist auch möglich, dass mesogene Verbindungen FK-Phasenverhalten nur nach Vermischung mit anderen Verbindungen und/oder nach Polymerisation zeigen. Typische mesogene Gruppen sind beispielsweise starre stäbchen- oder scheibchenförmige Einheiten. Ein Überblick über die im Zusammenhang mit mesogenen bzw. FK-Verbindungen verwendeten Begriffe und Definitionen findet sich in Pure Appl. Chem. 73(5), 888 (2001) und C. Tschierske, G. Pelzl, S. Diele, Angew. Chem. 2004, 116, 6340-6368.

Der Begriff "Abstandsgruppe" (engl. "spacer" oder "spacer group"), vor- und nachstehend auch als "Sp" bezeichnet, ist dem Fachmann bekannt und in der Literatur beschrieben, siehe beispielsweise Pure Appl. Chem. 73(5), 888 (2001) und C. Tschierske, G. Pelzl, S. Diele, Angew. Chem. 2004, 116, 6340-6368. Falls nicht anders angegeben, bezeichnet der Begriff "Abstandsgruppe" bzw. "Spacer" vor- und nachstehend eine flexible Gruppe, die in einer polymerisierbaren mesogenen Verbindung die mesogene Gruppe und die polymerisierbare(n) Gruppe(n) miteinander verbindet.

Der Begriff "reaktives Mesogen" oder "RM" bezeichnet eine Verbindung enthaltend eine mesogene Gruppe und eine oder mehrere funktionelle Gruppen, die zur Polymerisation geeignet sind (auch als polymerisierbare Gruppe oder Gruppe P bezeichnet).

Die Begriffe "niedermolekulare Verbindung" und "unpolymerisierbare Verbindung" bezeichnen, üblicherweise monomere, Verbindungen, die keine funktionelle Gruppe aufweisen, welche zur Polymerisation unter den üblichen dem Fachmann bekannten Bedingungen, insbesondere unter den zur Polymerisation der RMs verwendeten Bedingungen, geeignet ist.

Der Begriff "organische Gruppe" bedeutet eine Kohlenstoff- oder Kohlenwasserstoffgruppe.

Der Begriff "Kohlenstoffgruppe" bedeutet eine ein- oder mehrbindige organische Gruppe enthaltend mindestens ein Kohlenstoffatom, wobei diese entweder keine weiteren Atome enthält (wie z.B. -C≡C-), oder gegebenenfalls ein oder mehrere weitere Atome wie beispielsweise N, O, S, P, Si, Se, As, Te oder Ge enthält (z.B. Carbonyl etc.). Der Begriff "Kohlenwasserstoffgruppe" bedeutet eine Kohlenstoffgruppe, die zusätzlich ein oder mehrere H-Atome und gegebenenfalls ein oder mehrere Heteroatome wie beispielsweise N, O, S, P, Si, Se, As, Te oder Ge enthält.

"Halogen" bedeutet F, Cl, Br oder I.

Eine Kohlenstoff- oder Kohlenwasserstoffgruppe kann eine gesättigte oder ungesättigte Gruppe sein. Ungesättigte Gruppen sind beispielsweise Aryl-, Alkenyl- oder Alkinylgruppen. Ein Kohlenstoff- oder Kohlenwasserstoffrest mit mehr als 3 C-Atomen kann geradkettig, verzweigt und/oder cyclisch sein, und kann auch Spiroverküpfungen oder kondensierte Ringe aufweisen.

Die Begriffe "Alkyl", "Aryl", "Heteroaryl" etc. umfassen auch mehrbindige Gruppen, beispielsweise Alkylen, Arylen, Heteroarylen etc.

Der Begriff "Aryl" bedeutet eine aromatische Kohlenstoffgruppe oder eine davon abgeleitete Gruppe. Der Begriff "Heteroaryl" bedeutet "Aryl" gemäß vorstehender Definition, enthaltend ein oder mehrere Heteroatome.

Bevorzugte Kohlenstoff- und Kohlenwasserstoffgruppen sind gegebenenfalls substituiertes Alkyl, Alkenyl, Alkinyl, Alkoxy, Alkylcarbonyl, Alkoxycarbonyl, Alkylcarbonyloxy und Alkoxycarbonyloxy mit 1 bis 40, vorzugsweise 1 bis 25, besonders bevorzugt 1 bis 18 C-Atomen, gegebenenfalls substituiertes Aryl oder Aryloxy mit 6 bis 40, vorzugsweise 6 bis 25 C-Atomen, oder gegebenenfalls substituiertes Alkylaryl, Arylalkyl, Alkylaryloxy, Arylalkyloxy, Arylcarbonyl, Aryloxycarbonyl, Arylcarbonyloxy und Aryloxycarbonyloxy mit 6 bis 40, vorzugsweise 6 bis 25 C-Atomen.

Weitere bevorzugte Kohlenstoff- und Kohlenwasserstoffgruppen sind C₁-C₄₀ Alkyl, C₂-C₄₀ Alkenyl, C₂-C₄₀ Alkinyl, C₃-C₄₀ Allyl, C₄-C₄₀ Alkyldienyl, C₄-C₄₀ Polyenyl, C₆-C₄₀ Aryl, C₆-C₄₀ Alkylaryl, C₆-C₄₀ Arylalkyl, C₆-C₄₀ Alkylaryloxy, C₆-C₄₀ Arylalkyloxy, C₂-C₄₀ Heteroaryl, C₄-C₄₀ Cycloalkyl, C₄-C₄₀ Cycloalkenyl, etc. Besonders bevorzugt sind C₁-C₂₂ Alkyl, C₂-C₂₂ Alkenyl, C₂-C₂₂ Alkinyl, C₃-C₂₂ Allyl, C₄-C₂₂ Alkyldienyl, C₆-C₁ Aryl, C₆-C₂₀ Arylalkyl und C₂-C₂₀ Heteroaryl.

Weitere bevorzugte Kohlenstoff- und Kohlenwasserstoffgruppen sind geradkettige, verzweigte oder cyclische Alkylreste mit 1 bis 40, vorzugsweise 1 bis 25 C-Atomen, welche unsubstituiert oder durch F, Cl, Br, I oder CN ein- oder mehrfach substituiert sind, und worin ein mehrere nicht benachbarte CH₂-Gruppen jeweils unabhängig voneinander durch - C(R^{x})=C(R^{x})-, -C≡C-, -N(R^{x})-, -O-, -S-, -CO-, -CO-O-, -O-CO-, -O-CO-O-so ersetzt sein können, dass O- und/oder S-Atome nicht direkt miteinander verknüpft sind.

R^{x} bedeutet vorzugsweise H, Halogen, eine geradkettige, verzweigte oder cyclische Alkylkette mit 1 bis 25 C-Atomen, in der auch ein oder mehrere nicht benachbarte C-Atome durch -O-, -S-, -CO-, -CO-O-, -O-CO-, -O-CO-O- ersetzt sein können, wobei auch ein oder mehrere H-Atome durch Fluor ersetzt sein können, eine optional substituierte Aryl- oder Aryloxygruppe mit 6 bis 40 C-Atomen, oder eine optional substituierte Heteroaryl- oder Heteroaryloxygruppe mit 2 bis 40 C-Atomen.

Bevorzugte Alkoxygruppen sind beispielsweise Methoxy, Ethoxy, 2-Methoxyethoxy, n-Propoxy, i-Propoxy, n-Butoxy, i-Butoxy, s-Butoxy, t-Butoxy, 2-Methylbutoxy, n-Pentoxy, n-Hexoxy, n-Heptoxy, n-Octoxy, n-Nonoxy, n-Decoxy, n-Undecoxy, n-Dodecoxy, etc.

Bevorzugte Alkylgruppen sind beispielsweise Methyl, Ethyl, n-Propyl, Isopropyl, n-Butyl, Isobutyl, s-Butyl, t-Butyl, 2-Methylbutyl, n-Pentyl, s-Pentyl, Cyclopentyl, n-Hexyl, Cyclohexyl, 2-Ethylhexyl, n-Heptyl, Cycloheptyl, n-Octyl, Cyclooctyl, n-Nonyl, n-Decyl, n-Undecyl, n-Dodecyl, Dodecanyl, Trifluoromethyl, Perfluoro-n-butyl, 2,2,2-Trifluoroethyl, Perfluorooctyl, Perfluorohexyl etc.

Bevorzugte Alkenylgruppen sind beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl etc.

Bevorzugte Alkinylgruppen sind beispielsweise Ethinyl, Propinyl, Butinyl, Pentinyl, Hexinyl, Octinyl etc.

Bevorzugte Alkoxygruppen sind beispielsweise Methoxy, Ethoxy, 2-Methoxyethoxy, n-Propoxy, i-Propoxy, n-Butoxy, i-Butoxy, s-Butoxy, t-Butoxy, 2-Methylbutoxy, n-Pentoxy, n-Hexoxy, n-Heptoxy, n-Octoxy, n-Nonoxy, n-Decoxy, n-Undecoxy, n-Dodecoxy, etc.

Bevorzugte Aminogruppen sind beispielsweise Dimethylamino, Methylamino, Methylphenylamino, Phenylamino, etc.

Aryl- und Heteroarylgruppen können einkernig oder mehrkernig sein, d.h. sie können einen Ring (wie z.B. Phenyl) oder zwei oder mehr Ringe aufweisen, welche auch anelliert (wie z.B. Naphthyl) oder kovalent verknüpft sein können (wie z.B. Biphenyl), oder eine Kombination von anellierten und verknüpften Ringen beinhalten. Heteroarylgruppen enthalten ein oder mehrere Heteroatome, vorzugsweise ausgewählt aus O, N, S und Se.

Besonders bevorzugt sind ein-, zwei- oder dreikernige Arylgruppen mit 6 bis 25 C-Atomen sowie ein-, zwei- oder dreikernige Heteroarylgruppen mit 5 bis 25 Ringatomen, welche optional anellierte Ringe enthalten und optional substituiert sind. Ferner bevorzugt sind 5-, 6- oder 7-gliedrige Aryl- und Heteroarylgruppen, worin auch eine oder mehrere CH-Gruppen durch N, S oder O so ersetzt sein können, dass O-Atome und/oder S-Atome nicht direkt miteinander verknüpft sind.

Bevorzugte Arylgruppen sind beispielsweise Phenyl, Biphenyl, Terphenyl, [1,1':3',1"]Terphenyl-2'-yl, Naphthyl, Anthracen, Binaphthyl, Phenanthren, Pyren, Dihydropyren, Chrysen, Perylen, Tetracen, Pentacen, Benzpyren, Fluoren, Inden, Indenofluoren, Spirobifluoren, etc.

Bevorzugte Heteroarylgruppen sind beispielsweise 5-gliedrige Ringe wie Pyrrol, Pyrazol, Imidazol, 1,2,3-Triazol, 1 ,2,4-Triazol, Tetrazol, Furan, Thiophen, Selenophen, Oxazol, Isoxazol, 1,2-Thiazol, 1,3-Thiazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 6-gliedrige Ringe wie Pyridin, Pyridazin, Pyrimidin, Pyrazin, 1,3,5-Triazin, 1,2,4-Triazin, 1,2,3-Triazin, 1,2,4,5-Tetrazin, 1,2,3,4-Tetrazin, 1,2,3,5-Tetrazin, oder kondensierte Gruppen wie Indol, Isoindol, Indolizin, Indazol, Benzimidazol, Benzotriazol, Purin, Naphthimidazol, Phenanthrimidazol, Pyridimidazol, Pyrazinimidazol, Chinoxalinimidazol, Benzoxazol, Naphthoxazol, Anthroxazol, Phenanthroxazol, Isoxazol, Benzothiazol, Benzofuran, Isobenzofuran, Dibenzofuran, Chinolin, Isochinolin, Pteridin, Benzo-5,6-chinolin, Benzo-6,7-chinolin, Benzo-7,8-chinolin, Benzoisochinolin, Acridin, Phenothiazin, Phenoxazin, Benzopyridazin, Benzopyrimidin, Chinoxalin, Phenazin, Naphthyridin, Azacarbazol, Benzocarbolin, Phenanthridin, Phenanthrolin, Thieno[2,3b]thiophen, Thieno[3,2b]thiophen, Dithienothiophen, Isobenzothiophen, Dibenzothiophen, Benzothiadiazothiophen, oder Kombinationen dieser Gruppen. Die Heteroarylgruppen können auch mit Alkyl, Alkoxy, Thioalkyl, Fluor, Fluoralkyl oder weiteren Aryl- oder Heteroarylgruppen substituiert sein.

Die (nicht-aromatischen) alicyclischen und heterocyclischen Gruppen umfassen sowohl gesättigte Ringe, d.h. solche die ausschließlich Einfachbindungen enthalten, als auch teilweise ungesättigte Ringe, d.h. solche die auch Mehrfachbindungen enthalten können. Heterocyclische Ringe enthalten ein oder mehrere Heteroatome, vorzugsweise ausgewählt aus Si, O, N, S und Se.

Die (nicht-aromatischen) alicyclischen und heterocyclischen Gruppen können einkernig sein, d.h. nur einen Ring enthalten (wie z.B. Cyclohexan), oder mehrkernig sein, d.h. mehrere Ringe enthalten (wie z.B. Decahydronaphthalin oder Bicyclooctan). Besonders bevorzugt sind gesättigte Gruppen. Ferner bevorzugt sind ein-, zwei- oder dreikernige Gruppen mit 5 bis 25 Ringatomen, welche optional anellierte Ringe enthalten und optional substituiert sind. Ferner bevorzugt sind 5-, 6-, 7- oder 8-gliedrige carbocyclische Gruppen worin auch ein oder mehrere C-Atome durch Si ersetzt sein können und/oder eine oder mehrere CH-Gruppen durch N ersetzt sein können und/oder eine oder mehrere nichtbenachbarte CH₂-Gruppen durch -O- und/oder -S- ersetzt sein können.

Bevorzugte alicyclische und heterocyclische Gruppen sind beispielsweise 5-gliedrige Gruppen wie Cyclopentan, Tetrahydrofuran, Tetrahydrothiofuran, Pyrrolidin, 6-gliedrige Gruppen wie Cyclohexan, Silinan, Cyclohexen, Tetrahydropyran, Tetrahydrothiopyran, 1,3-Dioxan, 1,3-Dithian, Piperidin, 7- gliedrige Gruppen wie Cycloheptan, und anellierte Gruppen wie Tetrahydronaphthalin, Decahydronaphthalin, Indan, Bicyclo[1.1.1]pentan-1,3-diyl, Bicyclo[2.2.2]octan-1,4-diyl, Spiro[3.3]heptan-2,6-diyl, Octahydro-4,7-methano-indan-2,5-diyl.

Bevorzugte Substituenten sind beispielsweise löslichkeitsfördernde Gruppen wie Alkyl oder Alkoxy, elektronenziehende Gruppen wie Fluor, Nitro oder Nitril, oder Substituenten zur Erhöhung der Glastemperatur (Tg) im Polymer, insbesondere voluminöse Gruppen wie z.B. t-Butyl oder gegebenenfalls substituierte Arylgruppen.

Bevorzugte Substituenten, vor- und nachstehend auch als "L" bezeichnet, sind beispielsweise F, Cl, Br, I, -CN, -NO₂, -NCO, -NCS, -OCN, -SCN, - C(=O)N(R^{x})₂, -C(=O)Y¹, -C(=O)R^{x}, -N(R^{x})₂, worin R^{x} die oben angegebene Bedeutung hat und Y¹ Halogen bedeutet, optional substituiertes Silyl oder Aryl mit 6 bis 40, vorzugsweise 6 bis 20 C Atomen, und geradkettiges oder verzweigtes Alkyl, Alkoxy, Alkylcarbonyl, Alkoxycarbonyl, Alkylcarbonyloxy oder Alkoxycarbonyloxy mit 1 bis 25 C-Atomen, worin ein oder mehrere H-Atome gegebenenfalls durch F oder Cl ersetzt sein können.

"Substituiertes Silyl oder Aryl" bedeutet vorzugsweise durch Halogen, - CN, R⁰, -OR⁰, -CO-R⁰, -CO-O-R⁰, -O-CO-R⁰ oder -O-CO-O-R⁰ substituiert, worin R⁰ die oben angegebene Bedeutung hat.

Besonders bevorzugte Substituenten L sind beispielsweise F, Cl, CN, NO₂, CH₃, C₂H₅, OCH₃, OC₂H₅, COCH₃, COC₂H₅, COOCH₃, COOC₂H₅, CF₃, OCF₃, OCHF₂, OC₂F₅, ferner Phenyl. 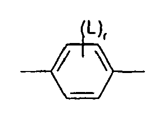 ist vorzugsweise 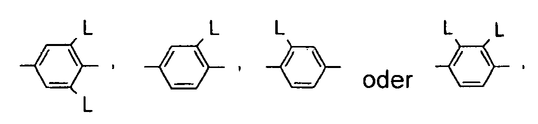 worin L eine der oben angegebenen Bedeutungen hat.

Die polymerisierbare Gruppe P ist eine Gruppe, die für eine Polymerisationsreaktion, wie beispielsweise die radikalische oder ionische Kettenpolymerisation, Polyaddition oder Polykondensation, oder für eine polymeranaloge Umsetzung, beispielsweise die Addition oder Kondensation an eine Polymerhauptkette, geeignet ist. Besonders bevorzugt sind Gruppen für die Kettenpolymerisation, insbesondere solche enthaltend eine C=C-Doppelbindung oder -C≡C-Dreifachbindung, sowie zur Polymerisation unter Ringöffnung geeignete Gruppen wie beispielsweise Oxetan- oder Epoxygruppen

Bevorzugte Gruppen P sind ausgewählt aus der Gruppe bestehend aus CH₂=CW¹-CO-O-, CH₂=CW¹-CO-, 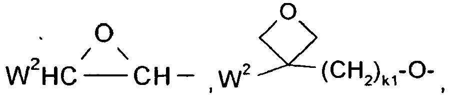 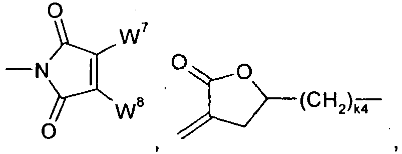 CH₂=CW²-(O)ₖ₃-, CW¹=CH-CO-(O)ₖ₃-, CW¹=CH-CO-NH-, CH₂=CW¹-CO-NH-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH-, HOOC-, OCN-, und W⁴W⁵W⁶Si-, worin W¹ H, F, Cl, CN, CF₃, Phenyl oder Alkyl mit 1 bis 5 C-Atomen, insbesondere H, F, Cl oder CH₃ bedeutet, W² und W³ jeweils unabhängig voneinander H oder Alkyl mit 1 bis 5 C-Atomen, insbesondere H, Methyl, Ethyl oder n-Propyl bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet, welches optional mit einem oder mehreren, von P-Sp- verschiedenen Resten L wie oben definiert substiuiert ist, k₁, k₂ und k₃ jeweils unabhängig voneinander 0 oder 1 bedeuten, k₃ vorzugsweise 1 bedeutet, und k₄ eine ganze Zahl von 1 bis 10 bedeutet.

Besonders bevorzugte Gruppen P sind ausgewählt aus der Gruppe bestehend aus CH₂=CW¹-CO-O-, CH₂=CW¹-CO-, 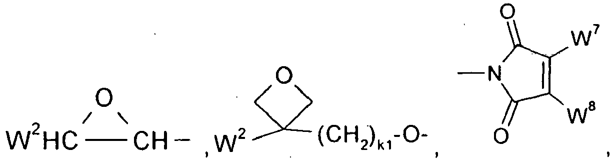 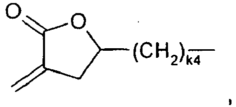 CH₂=CW²-O-, CW¹=CH-CO-(O)ₖ₃-, CW¹=CH-CO-NH-, CH₂=CW¹-CO-NH-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, CH₂=CW¹-CO-NH-, CH₂=CH-(COO)ₖ₁-Phe-(O)ₖ₂-, CH₂=CH-(CO)ₖᵢ-Phe-(O)ₖ₂-, Phe-CH=CH- und W⁴W⁵W⁶Si-, worin W¹ H, F, Cl, CN, CF₃, Phenyl oder Alkyl mit 1 bis 5 C-Atomen, insbesondere H, F, Cl oder CH₃ bedeutet, W² und W³ jeweils unabhängig voneinander H oder Alkyl mit 1 bis 5 C-Atomen, insbesondere H, Methyl, Ethyl oder n-Propyl bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet, k₁, k₂ und k₃ jeweils unabhängig voneinander 0 oder 1 bedeuten, k₃ vorzugsweise 1 bedeutet, und k₄ eine ganze Zahl von 1 bis 10 bedeutet.

Ganz besonders bevorzugte Gruppen P sind sind ausgewählt aus der Gruppe bestehend aus CH₂=CW¹-CO-O-, insbesondere CH₂=CH-CO-O-, CH₂=C(CH₃)-CO-O- und CH₂=CF-CO-O-, ferner CH₂=CH-O-, (CH₂=CH)₂CH-O-CO-, (CH₂=CH)₂CH-O-, 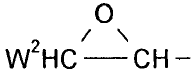 und

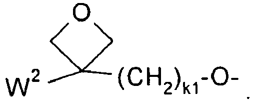

Weitere ganz besonders bevorzugte Gruppen P sind sind ausgewählt aus der Gruppe bestehend aus Vinyloxy-, Acrylat-, Methacrylat-, Fluoracrylat-, Chloracrylat-, Oxetan- und Epoxygruppen, und bedeuten besonders bevorzugt eine Acrylat- oder Methacrylatgruppe.

Bevorzugte, von einer Einfachbindung verschiedene Abstandsgruppen Sp sind ausgewählt aus der Formel Sp'-X', so dass der Rest P-Sp- der Formel P-Sp'-X'- entspricht, wobei
- Sp': Alkylen mit 1 bis 20, vorzugsweise 1 bis 12 C-Atomen bedeutet, welches optional durch F, Cl, Br, I oder CN ein- oder mehrfach substituiert ist, und worin auch eine oder mehrere nicht benachbarte CH₂-Gruppen jeweils unabhängig voneinander so durch -O-, -S-, -NH-, -N(R⁰)-, -Si(R⁰⁰R⁰⁰⁰)-, -CO-, -CO-O-, -O-CO-, -O-CO-O-, -S-CO-, -CO-S-, -N(R⁰⁰)-CO-O-, -O-CO-N(R⁰⁰)-, -N(R⁰⁰)-CO-N(R⁰⁰)-, -CH=CH- oder -C≡C- ersetzt sein können, dass O- und/oder S-Atome nicht direkt miteinander verknüpft sind,
- X': -O-, -S-, -CO-, -CO-O-, -O-CO-, -O-CO-O-, -CO-N(R⁰⁰)-, -N(R⁰⁰)-CO-, -N(R⁰⁰)-CO-N(R⁰⁰)-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=CY³-, -C≡C-, -CH=CH-CO-O-, -O-CO-CH=CH- oder eine Einfachbindung bedeutet,
- R⁰⁰ und R⁰⁰⁰: jeweils unabhängig voneinander H oder Alkyl mit 1 bis 12 C-Atomen bedeuten, und
- Y² und Y³: jeweils unabhängig voneinander H, F, Cl oder CN bedeuten.

X' ist vorzugsweise -O-, -S -CO-, -COO-, -OCO-, -O-COO-, -CO-NR⁰-, - NR⁰-CO-, -NR⁰-CO-NR⁰- oder eine Einfachbindung.

Typische Abstandsgruppen Sp' sind beispielsweise -(CH₂)p₁-, -(CH₂CH₂O)q₁ - CH₂CH₂-, -CH₂CH₂-S-CH₂CH₂-, -CH₂CH₂-NH-CH₂CH₂- oder -(SiR⁰⁰R⁰⁰⁰-O)ₚ₁-, worin p1 eine ganze Zahl von 1 bis 12 ist, q1 eine ganze Zahl von 1 bis 3 ist, und R⁰⁰ und R⁰⁰⁰ die oben angegebenen Bedeutungen besitzen.

Besonders bevorzugte Gruppen -Sp'-X'- sind -(CH₂)ₚ₁-, -(CH₂)p₁-O-, -(CH₂)ₚ₁-O-CO-, -(CH₂)ₚ₁-O-CO-O-, worin p1 und q1 die oben angebene Bedeutung haben.

Besonders bevorzugte Gruppen Sp' sind beispielsweise jeweils geradkettiges Ethylen, Propylen, Butylen, Pentylen, Hexylen, Heptylen, Octylen, Nonylen, Decylen, Undecylen, Dodecylen, Octadecylen, Ethylenoxyethylen, Methylenoxybutylen, Ethylenthioethylen, Ethylen-N-methyl-iminoethylen, 1-Methylalkylen, Ethenylen, Propenylen und Butenylen.

Ferner bevorzugt sind Gruppen Sp und Sp', welche eine oder mehrere C-C-Dreifachbindungen enthalten, insbesondere solche der Formel -(CH₂)ᵣ₁-C≡C- oder -C≡C-(CH₂)ᵣ₁-, worin r1 eine ganze Zahl von 1 bis 8, vorzugsweise 1, 2 oder 3 bedeutet.

In einer weiteren bevorzugten Ausführungsform der Erfindung bedeuten R^{a} und/oder R^{b} in Formel I einen Rest mit zwei oder mehreren polymerisierbaren Gruppen (multifunktionelle polymerisierbare Reste). Geeignete Reste dieses Typs, sowie diese enthaltende polymerisierbare Verbindungen und ihre Herstellung sind beispielsweise in US 7,060,200 B1 oder US 2006/0172090 A1 beschrieben. Besonders bevorzugt sind multifunktionelle polymerisierbare Reste ausgewählt aus folgenden Formeln

-X-alkyl-CHP¹-CH₂-CH₂P² I*a

-X-alkyl-C(CH₂P¹)(CH₂P²)-CH₂P³ I*b

-X-alkyl-CHP¹CHP²-CH₂P³ I*c

-X-alkyl-C(CH₂P¹)(CH₂P₂)-CₐₐH₂ₐₐ₊₁ I*d

-X-alkyl-CHP¹-CH₂P² I*e

-X-alkyl-CHP¹P² I*f

-X-alkyl-CP¹P²-CₐₐH₂ₐₐ₊₁ I*g

-X-alkyl-C(CH₂P¹)(CH₂P²)-CH₂OCH₂-C(CH₂P³)(CH₂P⁴)CH₂P⁵ I*h

-X-alkyl-CH((CH₂)ₐₐP¹)((CH₂)_{bb}P²) I*i

-X-alkyl-CHP¹CHP²-CₐₐH₂ₐₐ₊₁ I*k

-X'-alkyl-C(CH₃)(CH₂P¹)(CH₂P²) I*m

worin
- alkyl: eine Einfachbindung oder geradkettiges oder verzweigtes Alkylen mit 1 bis 12 C-Atomen bedeutet, worin eine oder mehrere nicht benachbarte CH₂-Gruppen jeweils unabhängig voneinander durch -C(R⁰⁰)=C(R⁰⁰⁰)-, -C≡C-, -N(R⁰⁰)-, -O-, -S-, - CO-, -CO-O-, -O-CO-, -O-CO-O- so ersetzt sein können, dass O-und/oder S-Atome nicht direkt miteinander verknüpft sind, und worin auch ein oder mehrere H-Atome durch F, Cl oder CN ersetzt sein können, wobei R⁰⁰ und R⁰⁰⁰ die oben angegebene Bedeutung haben,
- aa und bb: jeweils unabhängig voneinander 0, 1, 2, 3, 4, 5 oder 6 bedeuten,
- X: eine der für X' angegebenen Bedeutungen besitzt, und
- P¹⁻⁵: jeweils unabhängig voneinander eine der für P angegebenen Bedeutungen besitzen.

Besonders bevorzugte Verbindungen der Formel I sind solche der Unterformel IA 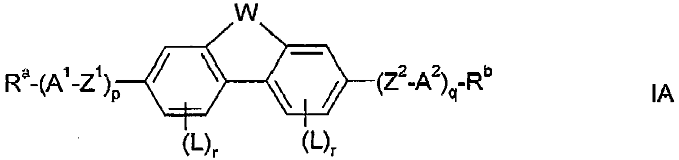 worin W, R^{a,b}, A^{1,2}, Z^{1,2}, L, p, q und r die in Formel I angegebene Bedeutung haben.

Bevorzugte Verbindungen der Formel I und IA sowie deren vor- und nachstehend angegebenen Unterformeln sind solche worin
- W -C(R^{c}R^{d})-, -CH₂CH₂- oder -CH₂-O- bedeutet,
- R^{c} und R^{d} jeweils unabhängig voneinander H, Alkyl mit 1 bis 12, vorzugsweise 5 bis 12 C-Atomen, Alkoxy mit 1 bis 12 C-Atomen oder Alkenyl mit 2 bis 11 C-Atomen bedeuten, wobei in allen diesen Resten auch ein oder mehrere H-Atome durch F ersetzt sein können, besonders bevorzugt Methyl,
- R^{c} und R^{d} H bedeuten,
- einer der Reste R^{c} und R^{d} H bedeutet und der andere von H verschieden ist,
- R^{a} und R^{b} gleiche oder verschiedene Reste P-Sp- bedeuten,
- R^{a} und R^{b} P-Sp- bedeuten, wobei in einem der Reste R^{a} und R^{b} Sp eine Einfachbindung bedeutet und in dem anderen der Reste R^{a} und R^{b} Sp von einer Einfachbindung verschieden ist und vorzugsweise eine Gruppe der Formel Sp'-X' bedeutet, so dass dieser Rest P-Spder Formel P-Sp'-X'- entspricht,
- R^{a} und R^{b} gleiche oder verschiedene Reste P-Sp- bedeuten, worin beide Reste Sp eine Einfachbindung bedeuten,
- einer der Reste R^{a} und R^{b} eine Gruppe P-Sp- bedeutet oder enthält, und der andere eine unpolymerisierbare Gruppe bedeutet, vorzugsweise ausgewählt aus geradkettigem oder verzweigtem Alkyl mit 1 bis 25 C-Atomen, worin auch eine oder mehrere nicht benachbarte CH₂-Gruppen jeweils unabhängig voneinander durch -C(R⁰⁰)=C(R⁰⁰⁰)-, -C≡C-, -N(R⁰⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, Br, I oder CN ersetzt sein können,
- R^{a} P-Sp- bedeutet,
- R^{b} P-Sp- bedeutet,
- Sp eine Einfachbindung bedeutet,
- Sp einen Rest ausgewählt aus der Gruppe bestehend aus -(CH₂)ₚ₁-, -(CH₂)ₚ₁-O-, -(CH₂)ₚ₁-O-CO-, -(CH₂)ₚ₁-O-CO-O-, -(CH₂)ᵣ₁-C≡C- oder -C≡C-(CH₂)ᵣ₁-, vorzugsweise -(CH₂)ₚ₁- oder -(CH₂)ₚ₁-O- bedeutet, worin p1 eine ganze Zahl von 1 bis 12 und r1 eine ganze Zahl von 1 bis 8 bedeutet,
- L keine polymerisierbare Gruppe bedeutet oder enthält,
- r 1 bedeutet,
- L F bedeutet,
- A¹ und A² ausgewählt sind aus der Gruppe bestehend aus 1,4-Phenylen und Naphthalin-2,6-diyl, wobei in diesen Ringen auch eine oder zwei CH-Gruppen durch N ersetzt sein können, wobei diese Ringe ein- oder mehrfach durch L wie vor- und nachstehend beschrieben substituiert sein können,
- Z¹ und Z² ausgewählt sind aus der Gruppe bestehend aus -O-, -CO-O-, -OCO-, -OCH₂-, -CH₂O-, -CF₂O-, -OCF₂-, -CH₂CH₂-, -CH=CH-, -CF=CF-, -CH=CF-, -CF=CH-, -C≡C-, Einfachbindung,
- p 0 oder 1, vorzugsweise 0 bedeutet,
- q 0 oder 1, vorzugsweise 0 bedeutet,
- L eine unpolymerisierbare Gruppe ist, vorzugsweise ausgewählt aus F, Cl, -CN und geradkettigem oder verzweigtem Alkyl mit 1 bis 25, besonders bevorzugt 1 bis 10 C-Atomen, worin auch eine oder mehrere nicht benachbarte CH₂-Gruppen jeweils unabhängig voneinander durch -C(R⁰⁰)=C(R⁰⁰⁰)-, -C≡C-, -N(R⁰⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, Br, I oder CN ersetzt sein können.

Besonders bevorzugte Verbindungen der Formel I und IA sind ausgewählt aus der Gruppe bestehend aus den folgenden Unterformeln 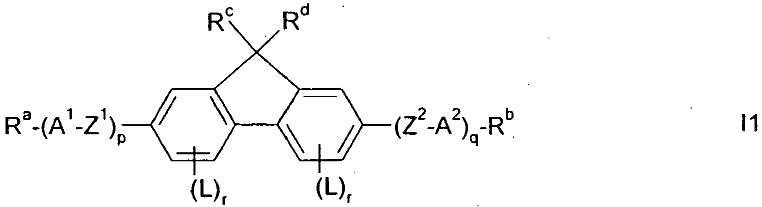 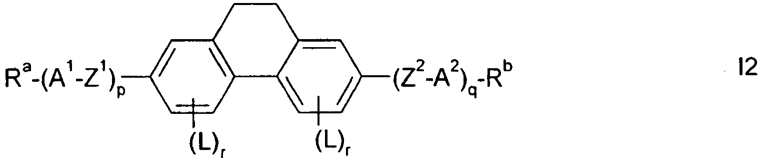 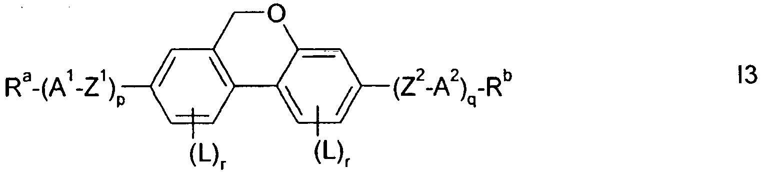 worin R^{a-d}, R^{1,2}, A^{1,2}, Z^{1,2}, L, p, q nd r jeweils unabhängig voneinander eine der in Formel I oder vor- und nachstehend angegebenen Bedeutungen besitzen.

Die Gruppen -(A¹-Z¹)ₚ- und -(Z²-A²)_{q}- in den Verbindungen der Formeln I, IA, I1, I2 und I3 bedeuten vorzugsweise 1,4-Phenylen oder Naphthalin-2,6-diyl, wobei in diesen Ringen auch eine oder zwei CH-Gruppen durch N ersetzt sein können, und wobei diese auch Ringe ein- oder mehrfach durch L wie vor- und nachstehend beschrieben substituiert sein können.

Besonders bevorzugt sind solche Verbindungen der Formeln I1 bis I3, worin p und q 0 bedeuten, sowie solche, worin einer der Indices p und q 0 und der andere 1 bedeutet.

Weiterhin besonders bevorzugt sind solche Verbindungen der Formeln I1 bis 13, worin die Reste R^{a} und R^{b}P-Sp- bedeuten, sowie solche, worin einer der Reste R^{a} und R^{b}, vorzugsweise R^{a}, P-Sp- bedeutet und der andere eine unpolymerisierbare Gruppe bedeutet, vorzugsweise ausgewählt aus geradkettigem oder verzweigtem Alkyl mit 1 bis 25 C-Atomen, worin auch eine oder mehrere nicht benachbarte CH₂-Gruppen jeweils unabhängig voneinander durch -C(R⁰⁰)=C(R⁰⁰⁰)-, -C≡C-, -N(R⁰⁰)-, -O-, -S-, -CO-, -CO-O-, -O-CO-, -O-CO-O- so ersetzt sein können, dass O-und/oder S-Atome nicht direkt miteinander verknüpft sind, und worin auch ein oder mehrere H-Atome durch F, Cl, Br, I oder CN ersetzt sein können.

Weiterhin besonders bevorzugt sind solche Verbindungen der Formeln I1 bis 13, worin beide Reste R^{a} und R^{b}P-Sp- bedeuten, wobei eine Gruppe Sp eine Einfachbindung bedeutet und die andere Gruppe Sp von einer Einfachbindung verschieden ist.

Ganz besonders bevorzugte Verbindungen der Formeln I1 bis I3 sind ausgewählt aus der Gruppe bestehend aus den folgenden Unterformeln 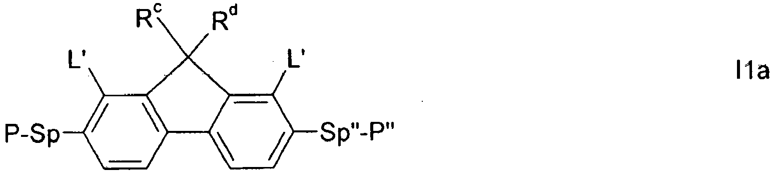 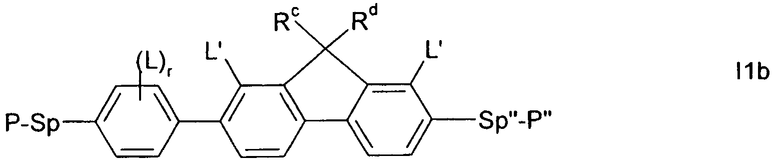 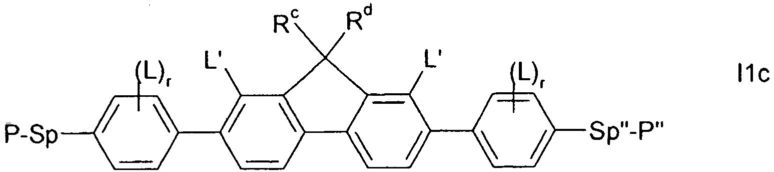 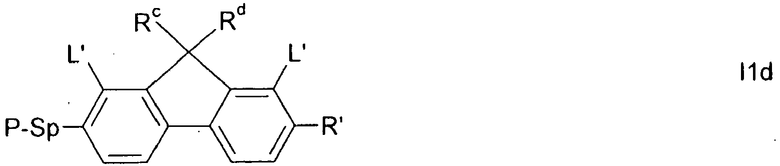 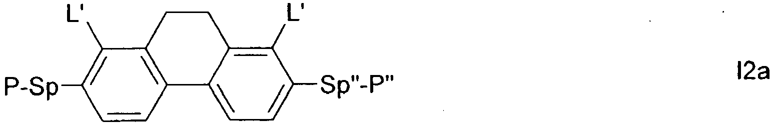 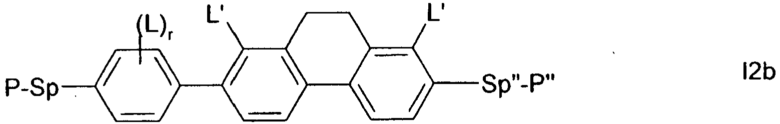 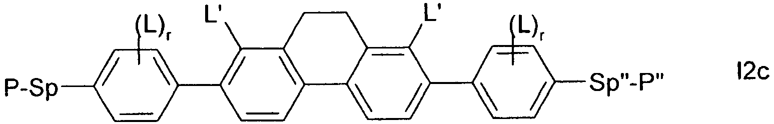 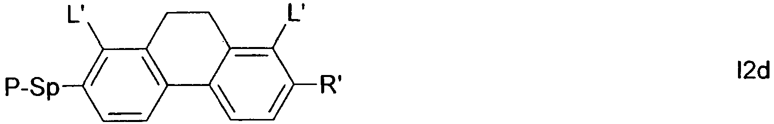 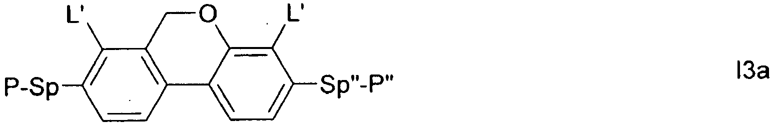 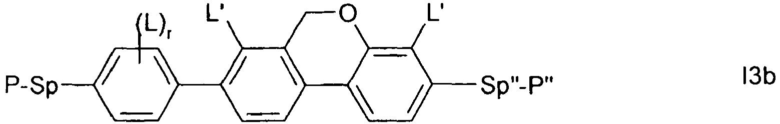 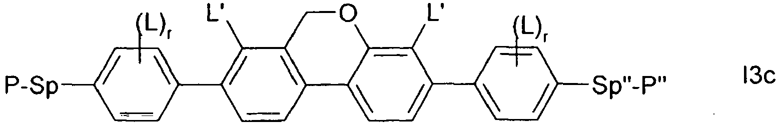 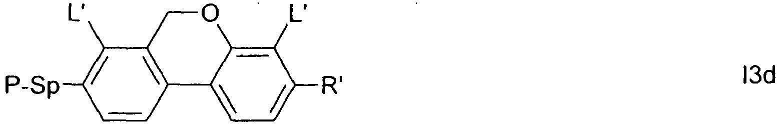 worin R^{c} und R^{d} die vor- und nachstehend angegebenen Bedeutungen besitzen, L' H oder F bedeutet, P und Sp eine der in Formel I oder vor- und nachstehend angegebenen Bedeutungen besitzen, P" eine der in Formel I oder vor- und nachstehend für P angegebenen Bedeutungen besitzt, Sp" eine der in Formel I oder vor- und nachstehend für Sp angegebenen Bedeutungen besitzt, und R' eine der in Formel I oder vor- und nachstehend für R^{a} angegebenen Bedeutungen besitzt, wobei R' von H verschieden ist und keine Gruppe P-Sp- bedeutet oder enthält.

P und P" bedeuten in den Verbindungen der Formeln I, IA, I1 bis I3 sowie deren Unterformeln vorzugsweise eine Acrylat-, Fluoracrylat- oder Methacrylatgruppe.

Sp und Sp" bedeuten Verbindungen der Formeln I, IA I1 bis I3 sowie deren Unterformeln vorzugsweise -(CH₂)ₚ₁-, -O-(CH₂)ₚ₁-, -(CH₂)ₚ₁-O-, -O-CO-(CH₂)ₚ₁-, -(CH₂)ₚ₁-O-CO-, -O-CO-O-(CH₂)ₚ₁-, -(CH₂)ₚ₁-O-CO-O-, -(CH₂)ᵣ₁-C≡C- oder -C≡C-(CH₂)ᵣ₁- oder eine Einfachbindung, worin p1 eine ganze Zahl von 1 bis 12, vorzugsweiswe von 1 bis 6, und r1 eine ganze Zahl von 1 bis 8, vorzugsweise 1, 2 oder 3 bedeuten, wobei diese Gruppen so mit P oder P" verknüpft sind, dass O-Atome nicht direkt miteinander verknüpft sind.

Besonders bevorzugt sind Verbindungen der oben gezeigten Unterformeln I1a-I3d, worin einer der beiden Reste L' F und der andere H bedeutet.

Ferner bevorzugt sind Verbindungen der oben gezeigten Unterformeln I1a-I3d, worin einer der Reste Sp und Sp", vorzugsweise Sp, eine Einfachbindung bedeutet und der andere der Reste Sp und Sp", vorzugsweise Sp", von einer Einfachbindung verschieden ist.

Ein weiterer Gegenstand der Erfindung sind neue Verbindungen der Formeln I und IA sowie deren Unterformeln wie in Anspruch 12, 13, 14, 15 oder 16 definiert.

Zwischenprodukte zur Herstellung von Verbindungen der Formel I und IA sind ausgewählt aus folgender Formel 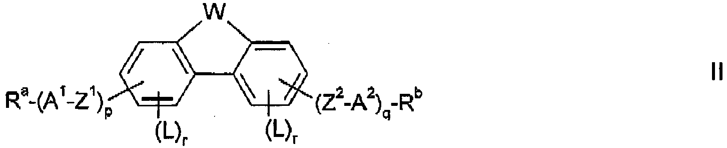 worin W, A^{1,2}, Z^{1,2}, L, p, q und r die in Formel I oder vor- und nachstehend angegebene Bedeutung besitzen, und die Reste R^{a} und R^{b} jeweils unabhängig voneinander -Sp-O-Sg bedeuten, wobei Sp die in Formel I oder vor- und nachstehend angegebene Bedeutung besitzt, und Sg ein H-Atom oder eine Schutzgruppe bedeutet.

Geeignete Schutzgruppen Sg sind dem Fachmann bekannt. Bevorzugte Schutzguppen sind Alkyl, Acyl und Alkylsilyl- oder Arylsilylgruppen, 2-Tetrahydropyranyl oder Methoxymethyl.

Besonders bevorzugte Zwischenprodukte der Formel II sind ausgewählt aus der Gruppe bestehend aus folgenden Unterformeln 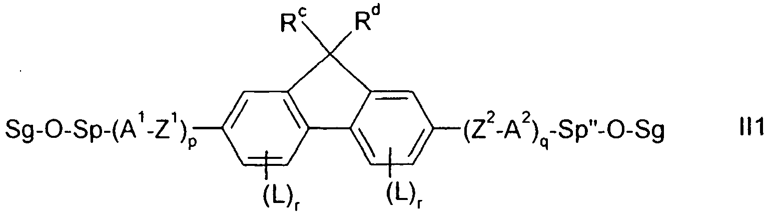 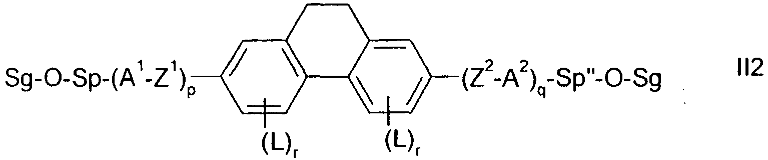 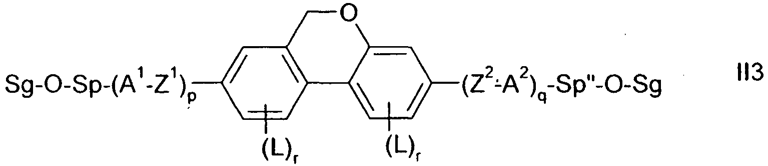 worin R^{c,d}, A^{1,2}, Z^{1,2}, L, p, q, r, Sp, und Sg die in Formel II angegebenen Bedeutungen besitzen, Sp" eine der für Sp angegebenen Bedeutungen besitzt, und Sg besonders bevorzugt H bedeutet.

Vorzugsweise bedeuten in den Verbindungen der Formeln II1-II3, vor allem in denen der Formel II1, einer der Reste Sp und Sp", vorzugsweise Sp, eine Einfachbindung und der andere, vorzugsweise Sp", eine von einer Einfachbindung verschiedene Gruppe.

Unter diesen bevorzugten Verbindungen sind solche besonders bevorzugt, worin einer der Reste Sp und Sp", vorzugsweise Sp, eine Einfachbindung und der andere, vorzugsweise Sp", -(CH₂)ₚ₁- bedeuten, wobei p1 wie oben definiert ist.

Ganz besonders bevorzugte Zwischenprodukte der Formel II sind ausgewählt den Unterformeln I1a-I1c, I2a-I2c und I3a-I3c wie oben angegeben, worin P Sg-O und P" O-Sg bedeuten.

Weitere Zwischen-produkte zur Herstellung von Verbindungen der Formel I2 sind ausgewählt aus folgender Formel 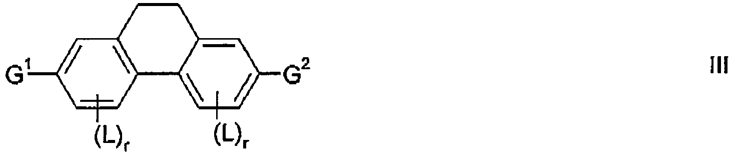 worin L und r die in Formel I angegebene Bedeutung besitzen und G¹ und G² gleiche oder verschiedene Reste ausgewählt aus Cl, Br, I und OH bedeuten. Besonders bevorzugt sind solche Verbindungen der Formel III worin G¹ und G² gleiche oder verschieden Reste Br oder I bedeuten, solche worin eine der Gruppen G¹ und G² I und die andere Br oder OH, bedeutet, sowie solche worin G¹ und G² OH bedeuten.

Ein weiterer Gegenstand der Erfindung sind neue Zwischenprodukte wie in Anspruch 20 oder 21 definiert.

Besonders geeignete und bevorzugte Verfahren zur Herstellung von Verbindungen und Zwischenprodukten der Formeln I, IA, II und III sind in folgenden Schemata beispielhaft dargestellt und enthalten vorzugsweise einen oder mehrere der nachfolgend beschriebenen Schritte.

Die Verbindungen und Zwischenprodukte der Formel I, IA, II und III sowie deren Unterformeln können in Analogie zu dem Fachmann bekannten und in Standardwerken der organischen Chemie beschriebenen Verfahren, wie beispielsweise in Houben-Weyl, Methoden der organischen Chemie, Thieme-Verlag, Stuttgart, hergestellt werden.

Beispielsweise erfolgt die Synthese von Verbindungen der Formel I und IA durch Veresterung oder Veretherung der Zwischenprodukte der Formel II mit entsprechenden Säuren, Säurederivaten, oder halogenierten Verbindungen enthaltend eine Gruppe P. Wie in Schema 1 beispielhaft dargestellt, können Ester **2a** der Acrylsäure oder Methacrylsäure durch Veresterung der entsprechenden Alkohole **3** mit Säurederivaten wie zum Beispiel (Meth)acrylsäurechlorid oder (Meth)acrylsäureanhydrid in Gegenwart einer Base erhalten werden. Weiterhin können die Alkohole **3** auch mit (Meth)acrylsäure in Gegenwart eines wasserentziehenden Mittels verestert werden, beispielsweise nach Steglich mit Dicyclohexylcarbodiimid (DCC), oder durch Behandlung der entsprechenden Alkohole mit (Meth)acrylsäureanhydrid in Gegenwart einer Base und 4-(N,N-Dimethylamino)pyridin (DMAP).

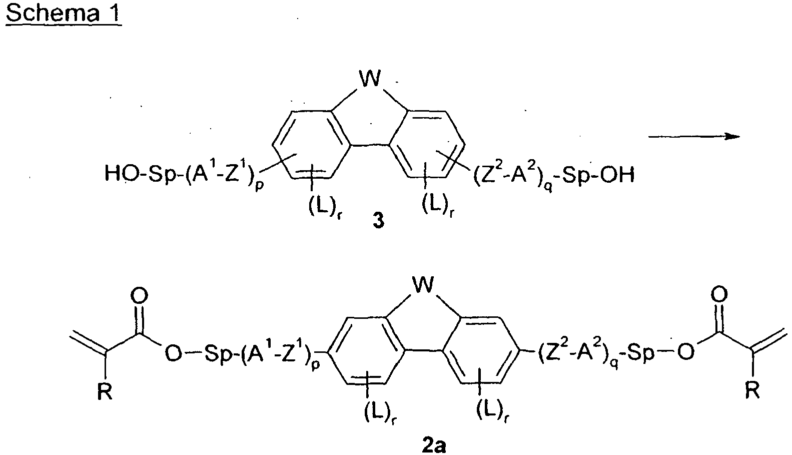

Ein Zugang zum Grundkörper 9,10-Dihydrophenanthrenen (Formel 2, worin W = CH₂CH₂) ist durch Hydrierung von Phenanthren gegeben, und läßt sich durch Halogenierung selektiv funktionalisieren, wie in Schema 2 beispielhaft dargestellt. Nach A. D. Abell et al., J. Chem. Soc., Perkin Trans. 1, 1997, 1663 - 1668 erhält man aus 9,10-Dihydrophenanthren **4** das 2,7-Dibrom-9,10-dihydrophenanthren (Formel **5**, worin Hal = Br). Iodierung nach H. Suzuki, Organic Syntheses, Coll. Vol. 6, S.700 (1988) liefert das entsprechende 2,7-Diiod-9,10-dihydrophenanthren (Formel **5**, worin Hal = Br). Das bisher nicht literaturbekannte 2-Brom-7-iod-9,10-dihydrophenanthren (Formel **5**, worin ein Rest Hal Br und der andere I bedeutet) ist analog durch Monoiodierung und anschließende Bromierung selektiv zugänglich und eröffnet so die möglichkeit einer gezielten Abwandlung zu einer Vielzahl von Derivaten.

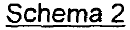

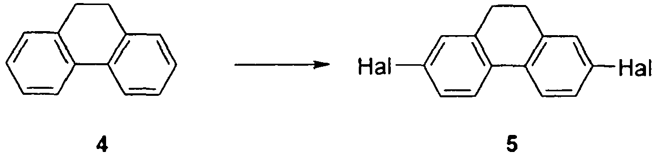

Aus den Dihalogenverbindungen **5** lassen sich z.B. durch Metallierung mit Butyllithium und Umsetzung mit Borsäureestern Boronsäuren herstellen, die zu den Phenolen **6** oxidiert werden können, wie in Schema 3 beispielhaft dargestellt. Eine Alternative ist die Behandlung mit KOH in Gegenwart von Palladiumkatalysatoren nach S. L. Buchwald et al. J. Am. Chem. Soc. 2006, 128, 10694-10695. Veresterung mit Acrylsäuren liefert die Acrylate **7**.

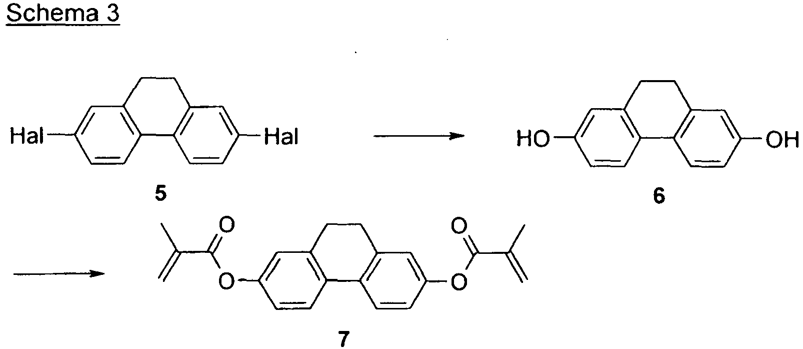

Durch Monometallierung in Gegenwart von Triisopropylborat können Monohydroxyverbindungen **8** erhalten werden, die in einem zweiten Schritt weiter abgewandelt werden können, wie in Schema 4 beispielhaft dargestellt. So liefert Sonogashira-Kupplung mit terminalen Alkinolen die Alkine **9**, aus denen durch Hydrierung und Veresterung die Verbindungen **10** mit einer Spacergruppe zugänglich sind.

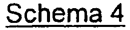

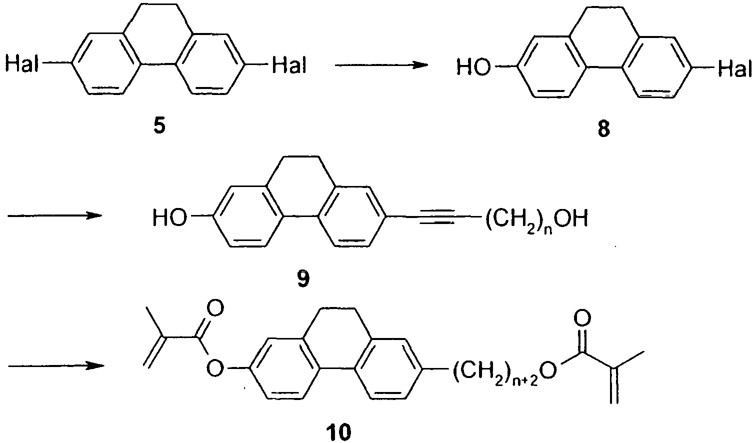

Entsprechend sind aus kommerziell erhältlichem Dibromfluoren **11** die Fluorenderivate **12** zugänglich, wie in Schema 5 beispielhaft dargestellt (worin R H oder CH₃ bedeutet), wobei ggf. aus **11** nach G. R. Bebernitz et al., J. Med. Chem. 2001, 44; 2601 - 2611, die entsprechenden alkylierten Derivate erhalten werden. Zur alternativen Herstellung von 2,7-Diihydroxyfluorenen durch Friedel-Crafts-Acylierung und anschließende Baeyer-Vllliger-Oxidation s. R. P. Lemieux et al., J. Materials Chem. 2008, 18(28), 3361-3365.

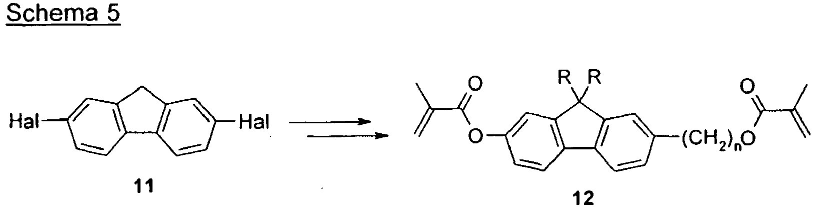

Ein Zugang zu den erfindungsgemäßen Benzochromenen (Formel **1**, worin W = CH₂O) ist nach Taugerbeck, Klasen-Memmer, WO2004076438, durch Reduktion der Benochromenone (**2**, worin W = C(O)O) gegeben. Zur Herstellung der Benzochromenone s.a. z.B. S. Kim et al. Organic Lett. 2005, 7, 411-414; die dort beschriebene Verbindung **13** kann nach Etherspaltung mit z.B. Bortribromid über **14** in die Acrylate **15** übergeführt werden, wie in Schema 6 beispielhaft dargestellt. Entsprechend sind aus **16** die Acrylate **18** zugänglich.

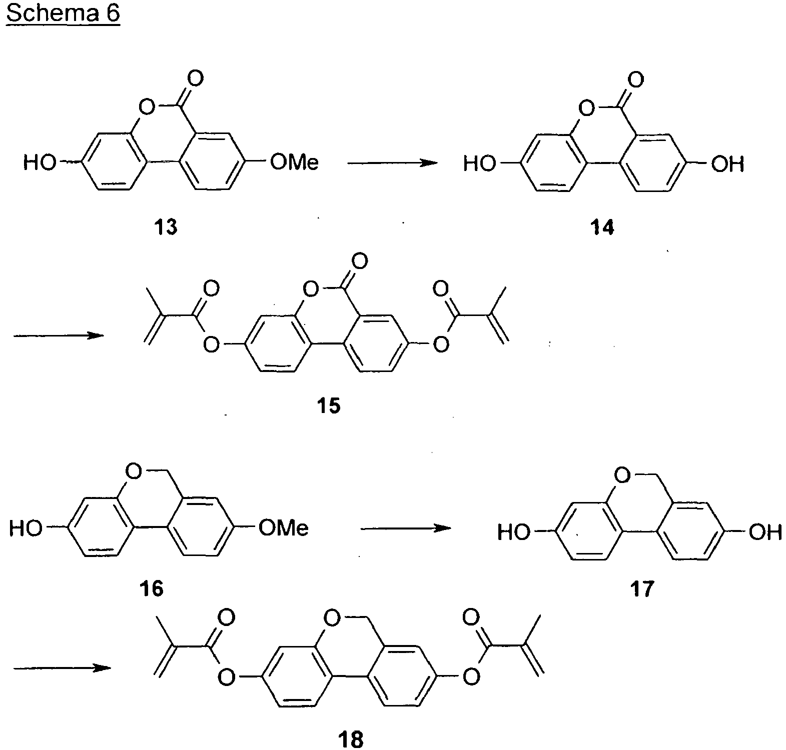

Spacergruppen können auch hier wie in Schema 7 gezeigt eingeführt werden, indem aus den Hydroxyverbindungen **20** durch Behandeln mit Trifluormethansulfonsäureanhydrid zunächst das Triflat **21** hergestellt wird, welches dann analog Schema **4** in einer Sonogashira-Reaktion umgesetzt wird.

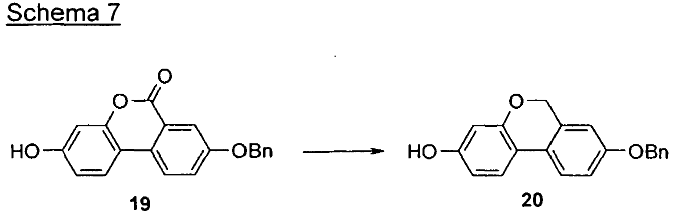

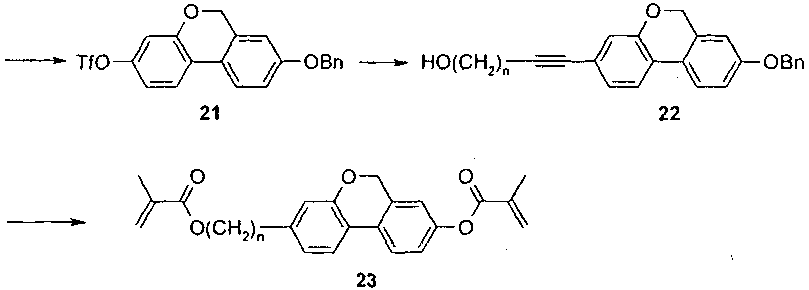

Die in den oben gezeigten Schemata aufgeführten Zwischenverbindungen können weiterhin durch eine Vielzahl von Standardreaktionen zu den erfindungsgemäßen Verbindungen abgewandelt werden, wie in Schema 8 beispielhaft dargestellt (worin die Reste W, X, Sp wie vor- und nachstehend definiert sind, G = H-Atom oder Schutzgruppe, und M = B(OH)₂, ZnHal oder MgHal). Beispielsweise sind durch Übergangsmetallkatalysierte Kupplungsreaktionen wie der Suzuki-Kupplung (**24**, M = - B(OH)₂) aus Arylmetallverbindungen Arylderivate **26** zugänglich, die analog zu den obigen Schemata zu den Zielverbindungen **27** abgewandelt werden können.

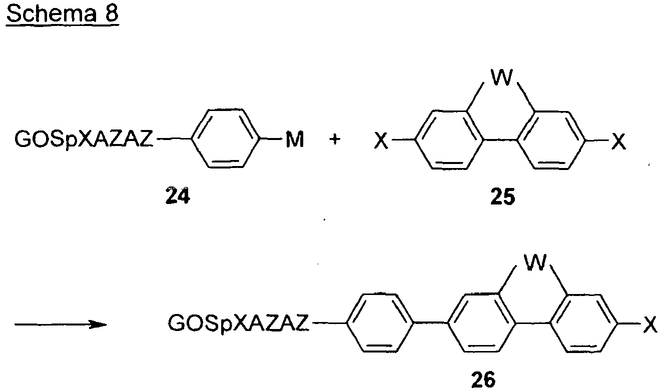

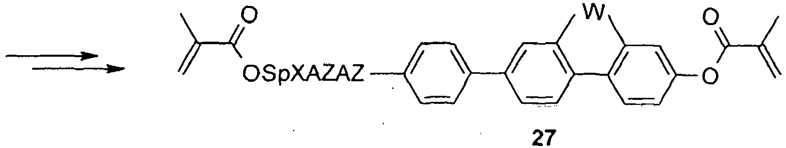

Zur Herstellung von PSA-Anzeigen werden die polymerisierbaren Verbindungen im FK-Medium zwischen den Substraten der FK-Anzeige unter Anlegen einer Spannung durch in-situ-Polymerisation polymerisiert oder vernetzt (falls eine Verbindung zwei oder mehr polymerisierbare Gruppen enthält). Die Polymerisation kann in einem Schritt durchgeführt werden. Es ist auch möglich, zunächst in einem ersten Schritt die Polymerisation unter Anlegen einer Spannung durchzuführen, um einen pretilt-Winkel zu erzeugen, und anschließend in einem zweiten Polymerisationsschritt ohne anliegende Spannung die im ersten Schritt nicht abreagierten Verbindungen zu polymerisieren bzw. zu vernetzen ("end curing").

Geeignete und bevorzugte Polymerisationsmethoden sind beispielsweise die thermische oder Photopolymerisation, vorzugsweise Photopolymerisation, insbesondere UV-Photopolymerisation. Dabei können gegebenfalls auch ein oder mehrere Initiatoren zugesetzt werden. Geeignete Bedingungen für die Polymerisation, sowie geeignete Arten und Mengen der Initiatoren, sind dem Fachmann bekannt und in der Literatur beschrieben. Für die radikalische Polymerisation eignen sich zum Beispiel die kommerziell erhältlichen Photoinitiatoren Irgacure651®, Irgacure184®, Irgacure907®, Irgacure369®, oder Darocure1173® (Ciba AG). Falls ein Initiator eingesetzt wird, beträgt dessen Anteil vorzugsweise 0,001 bis 5 Gew.-%, besonders bevorzugt 0,001 bis 1 Gew.-%. Die Polymerisation kann aber auch ohne Zusatz eines Initiators erfolgen. In einer weiteren bevorzugten Ausführungsform enthält das FK-Medium keinen Polymerisationsinitiator.

Die polymerisierbare Komponente A) oder das FK-Medium können auch einen oder mehrere Stabilisatoren enthalten, um eine unerwünschte spontane Polymerisation der RMs, beispielsweise während der Lagerung oder des Transports, zu verhindern. Geeignete Arten und Mengen der Stabilisatoren sind dem Fachmann bekannt und in der Literatur beschrieben. Besonders geeignet sind zum Beispiel die kommerziell erhältlichen Stabilisatoren der Serie Irganox® (Ciba AG), wie beispielsweise Irganox® 1076. Falls Stabilisatoren eingesetzt werden, beträgt deren Anteil, bezogen auf die Gesamtmenge der RMs beziehungsweise der polymerisierbaren Komponente A), vorzugsweise 10 - 10,000 ppm, besonders bevorzugt 50 - 500 ppm.

Die erfindungsgemäßen polymerisierbaren Verbindungen eignen sich auch für die Polymerisation ohne Initiator, was erhebliche Vorteile mit sich bringt, wie beispielsweise geringere Materialkosten und insbesondere eine geringere Verunreinigung des FK-Mediums durch mögliche Restmengen des Initiators oder dessen Abbauprodukte.

Die erfindungsgemäßen FK-Medien zur Verwendung in PSA-Anzeigen enthalten vorzugsweise < 5 Gew.-%, besonders bevorzugt < 1 Gew.-%, ganz besonders bevorzugt < 0.5 Gew.-% an polymerisierbaren Verbindungen, insbesondere polymerisierbaren Verbindungen der oben genannten Formeln.

Besonders bevorzugt sind FK-Medien enthaltend eine, zwei oder drei polymerisierbare Verbindungen der Formel I.

Ferner bevorzugt sind FK-Medien, worin die polymerisierbare Komponente (Komponente A) ausschließlich polymerisierbare Verbindungen der Formel I enthält.

Ferner bevorzugt sind FK-Medien, worin Komponente B) eine FK-Verbindung oder eine FK-Mischung ist, die eine nematische Flüssigkristallphase aufweist.

Ferner bevorzugt sind achirale polymerisierbare Verbindungen der Formel I, sowie FK-Medien worin die Verbindungen der Komponente A) und/oder B) ausschließlich aus der Gruppe bestehend aus achiralen Verbindungen ausgewählt sind.

Ferner bevorzugt sind FK-Medien, worin die polymerisierbare Komponente bzw. Komponente A) eine oder mehrere polymerisierbare Verbindungen der Formel I mit einer polymerisierbaren Gruppe (monoreaktiv) und eine oder mehrere polymerisierbare Verbindungen der Formel I mit zwei oder mehr, vorzugsweise zwei polymerisierbaren Gruppen (di- oder multireaktiv) enthält.

Ferner bevorzugt sind PSA-Anzeigen und FK-Medien, worin die polymerisierbare Komponente bzw. Komponente A) ausschließlich polymerisierbare Verbindungen der Formel I mit zwei polymerisierbaren Gruppen (direaktiv) enthält.

Der Anteil der polymerisierbaren Komponente bzw. Komponente A) in den erfindungsgemäßen FK-Medien ist vorzugsweise < 5%, besonders bevorzugt < 1%, ganz besonders bevorzugt < 0.5 %.

Der Anteil der flüssigkristallinen Komponente bzw. Komponente B) in den erfindungsgemäßen FK-Medien ist vorzugsweise > 95%, besonders bevorzugt > 99%.

Die polymerisierbaren Verbindungen der Formel I können einzeln polymerisiert werden, es können aber auch Mischungen polymerisiert werden, welche zwei oder mehr Verbindungen der Formel I enthalten, oder Mischungen enthaltend eine oder mehrere Verbindungen der Formel I und eine oder mehrere weitere polymerisierbare Verbindungen (Comonomere), welche vorzugsweise mesogen oder flüssigkristallin sind. Bei Polymerisation solcher Mischungen entstehen Copolymere. Die vor- und nachstehend genannten polymerisierbaren Mischungen sind ein weiterer Gegenstand der Erfindung. Die polymerisierbaren Verbindungen und Comonomere sind mesogen oder nicht-mesogen, vorzugsweise mesogen oder flüssigkristallin.

Geeignete und bevorzugte mesogene Comonomere, besonders für die Verwendung in PSA-Anzeigen, sind beispielsweise ausgewählt aus den folgenden Formeln: 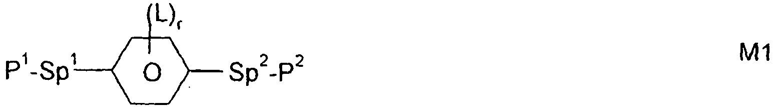 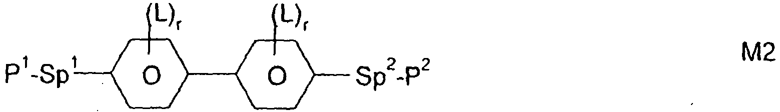 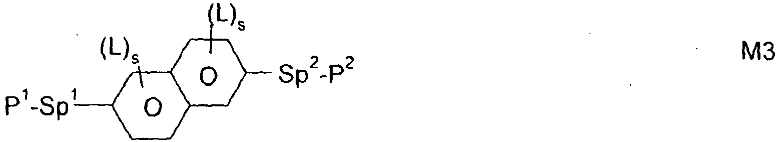 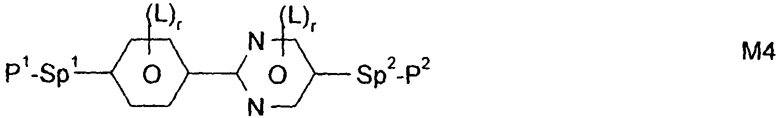 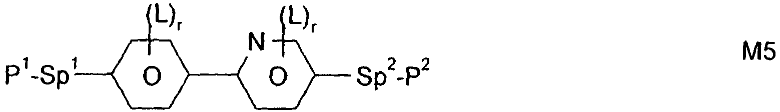 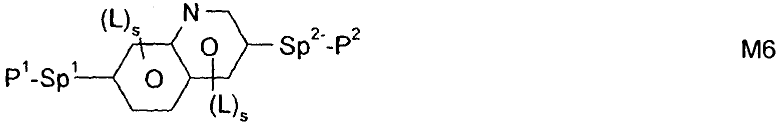 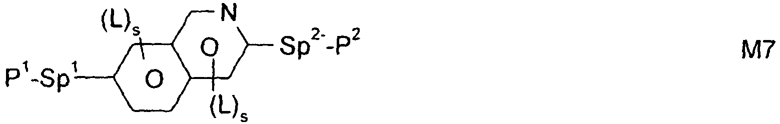 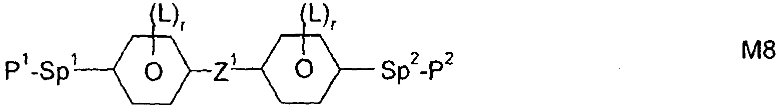 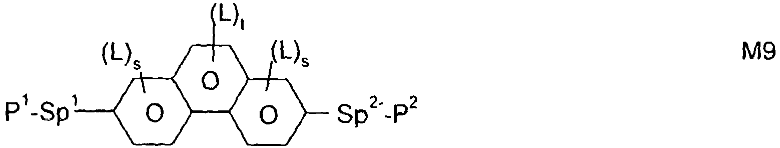 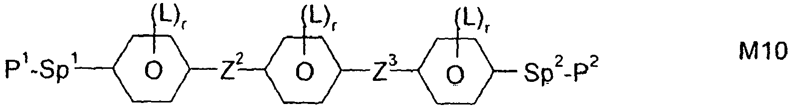 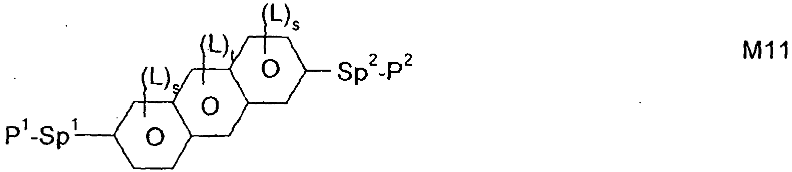 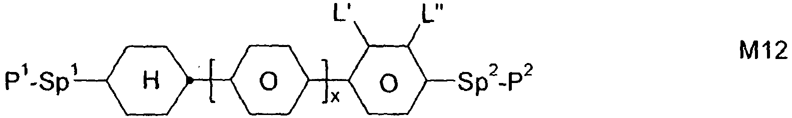 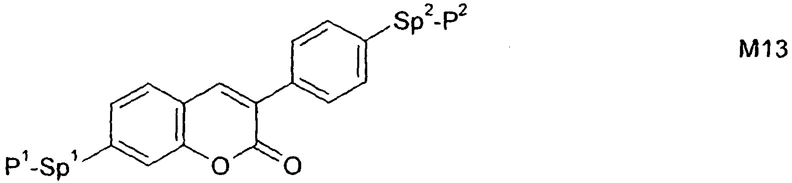 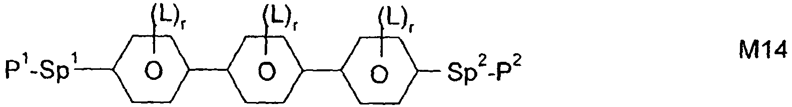 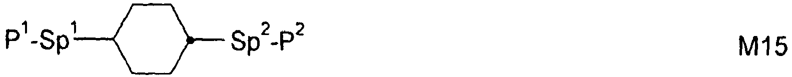 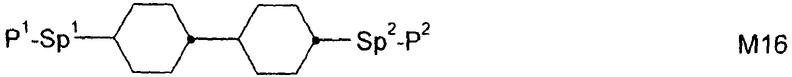 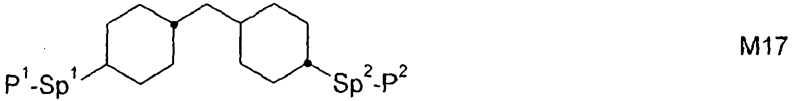 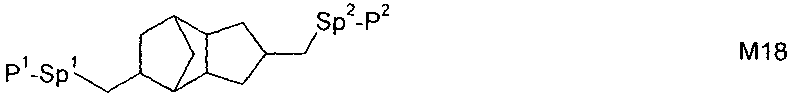 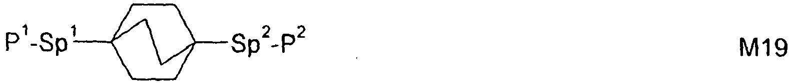 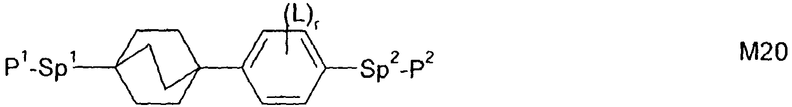 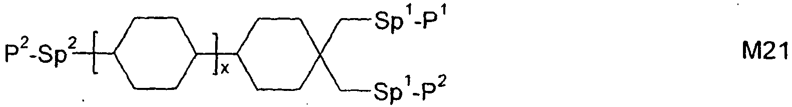 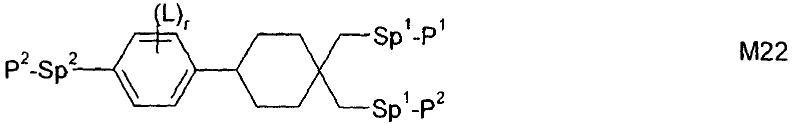 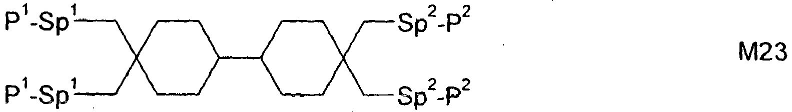 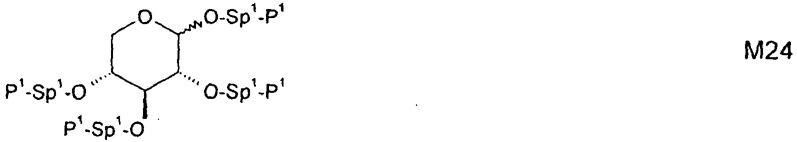 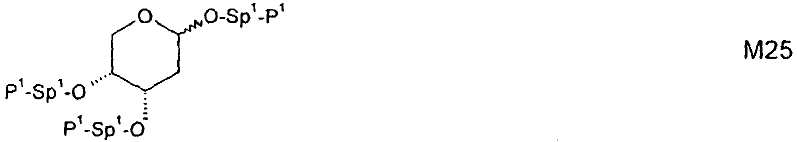 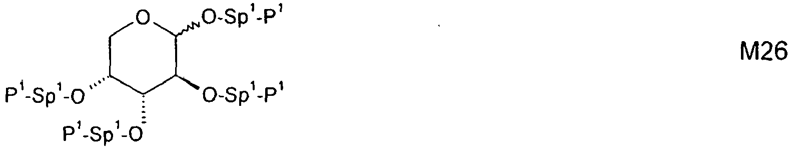 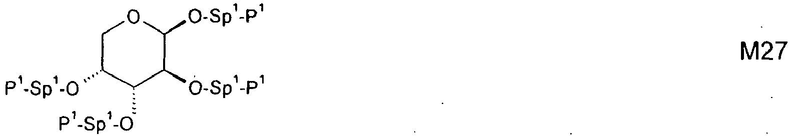 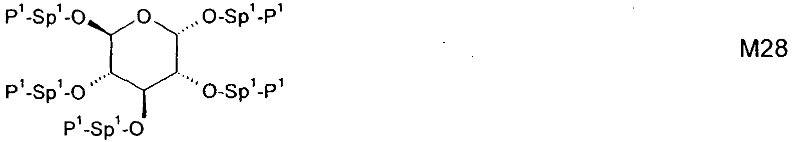 worin die einzelnen Reste folgende Bedeutung besitzen:
- P¹ und P²: jeweils unabhängig voneinander eine polymerisierbare Gruppe, vorzugsweise mit einer der vor- und nachstehend für P angegebenen Bedeutungen, besonders bevorzugt eine Acrylat-, Methacrylat-, Fluoracrylat-, Oxetan-, Vinyloxy- oder Epoxygruppe,
- Sp¹ und Sp²: jeweils unabhängig voneinander eine Einfachbindung oder eine Abstandsgruppe, vorzugsweise mit einer der vor- und nachstehend für Sp angegebenen Bedeutungen, und besonders bevorzugt -(CH₂)ₚ₁-, -(CH₂)ₚ₁-O-, -(CH₂)ₚ₁-CO-O-oder -(CH₂)ₚ₁-O-CO-O- bedeuten, worin p1 eine ganze Zahl von 1 bis 12 ist, und wobei in den letztgenannten Gruppen die Verknüpfung zur benachbarten Ring über das O-Atom erfolgt,
wobei auch einer oder mehrere der Reste P¹-Sp¹- und P²-Sp²- R^{aa} bedeuten können, mit der Maßgabe dass mindestens einer der vorhandenen Reste P¹-Sp¹- und P²-Sp²- nicht R^{aa} bedeutet,
- R^{aa}: H, F, Cl, CN oder geradkettiges oder verzweigtes Alkyl mit 1 bis 25 C-Atomen, worin auch eine oder mehrere nicht benachbarte CH₂-Gruppen jeweils unabhängig voneinander durch C(R⁰)=C(R⁰⁰)-, -C≡C-, -N(R⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, CN oder P¹-Sp¹- ersetzt sein können, besonders bevorzugt geradkettiges oder verzweigtes, optional ein- oder mehrfach fluoriertes, Alkyl, Alkoxy, Alkenyl, Alkinyl, Alkylcarbonyl, Alkoxycarbonyl, Alkylcarbonyloxy oder Alkoxycarbonyloxy mit 1 bis 12 C-Atomen (wobei die Alkenyl- und Alkinylreste mindestens zwei und die verzweigten Reste mindestens drei C-Atome aufweisen),
- R⁰, R⁰⁰: jeweils unabhängig voneinander und bei jedem Auftreten gleich oder verschieden H oder Alkyl mit 1 bis 12 C-Atomen,
- R^{y} und R^{z}: jeweils unabhängig voneinander H, F, CH₃ oder CF₃,
- Z¹: -O-, -CO-, -C(R^{y}R^{z})-, oder -CF₂CF₂-,
- Z² und Z³: jeweils unabhängig voneinander -CO-O-, -O-CO-, -CH₂O-, -OCH₂-, -CF₂O-, -OCF₂-, oder -(CH₂)ₙ-, wobei n 2, 3 oder 4 ist,
- L: bei jedem Auftreten gleich oder verschieden F, Cl, CN, oder geradkettiges oder verzweigtes, optional ein- oder mehrfach fluoriertes, Alkyl, Alkoxy, Alkenyl, Alkinyl, Alkylcarbonyl, Alkoxycarbonyl, Alkylcarbonyloxy oder Alkoxycarbonyloxy mit 1 bis 12 C-Atomen vorzugsweise F,
- L' und L": jeweils unabhängig voneinander H, F oder Cl,
- r: 0, 1, 2, 3 oder 4,
- s: 0, 1, 2 oder 3,
- t: 0, 1 oder 2,
- x: 0 oder 1.

Die FK-Medien zur Verwendung in den erfindungsgemäßen FK-Anzeigen enthalten, neben den oben beschriebenen polymerisierbaren Verbindungen, eine FK-Mischung ("Host-Mischung") enthaltend eine oder mehr, vorzugsweise zwei oder mehr niedermolekulare (d.h. monomere bzw. unpolymerisierte) Verbindungen. Letztere sind stabil bzw. unreaktiv gegenüber einer Polymerisationsreaktion unter den zur Polymerisation der polymerisierbaren Verbindungen verwendeten Bedingungen. Prinzipiell eignet sich als Host-Mischung jede zur Verwendung in herkömmlichen VA- und OCB-Anzeigen geeignete FK-Mischung. Geeignete FK-Mischungen sind dem Fachmann bekannt und in der Literatur beschrieben, beispielsweise Mischungen in VA-Anzeigen in EP 1 378 557 A1, und Mischungen für OCB-Anzeigen in EP 1 306 418 A1 und DE 102 24 046 A1.

Besonders bevorzugte FK-Anzeigen, FK-Host-Mischungen und FK-Medien werden im Folgenden genannt:
a) FK-Medium, welches eine oder mehrere Verbindungen der Formel CY und/oder PY enthält: 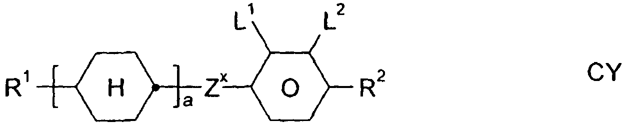 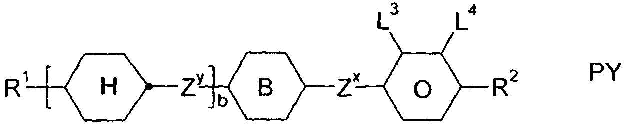 worin die einzelnen Reste folgende Bedeutung besitzen
   - a: 1 oder 2,
   - b: 0 oder 1,

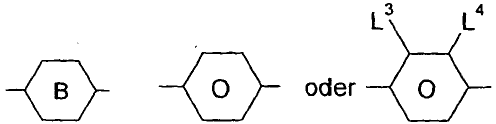

   - R¹ und R²: jeweils unabhängig voneinander Alkyl mit 1 bis 12 C-Atomen, wobei auch eine oder zwei nicht benachbarte CH₂-Gruppen durch -O-, -CH=CH-, -CO-, -OCO- oder - COO- so ersetzt sein können, dass O-Atome nicht direkt miteinander verknüpft sind, vorzugsweise Alkyl oder Alkoxy mit 1 bis 6 C-Atomen,
   - Z^{x} und Z^{y}: jeweils unabhängig voneinander -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, oder eine Einfachbindung, vorzugsweise eine Einfachbindung,
   - L¹⁻⁴: jeweils unabhängig voneinander F, Cl, OCF₃, CF₃, CH₃, CH₂F, CHF₂.

   Vorzugsweise bedeuten beide Reste L¹ und L² F, oder einer der Reste L¹ und L² F und der andere Cl, bzw. beide Reste L³ und L⁴ F, oder einer der Reste L³ und L⁴ F und der andere Cl.
   Die Verbindungen der Formel CY sind vorzugsweise ausgewählt aus der Gruppe bestehend aus folgenden Unterformeln 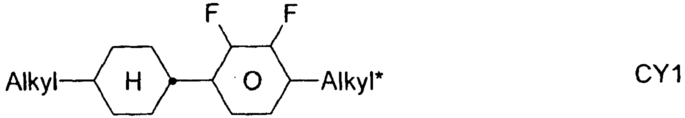 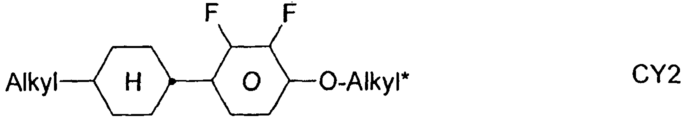 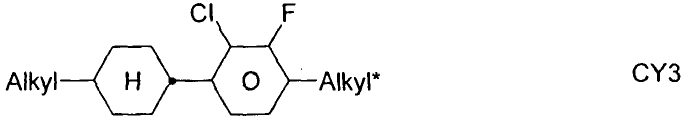 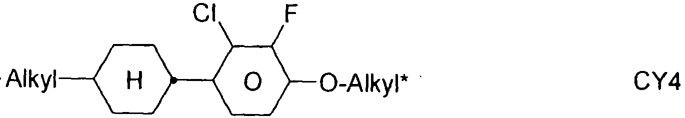 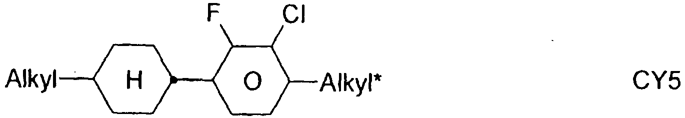 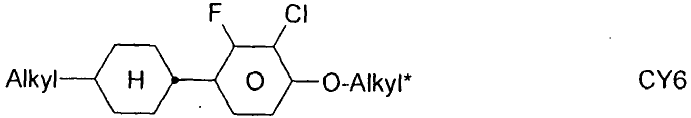 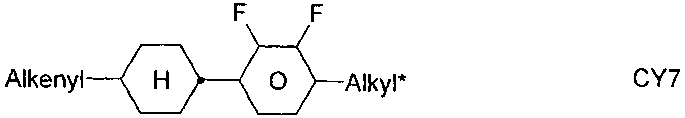 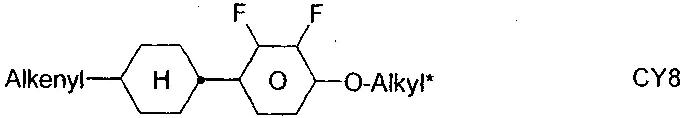 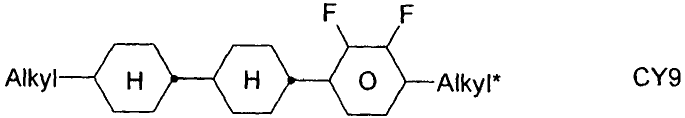 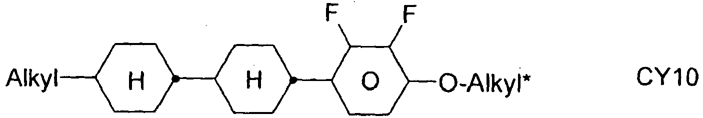 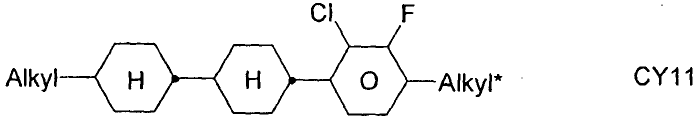 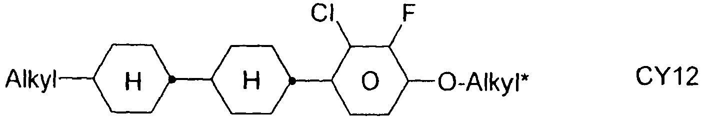 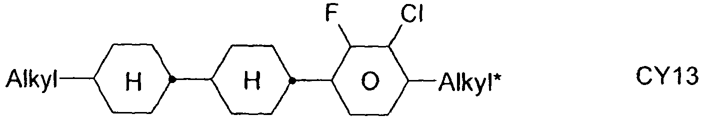 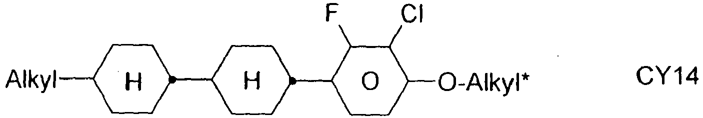 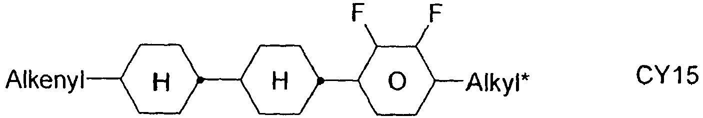 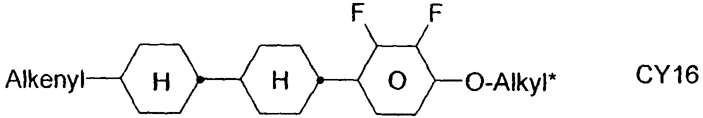 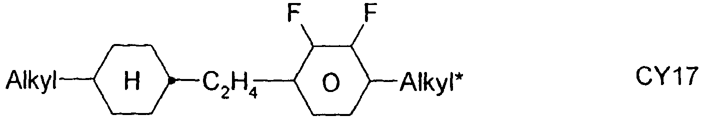 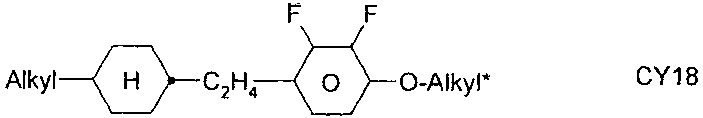 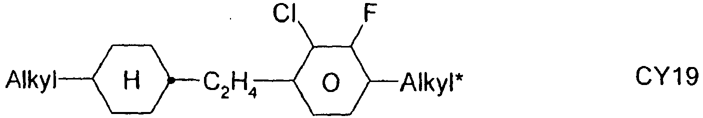 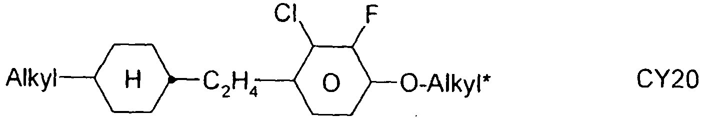 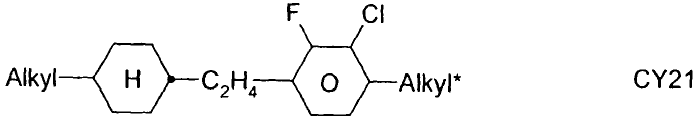 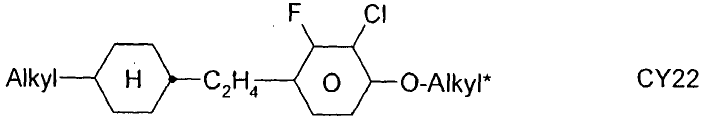 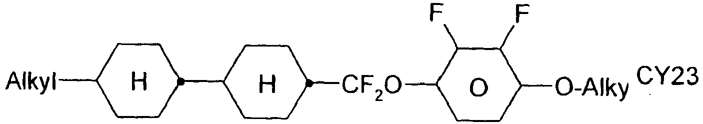 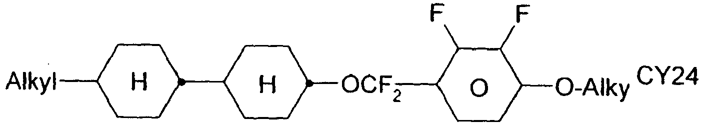 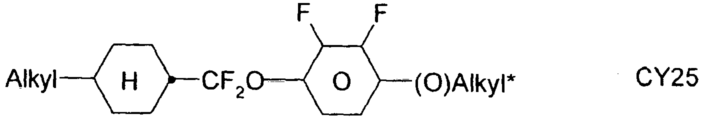 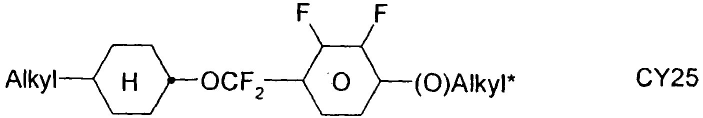 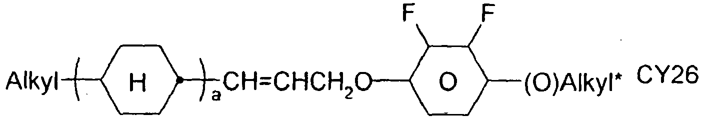 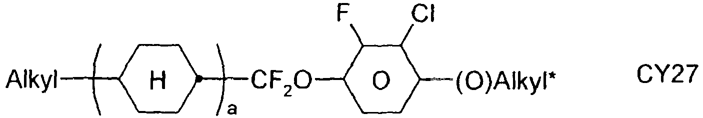 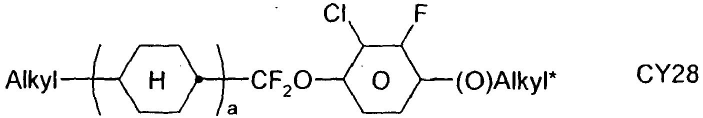 worin a 1 oder 2, Alkyl und Alkyl* jeweils unabhängig voneinander einen geradkettigen Alkylrest mit 1-6 C-Atomen, und Alkenyl einen geradkettigen Alkenylrest mit 2-6 C-Atomen, und (O) ein Sauerstoffatom oder eine Einfachbindung bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
   Die Verbindungen der Formel PY sind vorzugsweise ausgewählt aus der Gruppe bestehend aus folgenden Unterformeln: 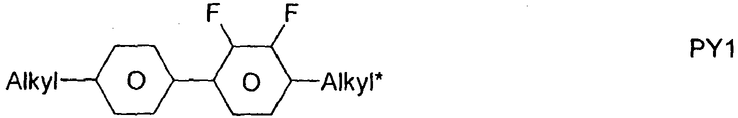 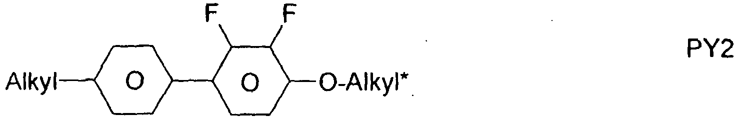 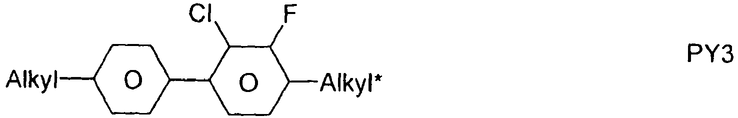 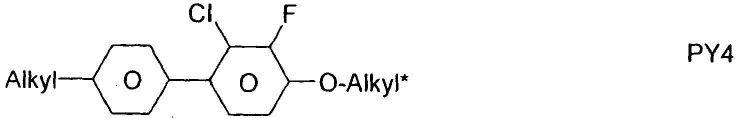 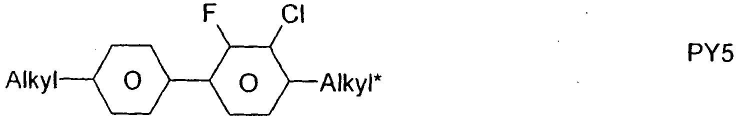 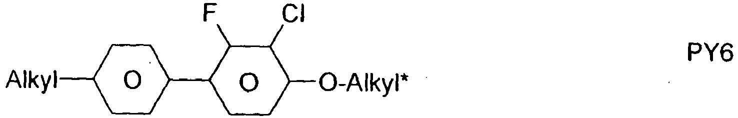 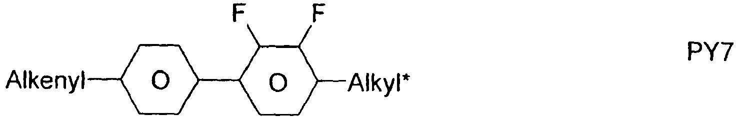 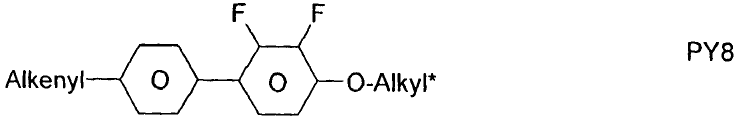 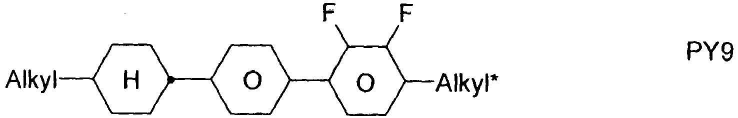 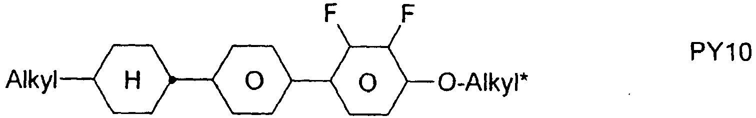 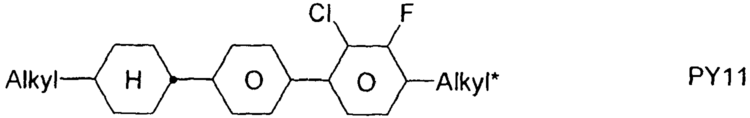 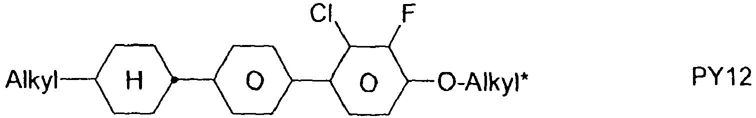 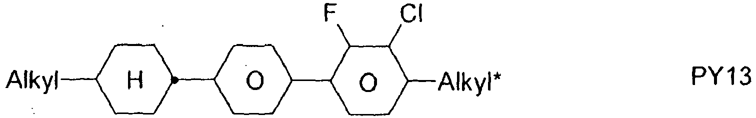 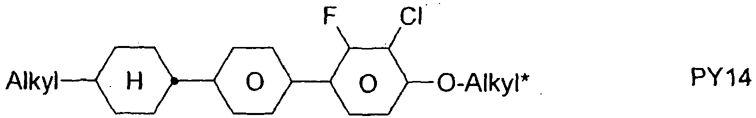 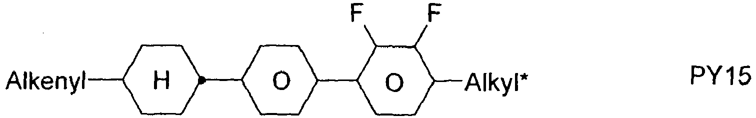 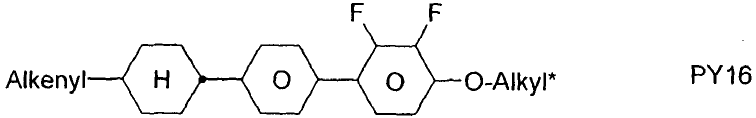 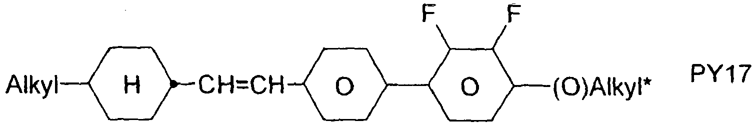 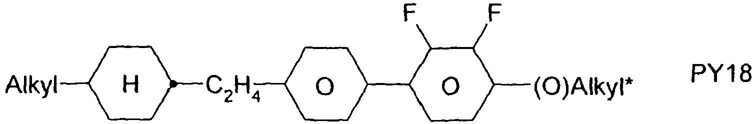 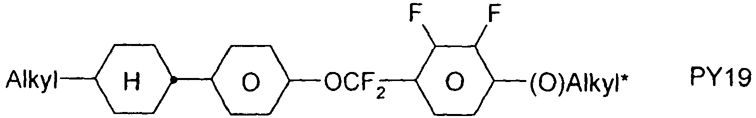 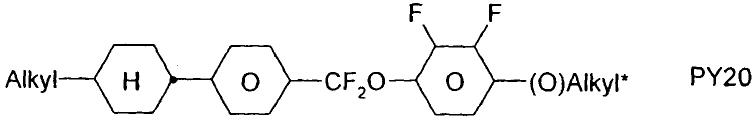 worin Alkyl und Alkyl* jeweils unabhängig voneinander einen geradkettigen Alkylrest mit 1-6 C-Atomen und Alkenyl einen geradkettigen Alkenylrest mit 2-6 C-Atomen, und (O) ein Sauerstoffatom oder eine Einfachbindung bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
b) FK-Medium, welches zusätzlich eine oder mehrere Verbindungen der folgenden Formel enthält: 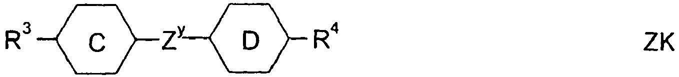 worin die einzelnen Reste folgende Bedeutung besitzen 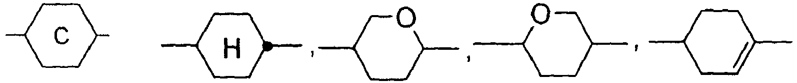 oder 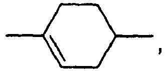 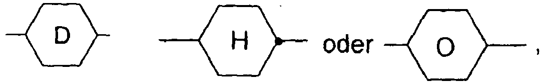
   - R³ und R⁴: jeweils unabhängig voneinander Alkyl mit 1 bis 12 C-Atomen, worin auch eine oder zwei nicht benachbarte CH₂-Gruppen durch -O-, -CH=CH-, -CO-, -O-CO- oder -CO-O- so ersetzt sein können, dass O-Atome nicht direkt miteinander verknüpft sind,
   - Z^{y}: -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, oder eine Einfachbindung, vorzugsweise eine Einfachbindung.

   Die Verbindungen der Formel ZK sind vorzugsweise ausgewählt aus der Gruppe bestehend aus folgenden Unterformeln: 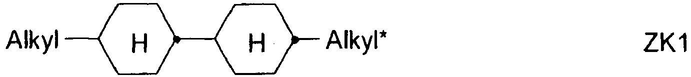 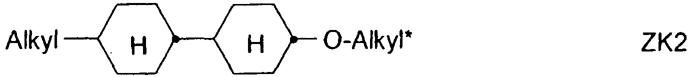 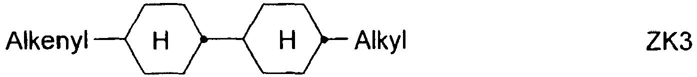 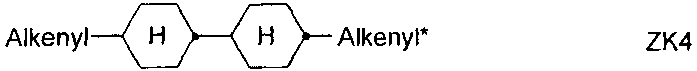 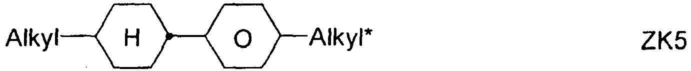 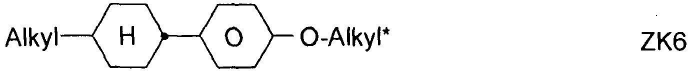 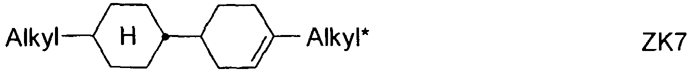 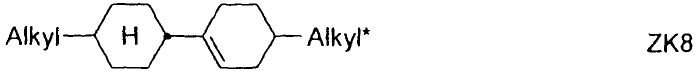 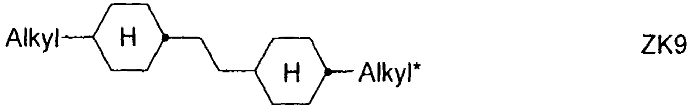 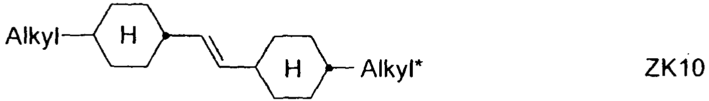 worin Alkyl und Alkyl* jeweils unabhängig voneinander einen geradkettigen Alkylrest mit 1-6 C-Atomen bedeuten, und Alkenyl einen geradkettigen Alkenylrest mit 2-6 C-Atomen bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
c) FK-Medium, welches zusätzlich eine oder mehrere Verbindungen der folgenden Formel enthält: 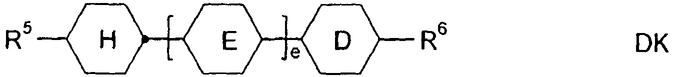 worin die einzelnen Reste bei jedem Auftreten gleich oder verschieden folgende Bedeutung haben:
   - R⁵ und R⁶: jeweils unabhängig voneinander eine der oben für R¹ angegebenen Bedeutungen, 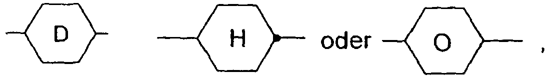 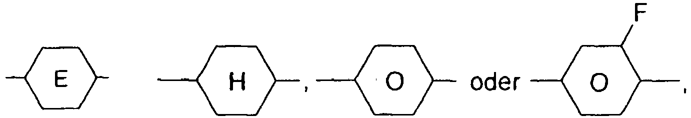 und
   - e: 1 oder 2.

   Die Verbindungen der Formel DK sind vorzugsweise ausgewählt aus der Gruppe bestehend aus folgenden Unterformeln: 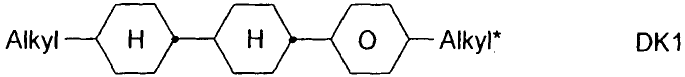 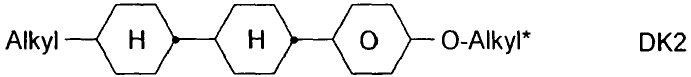 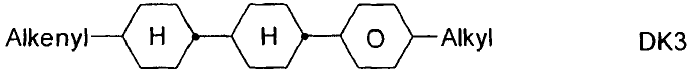 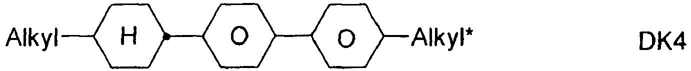 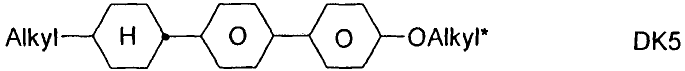 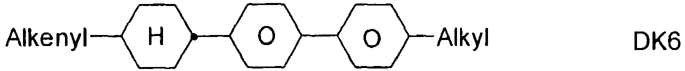 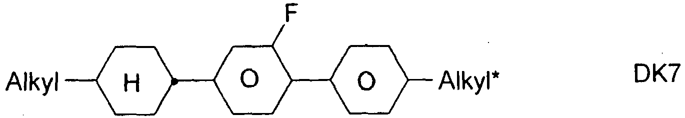 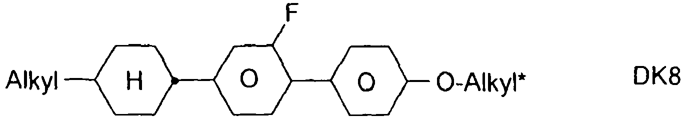 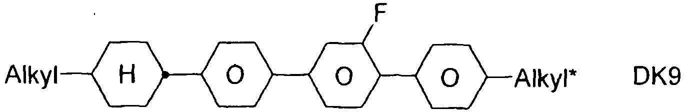 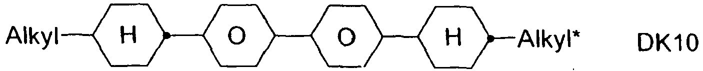 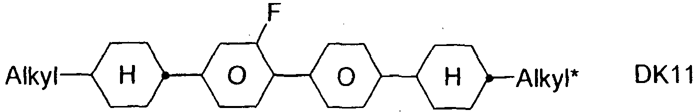 worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
d) FK-Medium, welches zusätzlich eine oder mehrere Verbindungen der folgenden Formel enthält: 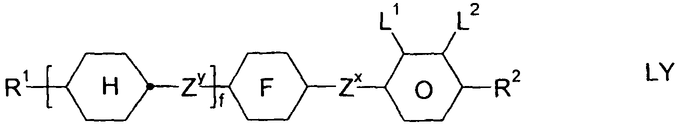 worin die einzelnen Reste folgende Bedeutung besitzen 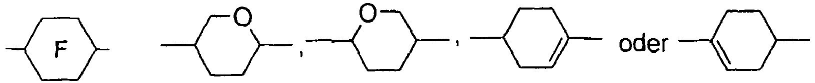
   - f: 0 oder 1,
   - R¹ und R²: jeweils unabhängig voneinander Alkyl mit 1 bis 12 C-Atomen, wobei auch eine oder zwei nicht benachbarte CH₂-Gruppen durch -O-, -CH=CH-, -CO-, -OCO- oder-COO- so ersetzt sein können, dass O-Atome nicht direkt miteinander verknüpft sind,
   - Z^{x} und Z^{y}: jeweils unabhängig voneinander -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, oder eine Einfachbindung, vorzugsweise eine Einfachbindung,
   - L¹ und L²: jeweils unabhängig voneinander F, Cl, OCF₃, CF₃, CH₃, CH₂F, CHF₂.

   Vorzugsweise bedeuten beide Reste L¹ und L² F oder einer der Reste L¹ und L² F und der andere Cl.
   Die Verbindungen der Formel LY sind vorzugsweise ausgewählt aus der Gruppe bestehend aus folgenden Unterformeln 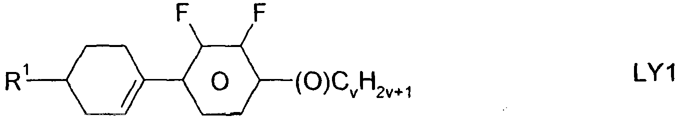 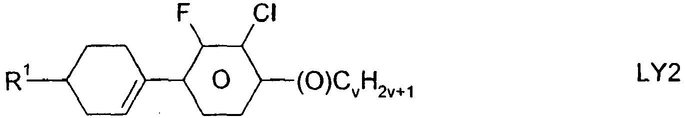 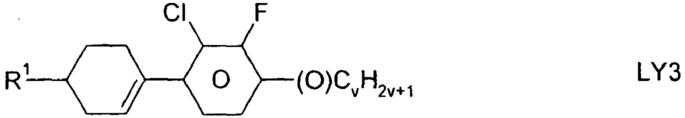 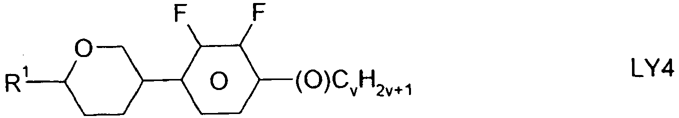 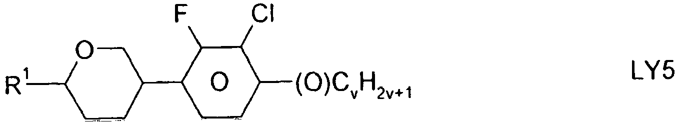 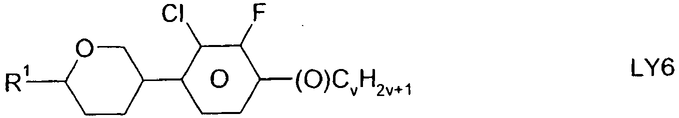 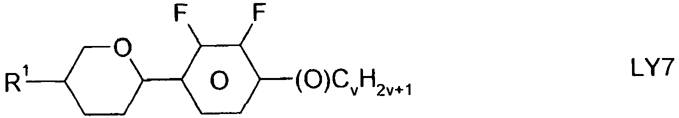 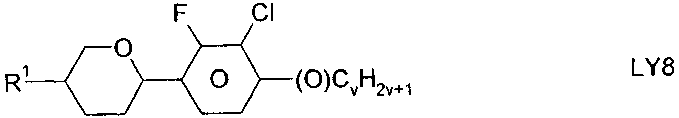 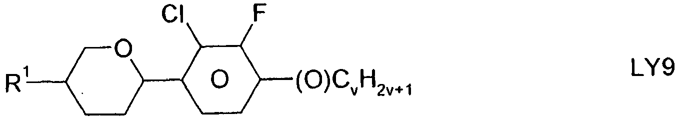 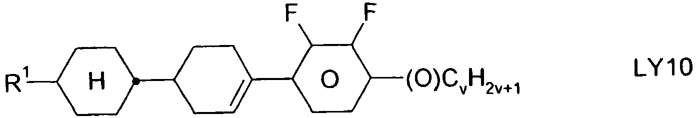 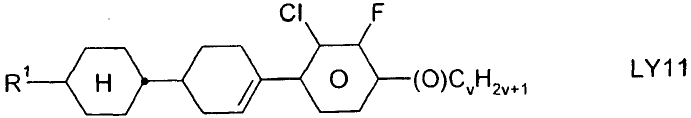 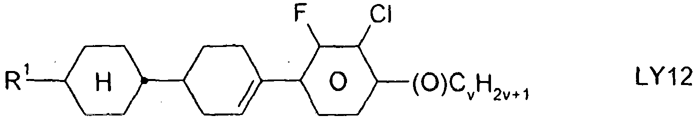 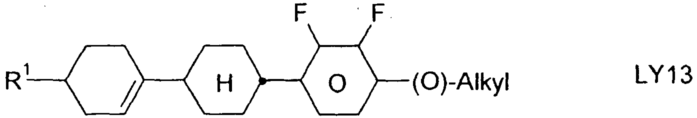 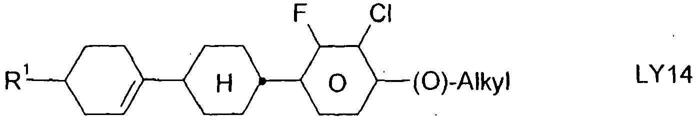 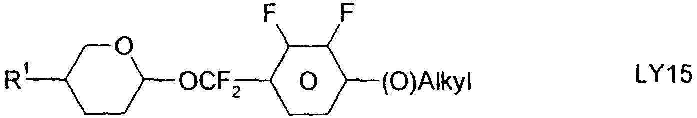 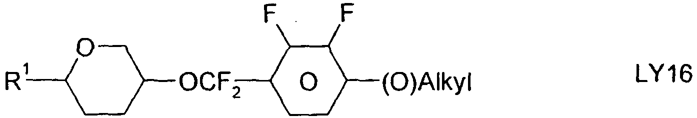 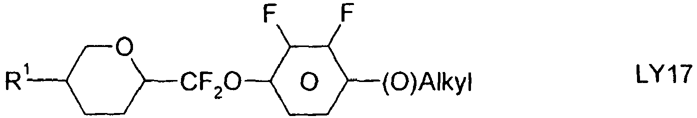 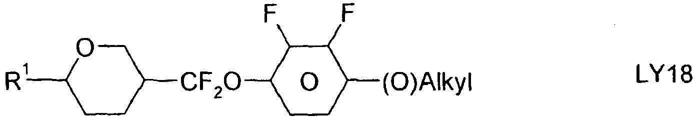 worin R¹ die oben angegebene Bedeutung hat, Alkyl einen geradkettigen Alkylrest mit 1-6 C-Atomen, (O) ein Sauerstoffatom oder eine Einfachbindung und v eine ganze Zahl von 1 bis 6 bedeuten. R¹ bedeutet vorzugsweise geradkettiges Alkyl mit 1 bis 6 C-Atomen oder geradkettiges Alkenyl mit 2 bis 6 C-Atomen,
   insbesondere CH₃, C₂H₅, n-C₃H₇, n-C₄H₉, n-C₅H₁₁, CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
e) FK-Medium, welches zusätzlich eine oder mehrere Verbindungen ausgewählt aus der Gruppe bestehend aus folgenden Formeln enthält: 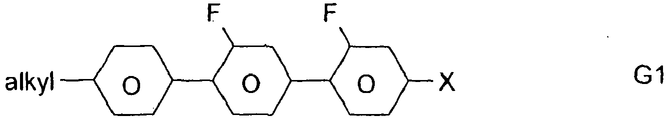 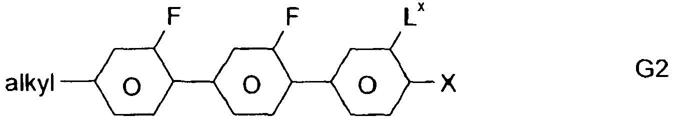 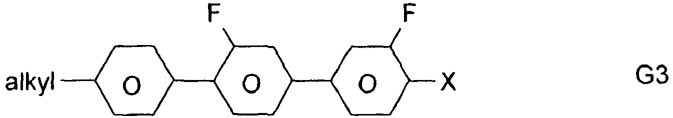 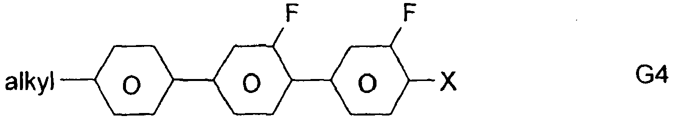 worin alkyl C₁₋₆-alkyl, L^{x} H oder F und X F, Cl, OCF₃, OCHF₂ oder OCH=CF₂ bedeutet. Besonders bevorzugt sind Verbindungen der Formel G1, worin X F bedeutet.
f) FK-Medium, welches zusätzlich eine oder mehrere Verbindungen ausgewählt aus der Gruppe bestehend aus folgenden Formeln enthält: 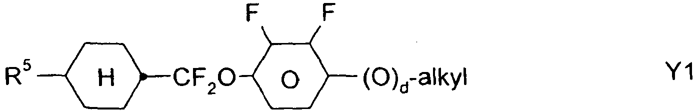 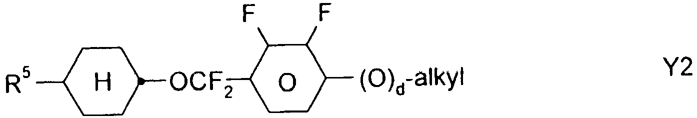 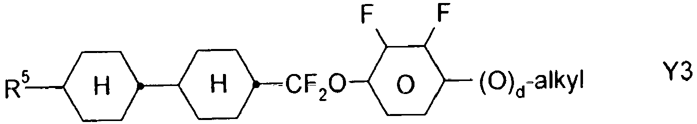 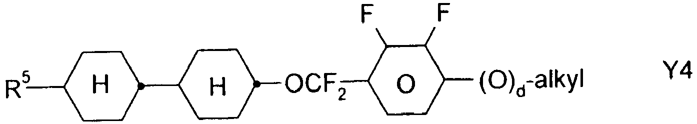 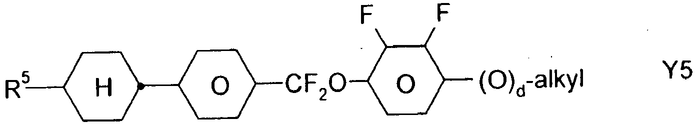 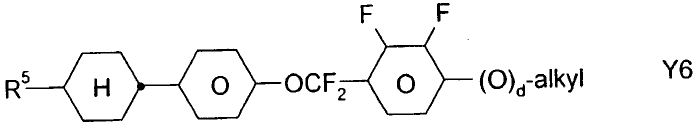 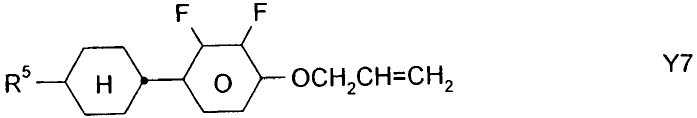 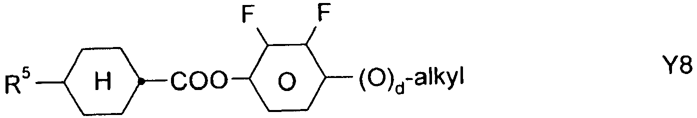 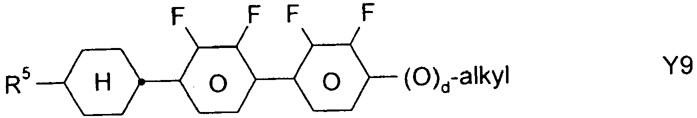 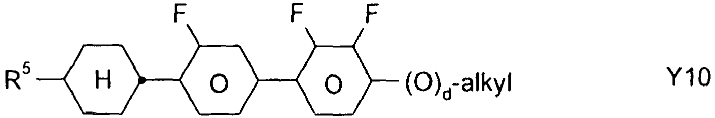 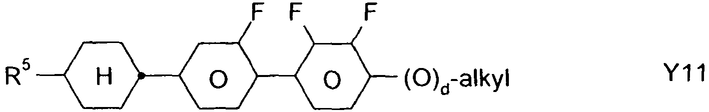 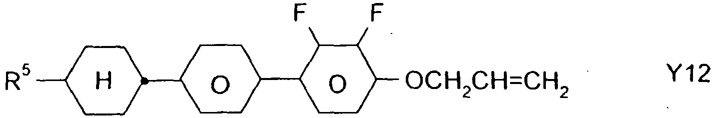 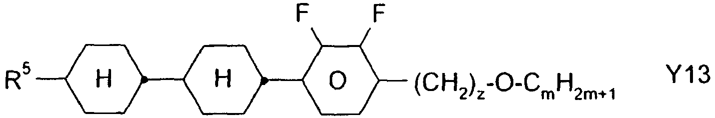 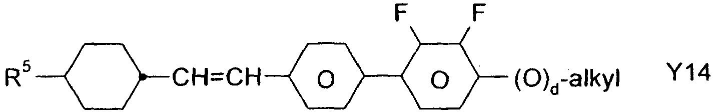 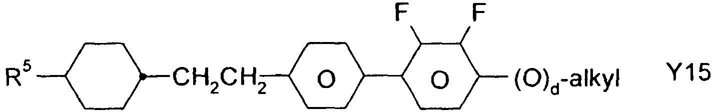 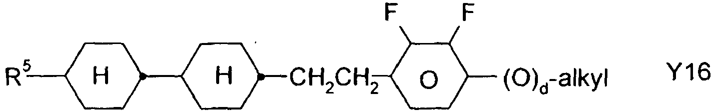 worin R⁵ eine der oben für R¹ angegebenen Bedeutungen besitzt, alkyl C₁₋₆-alkyl, d 0 oder 1, und z und m jeweils unabhängig voneinander eine ganze Zahl von 1 bis 6 bedeuten. R⁵ ist in diesen Verbindungen besonders bevorzugt C₁₋₆-alkyl oder -alkoxy oder C₂₋₆-alkenyl, d ist vorzugsweise 1. Vorzugsweise enthält das erfindungsgemäße FK-Medium eine oder mehrere Verbindungen der oben genannten Formeln in Mengen von ≥ 5 Gew.%.
g) FK-Medium, welches zusätzlich eine oder mehrere Biphenylverbindungen ausgewählt aus der Gruppe bestehend aus folgenden Formeln enthält: 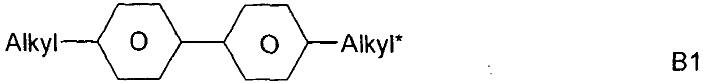 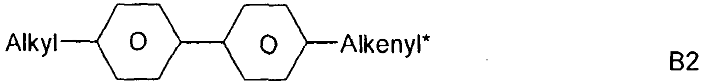 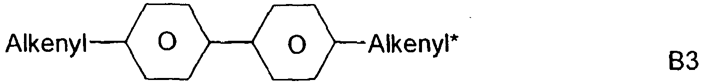 worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
   Der Anteil der Biphenyle der Formeln B1 bis B3 in der FK-Mischung beträgt vorzugsweise mindestens 3 Gew.%, insbesondere ≥ 5 Gew.%.
   Die Verbindungen der Formel B2 sind besonders bevorzugt.
   Die Verbindungen der Formel B1 bis B3 sind vorzugsweise ausgewählt aus der Gruppe bestehend aus folgenden Unterformeln 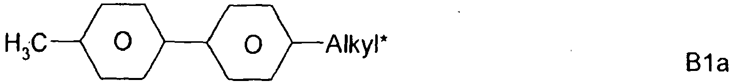 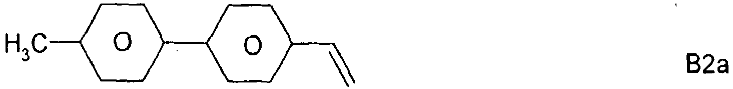 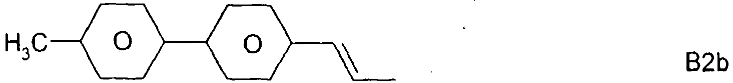 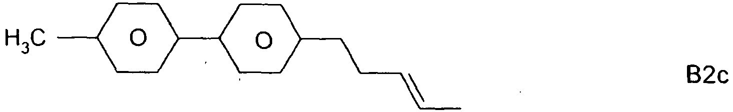 worin Alkyl* einen Alkylrest mit 1-6 C-Atomen bedeutet. Insbesondere bevorzugt enthält das erfindungsgemäße Medium eine oder mehrere Verbindungen der Formeln B1a und/oder B2c.
h) FK-Medium, welches zusätzlich eine oder mehrere Terphenylverbindungen der folgenden Formel enthält: 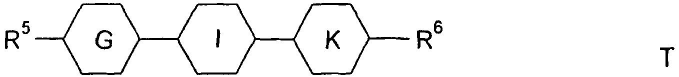 worin R⁵ und R⁶ jeweils unabhängig voneinander eine der oben für R¹ angegebenen Bedeutungen besitzen und 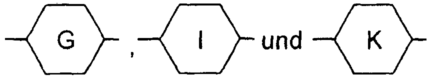 jeweils unabhängig voneinander 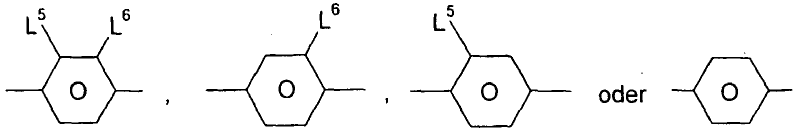 bedeuten, worin L⁵ F oder Cl, vorzugsweise F, und L⁶ F, Cl, OCF₃, CF₃, CH₃, CH₂F oder CHF₂, vorzugsweise F, bedeuten.
   Die Verbindungen der Formel T sind vorzugsweise ausgewählt aus der Gruppe bestehend aus folgenden Unterformeln 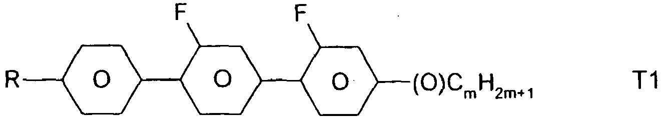 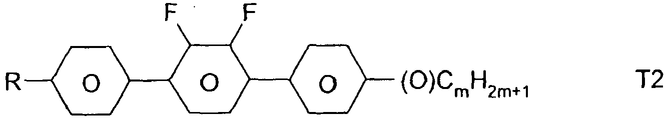 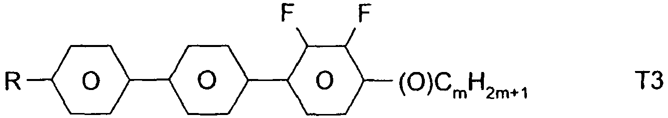 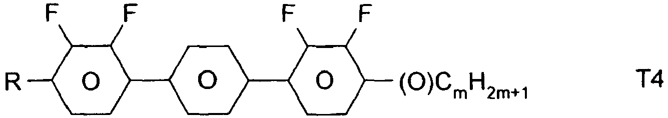 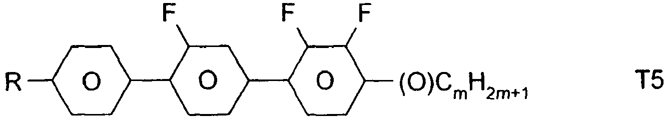 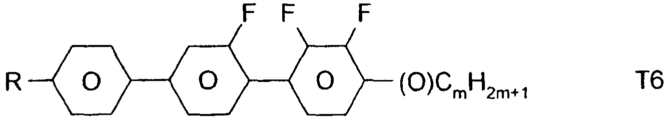 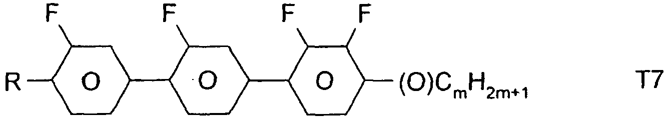 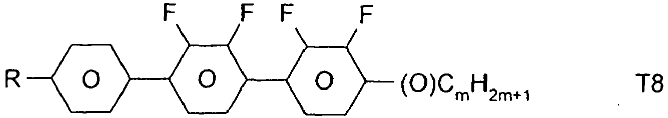 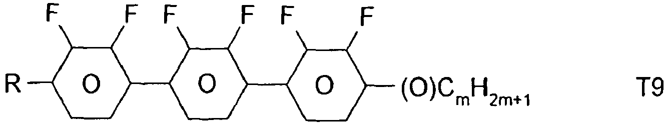 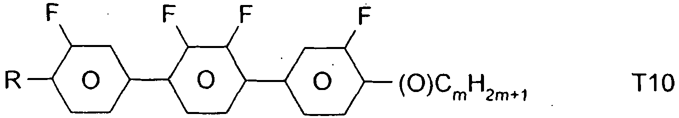 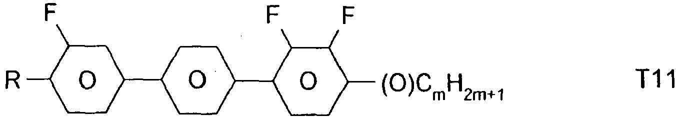 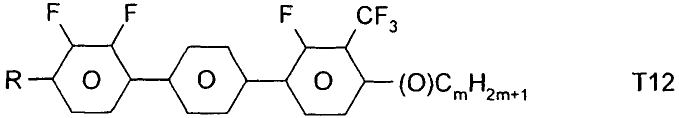 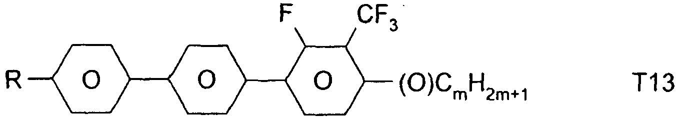 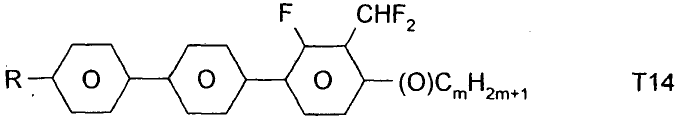 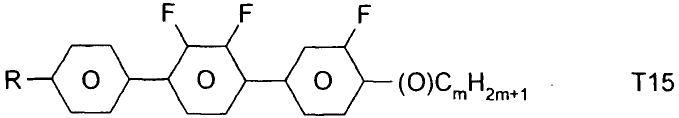 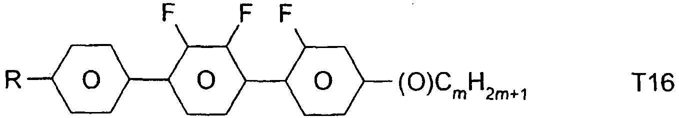 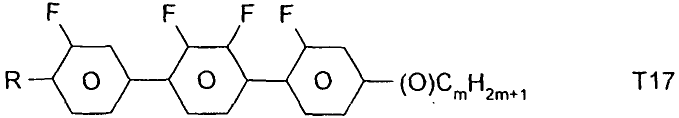 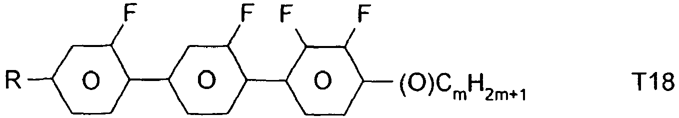 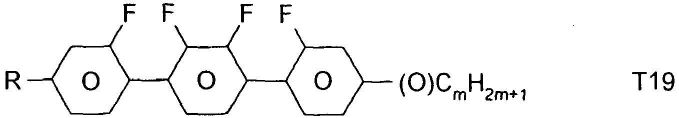 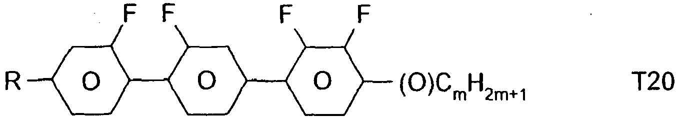 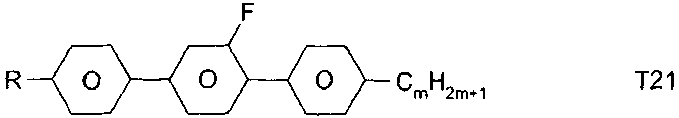 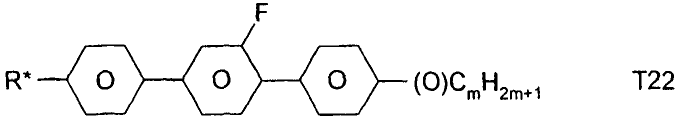 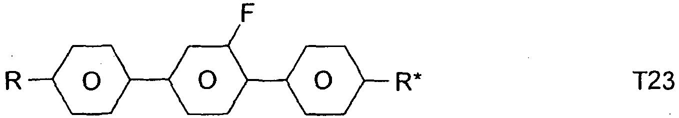 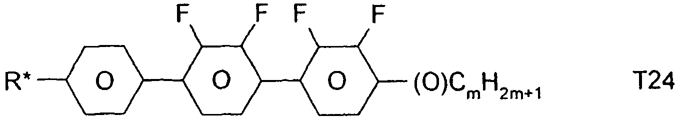 worin R einen geradkettigen Alkyl- oder Alkoxyrest mit 1-7 C-Atomen, R* einen geradkettigen Alkenylrest mit 2-7 C-Atomen, (O) ein Sauerstoffatom oder eine Einfachbindung, und m eine ganze Zahl von 1 bis 6 bedeutet. R* bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
   Vorzugsweise bedeutet R Methyl, Ethyl, Propyl, Butyl, Pentyl, Hexyl Methoxy, Ethoxy, Propoxy, Butoxy oder Pentoxy.
   Das erfindungsgemäße FK-Medium enthält die Terphenyle der Formeln T und deren bevorzugte Unterformeln vorzugsweise in einer Menge von 2-30 Gew.%, insbesondere von 5-20 Gew.%.
   Besonders bevorzugt sind Verbindungen der Formeln T1, T2, T3 und T21. In diesen Verbindungen bedeutet R vorzugsweise Alkyl, ferner Alkoxy jeweils mit 1-5 C-Atomen.
   Vorzugsweise werden die Terphenyle in erfindungsgemäßen Mischungen eingesetzt, wenn der Δn-Wert der Mischung ≥ 0,1 sein soll. Bevorzugte Mischungen enthalten 2-20 Gew.% einer oder mehrerer Terphenyl-Verbindungen der Formel T, vorzugsweise ausgewählt aus der Gruppe der Verbindungen T1 bis T22.
i) FK-Medium, welches zusätzlich eine oder mehrere Verbindungen ausgewählt aus der Gruppe bestehend aus folgenden Formeln enthält: 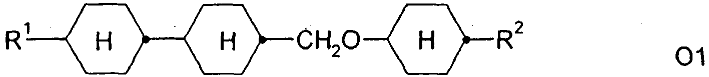 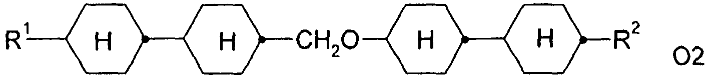 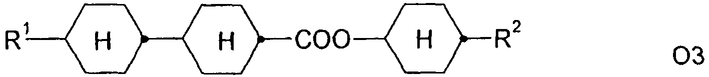 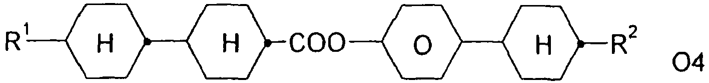 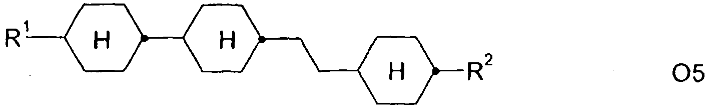 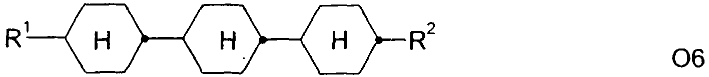 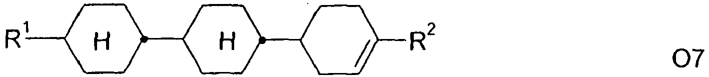 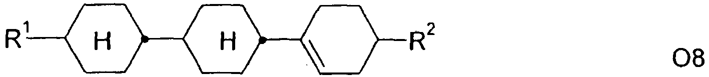 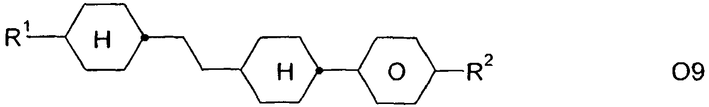 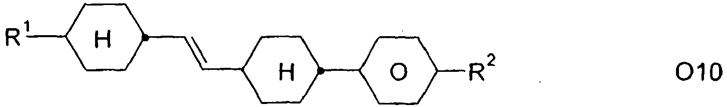 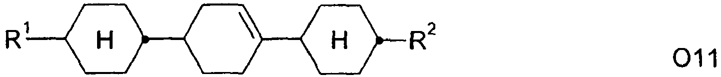 worin R¹ und R² die oben angegebenen Bedeutungen haben, und vorzugsweise jeweils unabhängig voneinander geradkettiges Alkyl mit 1 bis 6 C-Atomen oder geradkettiges Alkenyl mit 2 bis 6 C-Atomen bedeuten.
   Bevorzugte Medien enthalten eine oder mehrere Verbindungen ausgewählt aus den Formeln O1, O3 und O4.
k) FK-Medium, welches zusätzlich eine oder mehrere Verbindungen der folgenden Formel enthält: 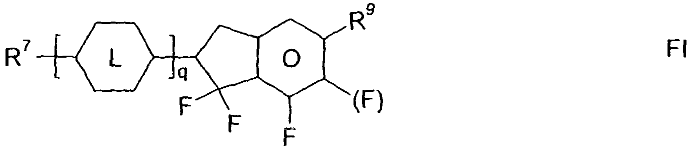 worin 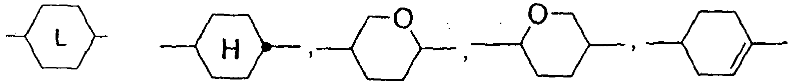 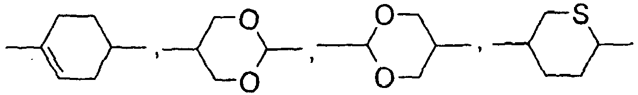 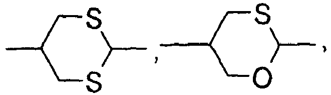 R⁹ H, CH₃, C₂H₅ oder n-C₃H₇, (F) einen optionalen Fluorsubstituenten und q 1, 2 oder 3 bedeutet, und R⁷ eine der für R¹ angegebenen Bedeutungen hat, vorzugsweise in Mengen von > 3 Gew.%, insbesondere ≥ 5 Gew.%, und ganz besonders bevorzugt von 5-30 Gew.%.
   Besonders bevorzugte Verbindungen der Formel IF sind ausgewählt aus der Gruppe bestehend aus folgenden Unterformeln: 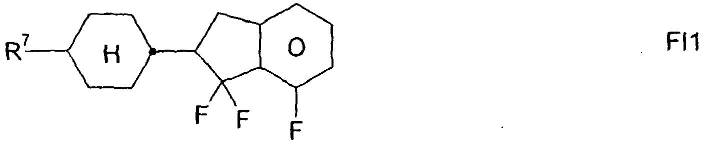 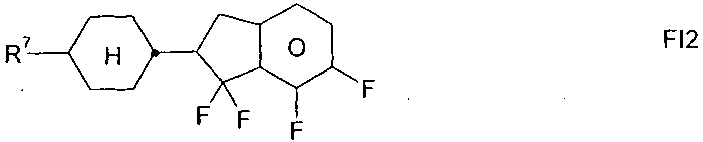 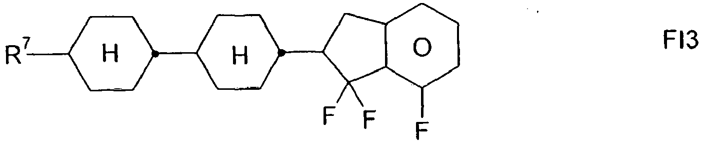 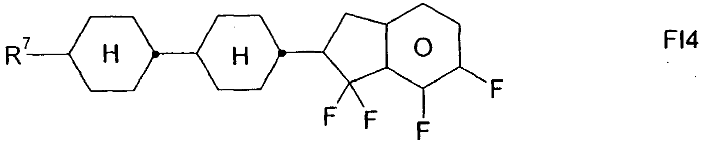 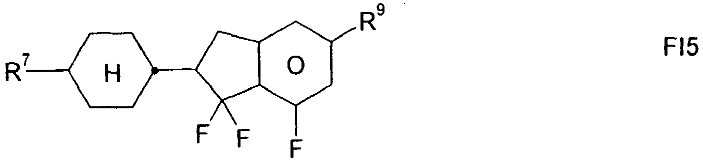 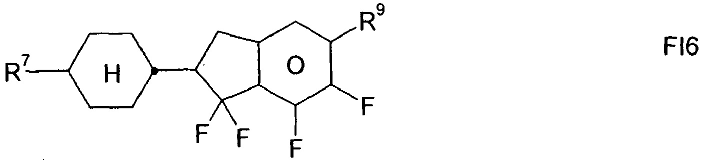 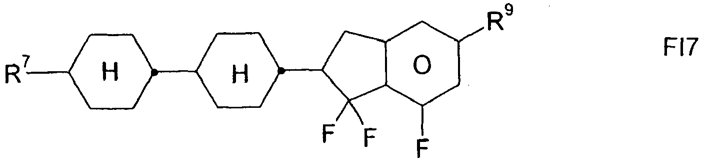 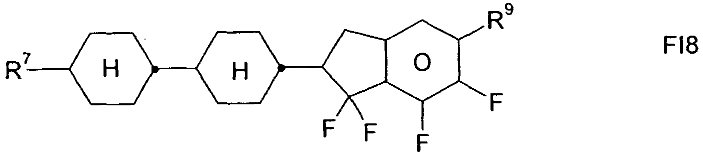 worin R⁷ vorzugsweise geradkettiges Alkyl bedeutet und R⁹ CH₃, C₂H₅ oder n-C₃H₇ bedeutet. Besonders bevorzugt sind die Verbindungen der Formel FI1, FI2 und FI3.
m) FK-Medium, welches zusätzlich eine oder mehrere Verbindungen ausgewählt aus der Gruppe bestehend aus folgenden Formeln enthält: 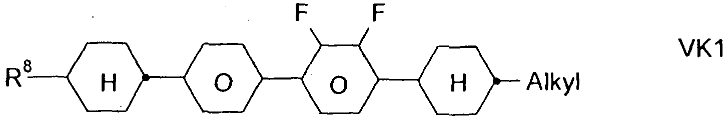 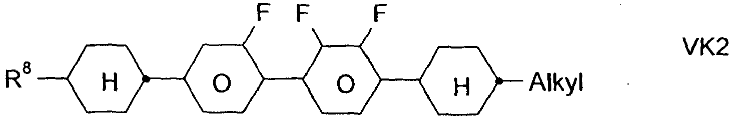 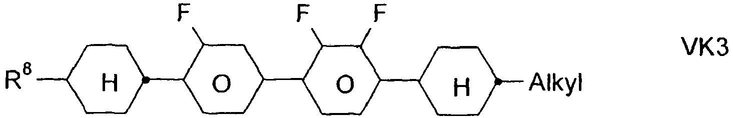 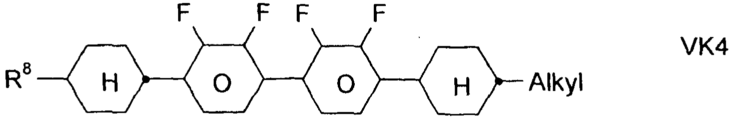 worin R⁸ die für R¹ angegebene Bedeutung hat und Alkyl einen geradkettigen Alkylrest mit 1-6 C-Atomen bedeutet.
n) FK-Medium, welches zusätzlich eine oder mehrere Verbindungen enthält, die eine Tetrahydronaphthyl- oder Naphthyl-Einheit aufweisen, wie z.B. die Verbindungen ausgewählt aus der Gruppe bestehend aus folgenden Formeln: 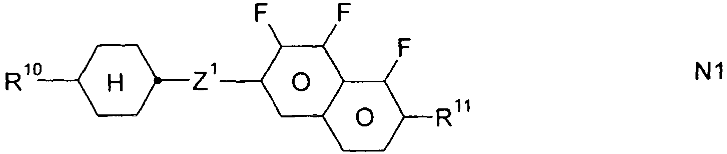 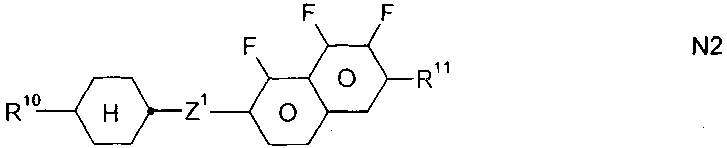 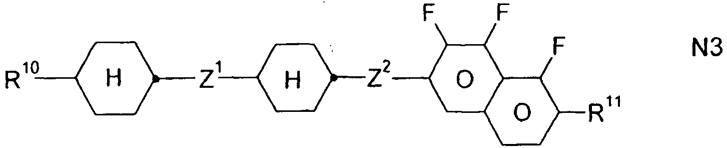 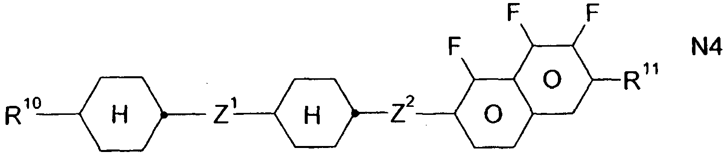 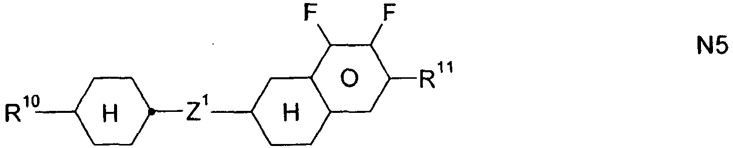 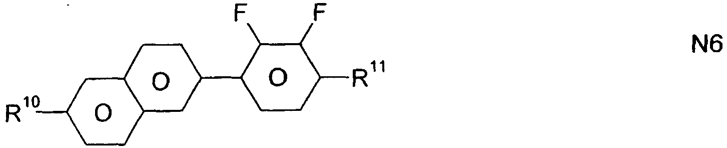 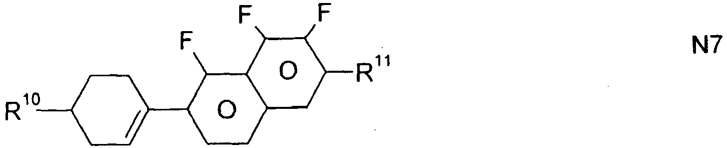 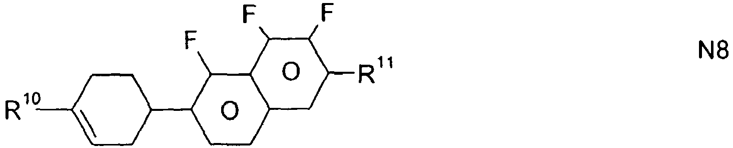 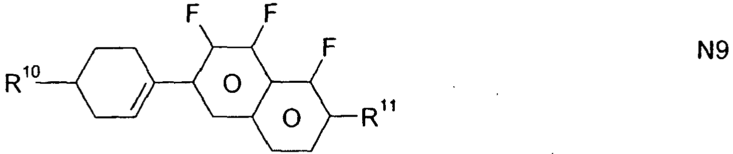 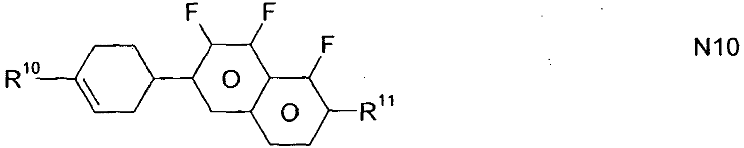 worin R¹⁰ und R¹¹ jeweils unabhängig voneinander eine der für R¹ angegebenen Bedeutungen haben, vorzugsweise geradkettiges Alkyl oder Alkoxy mit 1 bis 6 C-Atomen oder geradkettiges Alkenyl mit 2 bis 6 C-Atomen bedeuten, und Z¹ und Z² jeweils unabhängig voneinander -C₂H₄-, -CH=CH-, -(CH₂)₄-, -(CH₂)₃O-, -O(CH₂)₃-, -CH=CH-CH₂CH₂-, -CH₂CH₂CH=CH-, -CH₂O-, -OCH₂-, -CO-O-, -O-CO-, -C₂F₄-, -CF=CF-, -CF=CH-, -CH=CF-, -CH₂- oder eine Einfachbindung bedeuten.
o) FK-Medium, welches zusätzlich eine oder mehrere eine oder mehrere Difluordibenzochromane und/oder Chromane der folgenden Formeln enthält: 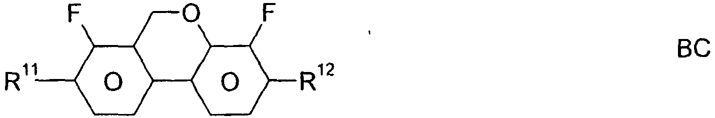 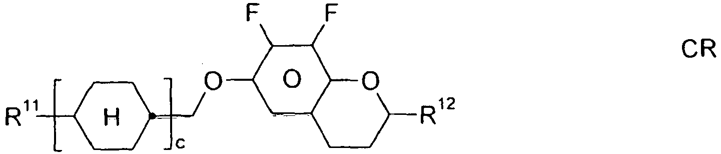 worin R¹¹ und R¹² jeweils unabhängig voneinander die oben angegebene Bedeutung aufweisen, und c 0 oder 1 bedeutet, vorzugsweise in Mengen von 3 bis 20 Gew.%, insbesondere in Mengen von 3 bis 15 Gew.%.
   Besonders bevorzugte Verbindungen der Formeln BC und CR sind ausgewählt aus der Gruppe bestehend aus folgenden Unterformeln: 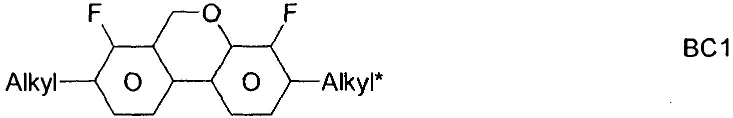 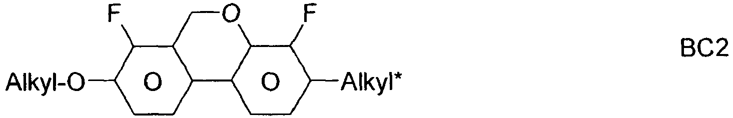 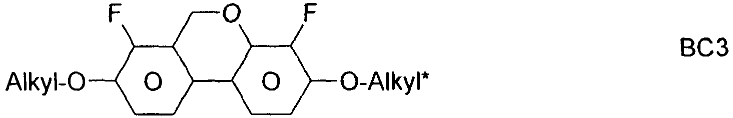 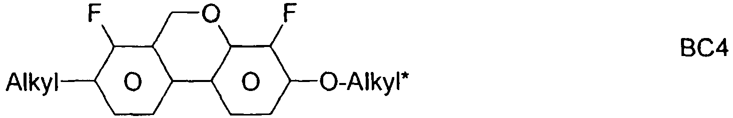 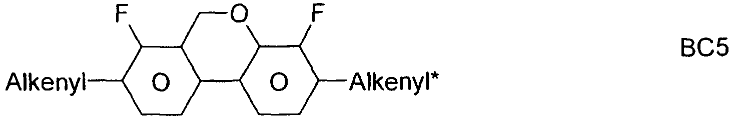 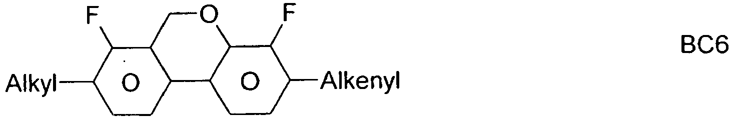 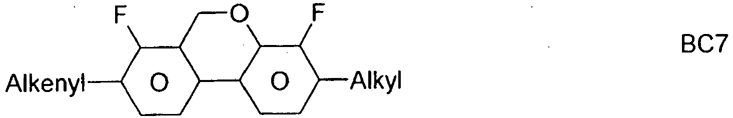 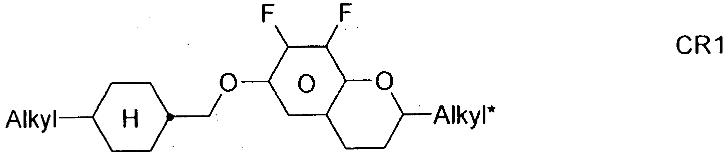 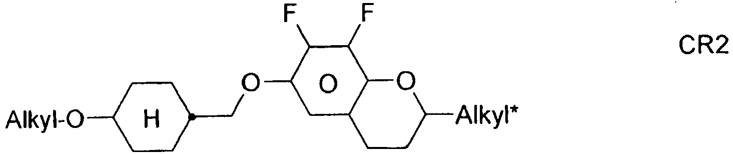 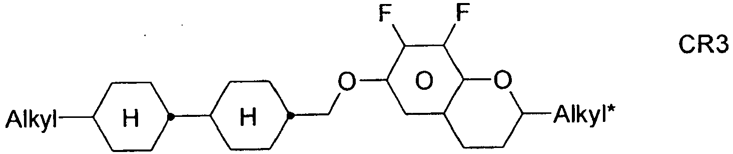 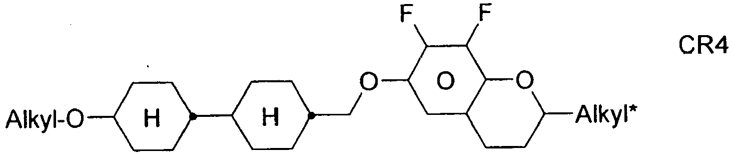 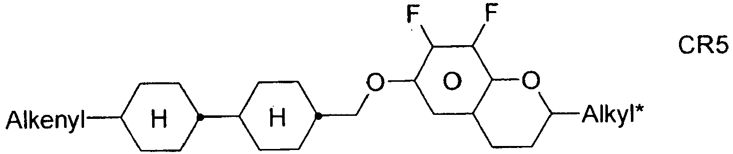 worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
   Ganz besonders bevorzugt sind Mischungen enthaltend eine, zwei oder drei Verbindungen der Formel BC-2.
p) FK-Medium, welches zusätzlich eine oder mehrere fluorierte Phenanthrene und/oder Dibenzofurane der folgenden Formeln enthält: 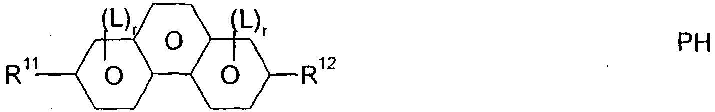 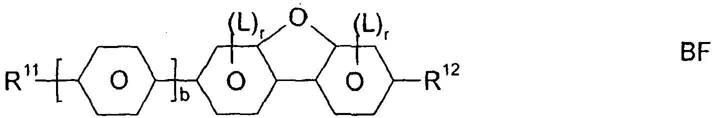 worin R¹¹ und R¹² jeweils unabhängig voneinander die oben angegebenen Bedeutungen besitzen, b 0 oder 1, L F und r 1, 2 oder 3 bedeutet.
   Besonders bevorzugte Verbindungen der Formeln PH und BF sind ausgewählt aus der Gruppe bestehend aus folgenden Unterformeln: 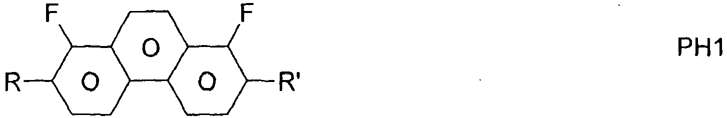 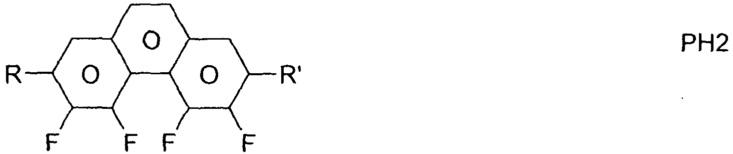 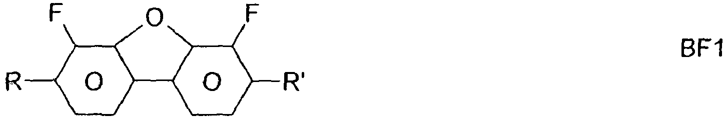 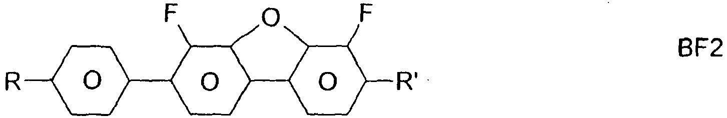 worin R und R' jeweils unabhängig voneinander einen geradkettigen Alkyl- oder Alkoxyrest mit 1-7 C-Atomen bedeuten.
q) FK-Medium, vorzugsweise zur Verwendung in PSA-OCB-Anzeigen, welches eine oder mehrere Verbindungen ausgewählt aus der Gruppe bestehend aus folgenden Formeln enthält: 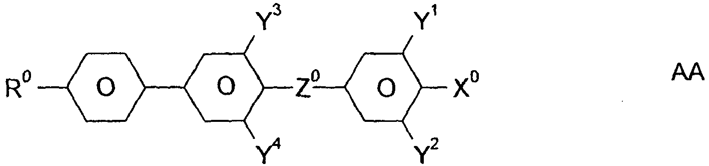 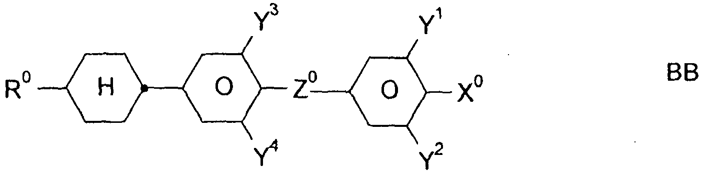 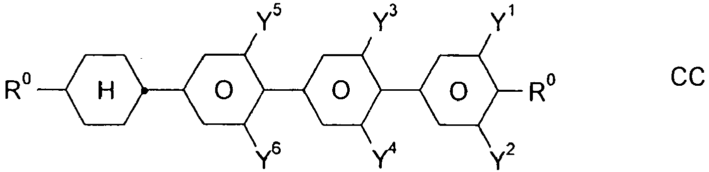 worin
   - R⁰: bei jedem Auftreten gleich oder verschieden n-Alkyl, Alkoxy, Oxaalkyl, Fluoralkyl oder Alkenyl mit jeweils bis zu 9 C-Atomen,
   - X⁰: F, Cl oder jeweils halogeniertes Alkyl, Alkenyl, Alkenyloxy oder Alkoxy mit jeweils bis zu 6 C-Atomen,
   - Z⁰: -CF₂O- oder eine Einfachbindung,
   - Y¹⁻⁶: jeweils unabhängig voneinander H oder F,
   bedeuten.
   X⁰ ist vorzugsweise F, Cl, CF₃, CHF₂, OCF₃, OCHF₂, OCFHCF₃, OCFHCHF₂, OCFHCHF₂, OCF₂CH₃, OCF₂CHF₂, OCF₂CHF₂, OCF₂CF₂CHF₂, OCF₂CF₂CHF₂, OCFHCF₂CF₃, OCFHCF₂CHF₂, OCF₂CF₂CF₃, OCF₂CF₂CClF₂, OCClFCF₂CF₃ oder CH=CF₂, besonders bevorzugt F oder OCF₃.
   Die Verbindungen der Formel AA sind vorzugsweise ausgewählt aus der Gruppe bestehend aus folgenden Formeln: 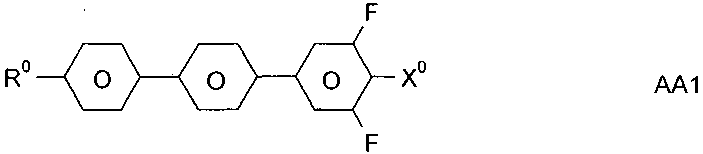 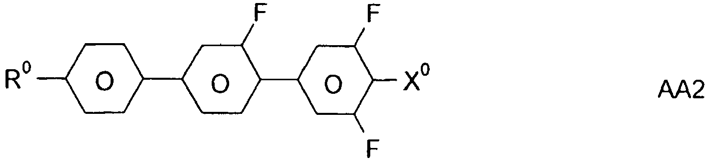 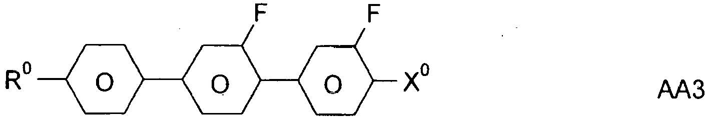 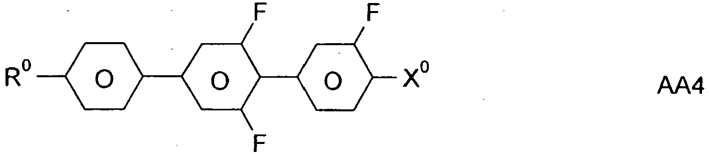 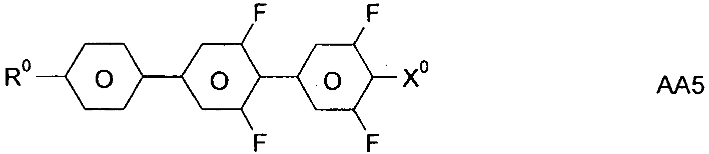 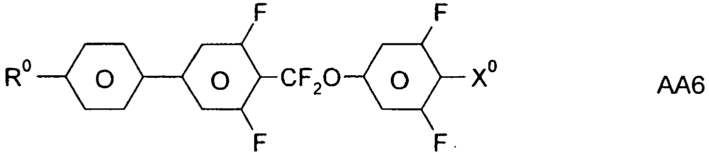 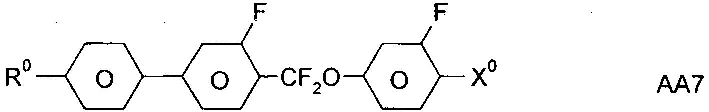 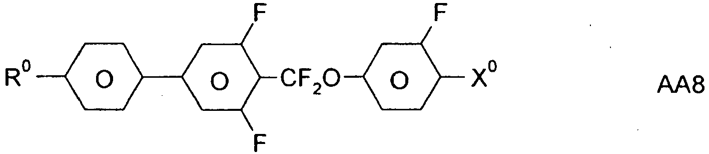 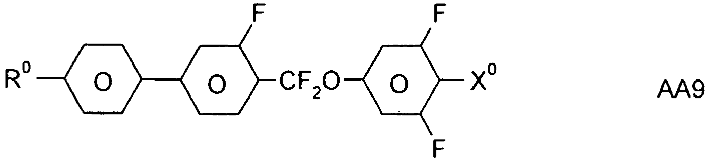 worin R⁰ und X⁰ die oben angegebene Bedeutung besitzen, und X⁰ vorzugsweise F bedeutet. Besonders bevorzugt sind Verbindungen der Formeln AA2 und AA6.
   Die Verbindungen der Formel BB sind vorzugsweise ausgewählt aus der Gruppe bestehend aus folgenden Formeln: 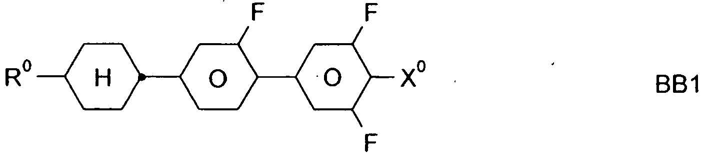 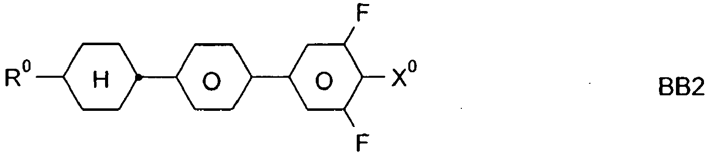 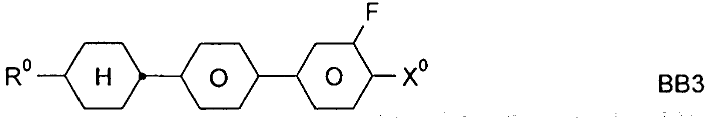 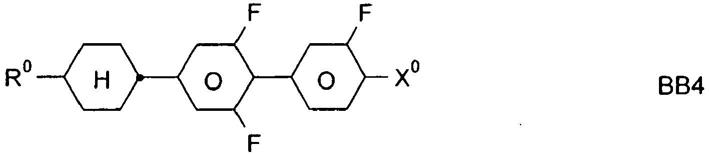 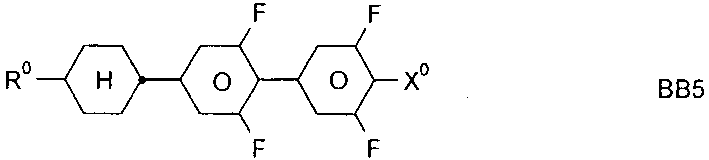 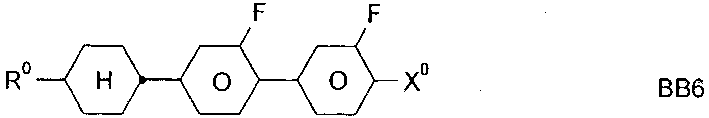 worin R⁰ und X⁰ die oben angegebene Bedeutung besitzen, und X⁰ vorzugsweise F bedeutet. Besonders bevorzugt sind Verbindungen der Formeln BB1, BB2 und BB5.
   Die Verbindungen der Formel CC sind vorzugsweise ausgewählt aus folgender Formel: 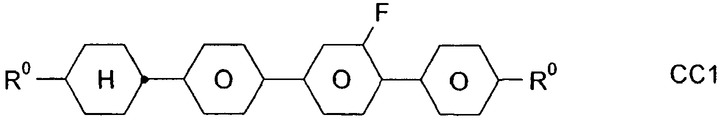 worin R⁰ bei jedem Auftreten gleich oder verschieden die oben angegebene Bedeutung besitzt, und vorzugsweise Alkyl mit 1 bis 6 C-Atomen bedeutet.
r) FK-Medium, welches außer den polymerisierbaren Verbindungen der Formel I oder deren Unterformeln sowie den Comonomeren keine Verbindungen enthält, die eine endständige Vinyloxygruppe (-O-CH=CH₂) aufweisen.
s) FK-Medium, welches 1 bis 5, vorzugsweise 1, 2 oder 3 polymerisierbare Verbindungen enthält.
t) FK-Medium, worin der Anteil an polymerisierbaren Verbindungen im Gesamtgemisch 0,05 bis 5 %, vorzugsweise 0,1 bis 1 % beträgt.
u) FK-Medium, welches 1 bis 8, vorzugsweise 1 bis 5 Verbindungen der Formel CY1, CY2, PY1 und/oder PY2 enthält. Der Anteil dieser Verbindungen im Gesamtgemisch beträgt vorzugsweise 5 bis 60 %, besonders bevorzugt 10 bis 35 %. Der Gehalt dieser einzelnen Verbindungen beträgt vorzugsweise jeweils 2 bis 20 %.
v) FK-Medium, welches 1 bis 8, vorzugsweise 1 bis 5 Verbindungen der Formel CY9, CY10, PY9 und/oder PY10 enthält. Der Anteil dieser Verbindungen im Gesamtgemisch beträgt vorzugsweise 5 bis 60 %, besonders bevorzugt 10 bis 35 %. Der Gehalt dieser einzelnen Verbindungen beträgt vorzugsweise jeweils 2 bis 20 %.
w) FK-Medium, welches 1 bis 10, vorzugsweise 1 bis 8 Verbindungen der Formel ZK enthält, insbesondere Verbindungen der Formel ZK1, ZK2 und/oder ZK6. Der Anteil dieser Verbindungen im Gesamtgemisch beträgt vorzugsweise 3 bis 25 %, besonders bevorzugt 5 bis 45 %. Der Gehalt dieser einzelnen Verbindungen beträgt vorzugsweise jeweils 2 bis 20 %.
x) FK-Medium, worin der Anteil an Verbindungen der Formel CY, PY und ZK im Gesamtgemisch mehr als 70 %, vorzugsweise mehr als 80 % beträgt,
y) PSA-VA-Anzeige, worin der pretilt-Winkel vorzugsweise ≤ 85°, besonderes bevorzugt ≤ 80° beträgt.

Die Kombination von Verbindungen der oben genannten bevorzugten Ausführungsformen a)-y) mit den oben beschriebenen polymerisierten Verbindungen bewirkt in den erfindungsgemäßen FK-Medien niedrige Schwellenspannungen, niedrige Rotationsviskositäten und sehr gute Tieftemperaturstabilitäten bei gleichbleibend hohen Klärpunkten und hohen HR-Werten, und erlaubt die schnelle Einstellung eines besonders niedrigen pretilt-Winkels in PSA-Anzeigen. Insbesondere zeigen die FK-Medien in PSA-Anzeigen im Vergleich zu den Medien aus dem Stand der Technik deutlich verringerte Schaltzeiten, insbesondere auch der Graustufenschaltzeiten.

Vorzugsweise weist die Flüssigkristallmischung einen nematischen Phasenbereich von mindestens 80 K, besonders bevorzugt von mindestens 100 K, und eine Rotationsviskosität von nicht mehr als 250, vorzugsweise nicht mehr als 200 mPa·s, bei 20°C auf.

Erfindungsgemäße FK-Medien zur Verwendung in Anzeigen des VA-Typs weisen eine negative dielektrische Anisotropie Δε auf, vorzugsweise von etwa -0,5 bis -10, insbesondere von etwa -2,5 bis -7,5 bei 20°C und 1 kHz.

In den erfindungsgemäßen Anzeigen des VA-Typs sind die Moleküle in der Schicht des FK-Mediums im ausgeschalteten Zustand senkrecht zu den Elektrodenflächen (homöotrop) oder gekippt homöotrop (engl. "tilted") orientiert. Bei Anlegen einer elektrischen Spannung an die Elektroden findet eine Umorientierung der FK-Moleküle mit den Moleküllängsachsen parallel zu den Elektrodenflächen statt.

In den erfindungsgemäßen Anzeigen des OCB-Typs sind die Moleküle in der Schicht des FK-Mediums eine "bend"-Orientierung auf. Bei Anlegen einer elektrischen Spannung findet eine Umorientierung der FK-Moleküle mit den Moleküllängsachsen senkrecht zu den Elektrodenflächen statt.

Erfindungsgemäße FK-Medien zur Verwendung in Anzeigen des OCB-Typs weisen eine positive dielektrische Anisotropie Δε auf, vorzugsweise von etwa +7 bis +17 bei 20°C und 1 kHz.

Die Doppelbrechung Δn in erfindungsgemäßen FK-Medien zur Verwendung in Anzeigen des VA-Typs liegt vorzugsweise unter 0,16, besonders bevorzugt zwischen 0,06 und 0,14, insbesondere zwischen 0,07 und 0,12.

Die Doppelbrechung Δn in erfindungsgemäßen FK-Medien zur Verwendung in Anzeigen des OCB-Typs liegt vorzugsweise zwischen 0,14 und 0,22, insbesondere zwischen 0,16 und 0,22.

Die erfindungsgemäßen FK-Medien können auch weitere, dem Fachmann bekannte und in der Literatur beschriebene Zusätze oder Additive enthalten, wie beispielsweise Polymerisationsinitiatoren, Inhibitoren, Stabilisatoren, oberflächenaktive Substanzen oder chirale Dotierstoffe. Diese können polymerisierbar oder unpolymerisierbar sein. Polymerisierbare Additive werden dementsprechend der polymerisierbaren Komponente oder Komponente A) zugerechnet. Unpolymerisierbare Additive werden dementsprechend der unpolymerisierbaren Komponente oder Komponente B) zugerechnet.

Die FK.Medien können beispielsweise einen oder mehrere chirale Dotierstoffe enthalten, vorzusgweise solche ausgewählt aus der Gruppe bestehned aus Verbindungen der nachfolgenden Tabelle B.

Ferner können den FK.Medien beispielsweise 0 bis 15 Gew.-% pleochroitische Farbstoffe zugesetzt werden, ferner Nanopartikel, Leitsalze, vorzugsweise Ethyl-dimethyldodecylammonium-4-hexoxy-benzoat, Tetrabutylammoniumtetraphenylborat oder Komplexsalze von Kronenethern (vgl. z.B. Haller et al., Mol. Cryst. Liq. Cryst. 24, 249-258 (1973)) zur Verbesserung der Leitfähigkeit, oder Substanzen zur Veränderung der dielektrischen Anisotropie, der Viskosität und/oder der Orientierung der nematischen Phasen. Derartige Substanzen sind z.B. in DE-A 22 09 127, 22 40 864, 23 21 632, 23 38 281, 24 50 088, 26 37 430 und 28 53 728 beschrieben.

Die einzelnen Komponenten der bevorzugten Ausführungsformen a)-z) der erfindungsgemäßen FK-Medien sind entweder bekannt, oder ihre Herstellungsweisen sind für den einschlägigen Fachmann aus dem Stand der Technik ohne weiteres abzuleiten, da sie auf in der Literatur beschriebenen Standardverfahren basieren. Entsprechende Verbindungen der Formel CY werden beispielsweise in EP-A-0 364 538 beschrieben. Entsprechende Verbindungen der Formel ZK werden beispielsweise in DE-A-26 36 684 und DE-A-33 21 373 beschrieben.

Die Herstellung der erfindungsgemäß verwendbaren FK-Medien erfolgt in an sich üblicher Weise, beispielsweise indem man eine oder mehrere der oben genannten Verbindungen mit einer oder mehreren polymerisierbaren Verbindungen wie oben definiert, und ggf. mit weiteren flüssigkristallinen Verbindungen und/oder Additiven mischt. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Das Verfahren zur Herstellung der erfindungsgemäßen FK-Medien ist ein weiterer Gegenstand der Erfindung.

Es versteht sich für den Fachmann von selbst, daß die erfindungsgemäßen FK-Medien auch Verbindungen enthalten können, worin beispielsweise H, N, O, Cl, F durch die entsprechenden Isotope ersetzt sind.

Der Aufbau der erfindungsgemäßen FK-Anzeigen entspricht der für PSA-Anzeigen üblichen Geometrie, wie er im eingangs zitierten Stand der Technik beschrieben ist. Es sind Geometrien ohne Protrusions bevorzugt, insbesondere diejenigen, bei denen darüber hinaus die Elektrode auf der Colour Filter-Seite unstrukturiert ist und lediglich die Elektrode auf der TFT-Seite Schlitze aufweist. Besonders geeignete und bevorzugte Elektrodenstrukturen für PSA-VA-Anzeigen sind beispielsweise in US 2006/0066793 A1 beschrieben.

Die folgenden Beispiele erläutern die vorliegende Erfindung ohne sie zu begrenzen. Sie zeigen dem Fachmann jedoch bevorzugte Mischungskonzepte mit bevorzugt einzusetzenden Verbindungen und deren jeweiligen Konzentrationen sowie deren Kombinationen miteinander. Außerdem illustrieren die Beispiele, welche Eigenschaften und Eigenschftskombinationen zugänglich sind.

Folgende Abkürzungen werden verwendet:
(n, m, z: jeweils unabhängig voneinander 1, 2, 3, 4, 5 oder 6)

**Tabelle A**

| | |
|---|---|
| 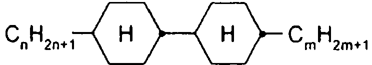 | 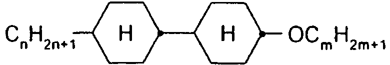 |
| CCH-nm | CCH-nOm |
| 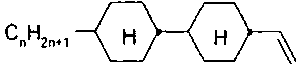 | 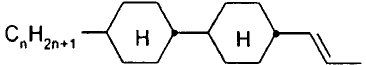 |
| CC-n-V | CC-n-V1 |
| 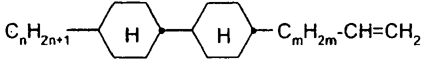 | 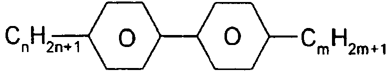 |
| CC-n-mV | PP-n-m |
| 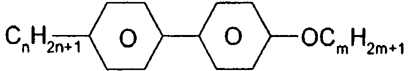 | 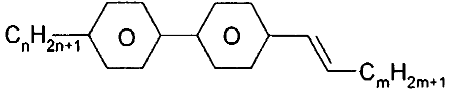 |
| PP-n-Om | PP-n-Vm |
| 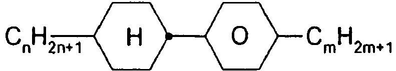 | 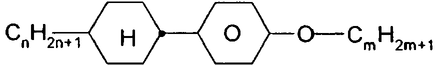 |
| PCH-nm | PCH-nOm |
| 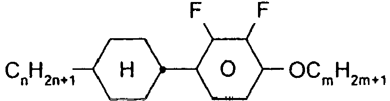 | 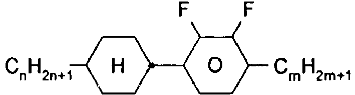 |
| CY-n-Om | CY-n-m |
| 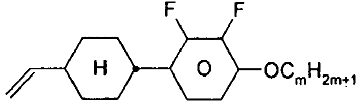 | 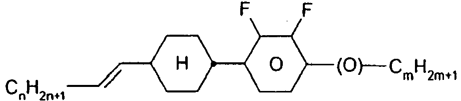 |
| CY-V-Om | CY-nV-(O)m |
| 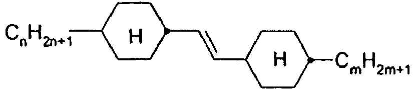 | 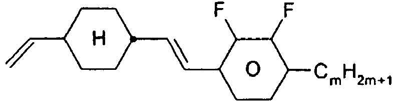 |
| CVC-n-m | CVY-V-m |
| 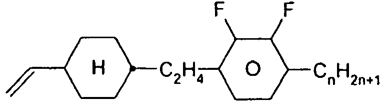 | 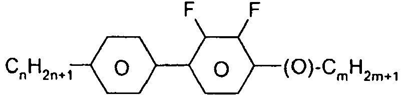 |
| CEY-V-m | PY-n-(O)m |
| 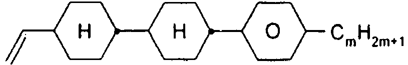 | 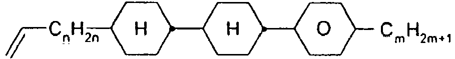 |
| CCP-V-m | CCP-Vn-m |
| 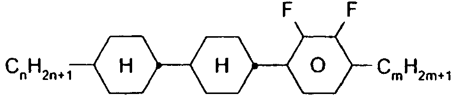 | 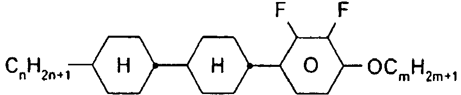 |
| CCY-n-m | CCY-n-Om |
| 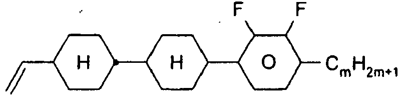 | 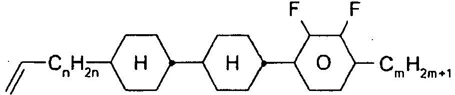 |
| CCY-V-m | CCY-Vn-m |
| 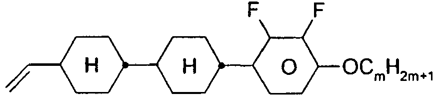 | 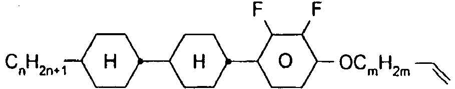 |
| CCY-V-Om | CCY-n-OmV |
| 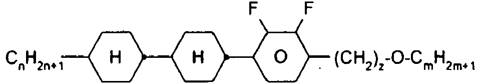 | 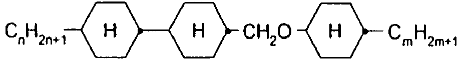 |
| CCY-n-zOm | CCOC-n-m |
| 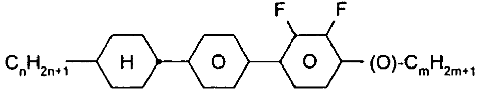 | 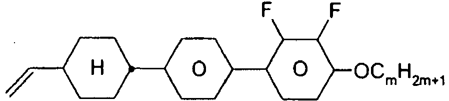 |
| CPY-n-(O)m | C PY-V-Om |
| 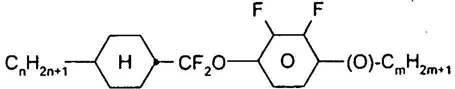 | 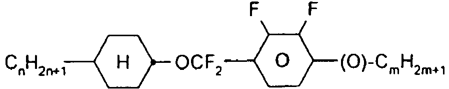 |
| CQY-n-(O)m | CQIY-n-(O)m |
| 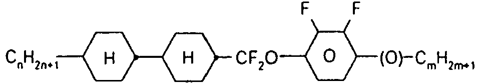 | 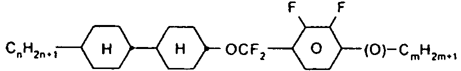 |
| CCQY-n-(O)m | CCQIY-n-(O)m |
| 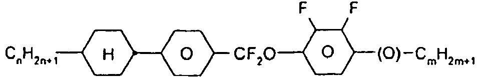 | 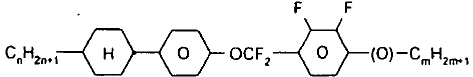 |
| CPQY-n-(O)m | CPQIY-n-Om |
| 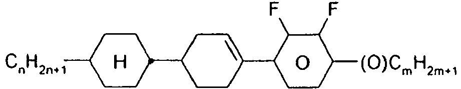 | 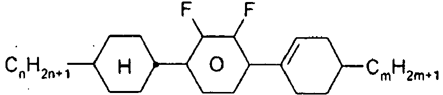 |
| CLY-n-(O)m | CYLI-n-m |
| 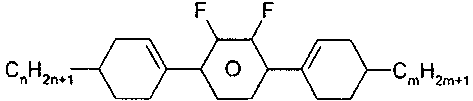 | 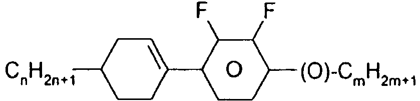 |
| LYLI-n-m | LY-n-(O)m |
| 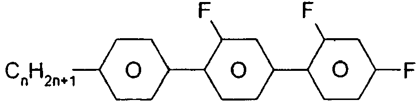 | 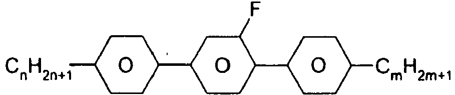 |
| PGIGI-n-F | PGP-n-m |
| 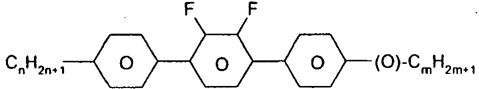 | 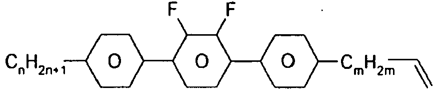 |
| PYP-n-(O)m | PYP-n-mV |
| 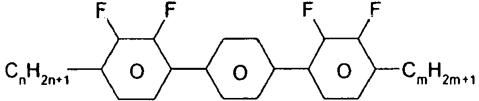 | 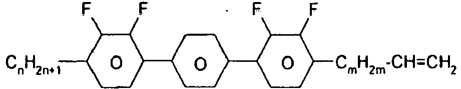 |
| YPY-n-m | YPY-n-mV |
| 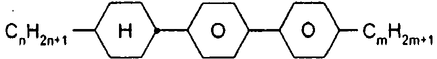 | 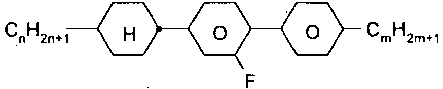 |
| BCH-nm | BCH-nmF |
| 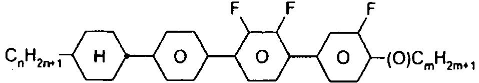 | 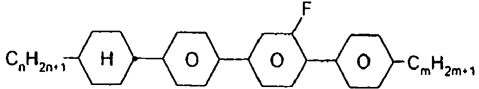 |
| CPYP-n-(O)m | CPGP-n-m |
| 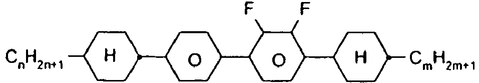 | 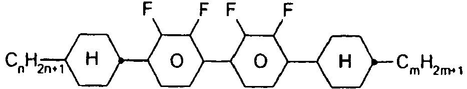 |
| CPYC-n-m | CYYC-n-m |
| 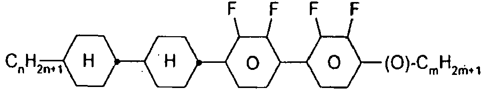 | 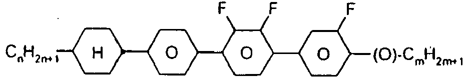 |
| CCYY-n-m | CPYG-n-(O)m |
| 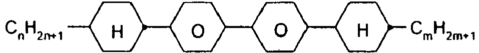 | 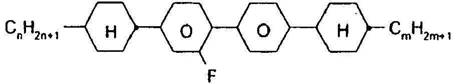 |
| CBC-nm | CBC-nmF |
| 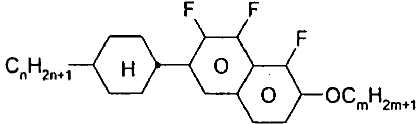 | 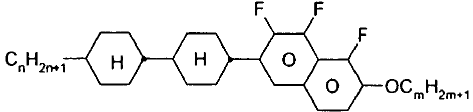 |
| CNap-n-Om | CCNap-n-Om |
| 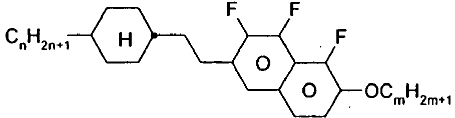 | 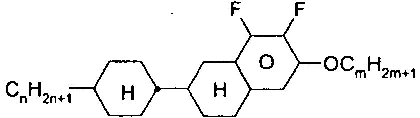 |
| CENap-n-Om | CTNap-n-Om |
| 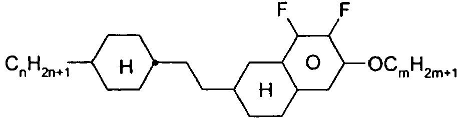 | 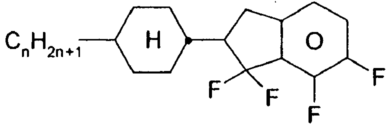 |
| CETNap-n-Om | CK-n-F |
| 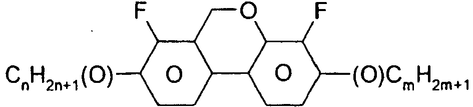 | 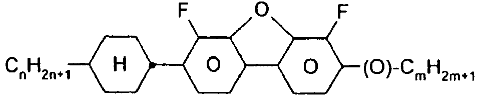 |
| DFDBC-n(O)-(O)m | C-DFDBF-n-(O)m |

In einer bevorzugten Ausführungsform der vorliegenden Erfindung enthalten die erfindungsgemäßen FK-Medien eine oder mehrere Verbindungen ausgewählt aus der Guppe bestehend aus Verbindungen der Tabelle A.

**Tabelle B**

| In der Tabelle B werden mögliche chirale Dotierstoffe angegeben, die den erfindungsgemäßen FK-Medien zugesetzt werden können. | |
|---|---|
| 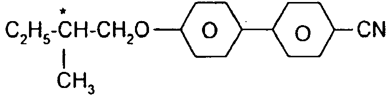 | 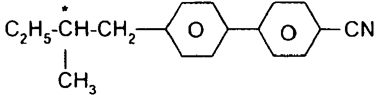 |
| **C15** | **CB 15** |
| 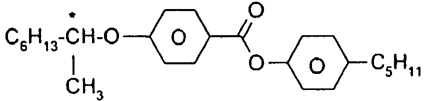 | 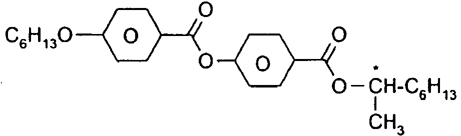 |
| **CM 21** | **R/S-811** |
| 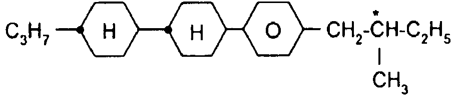 | 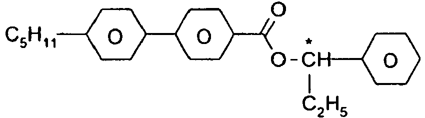 |
| **CM 44** | **CM 45** |
| 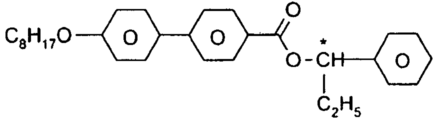 | 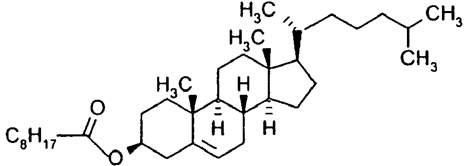 |
| **CM 47** | **CN** |
| 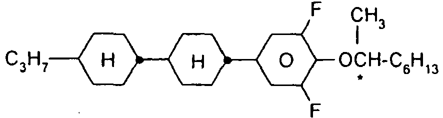 | 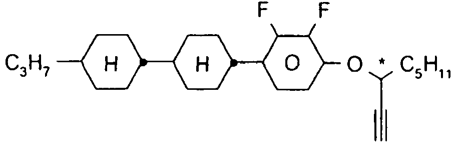 |
| **R/S-2011** | **R/S-3011** |
| 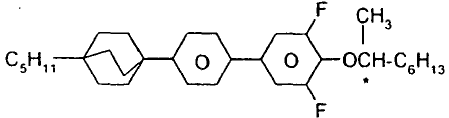 | 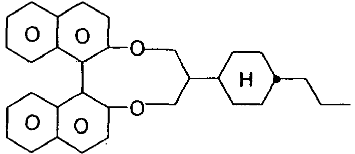 |
| **R/S-4011** | **R/S-5011** |
| 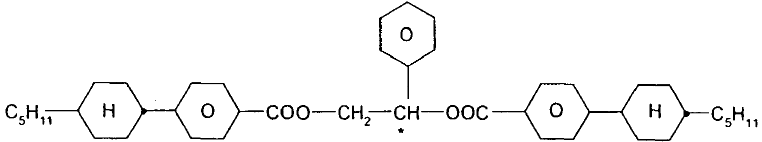 | |
| **R/S-1011** | |

Vorzugsweise enthalten die FK-Medien 0 bis 10 Gew.%, insbesondere 0,01 bis 5 Gew.%, besonders bevorzugt 0,1 bis 3 Gew.% an Dotierstoffen. Vorzugsweise enthalten die FK-Medien einen oder mehrere Dotierstoffe ausgewählt aus der Guppe bestehend aus Verbindungen der Tabelle B.

**Tabelle C**

| In der Tabelle C werden mögliche Stabilisatoren angegeben, die den erfindungsgemäßen FK-Medien zugesetzt werden können. (n bedeutet hier eine ganze Zahl von 1 bis 12, vorzugsweise 1, 2, 3, 4, 5, 6, 7 oder 8, endständige Methylgruppen sind nicht gezeigt). | |
|---|---|
| 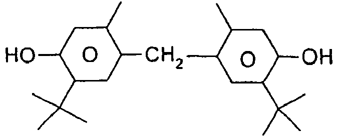 | 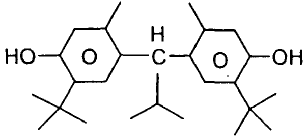 |
| 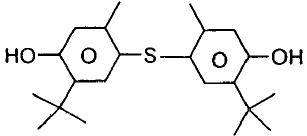 | 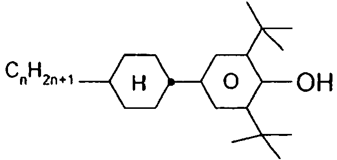 |
| 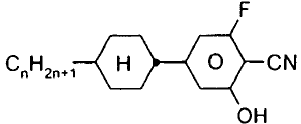 | 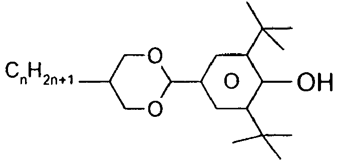 |
| 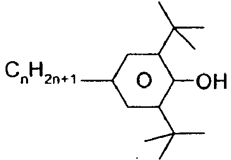 | 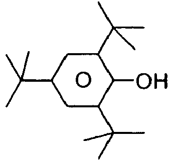 |
| 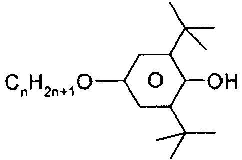 | 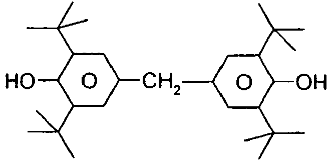 |
| 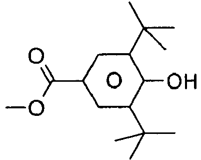 | 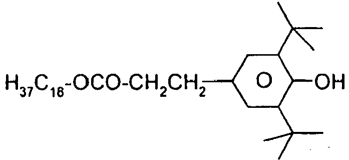 |
| 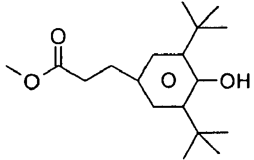 | 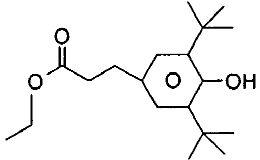 |
| 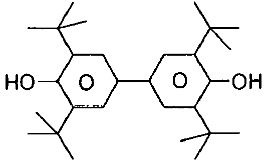 | 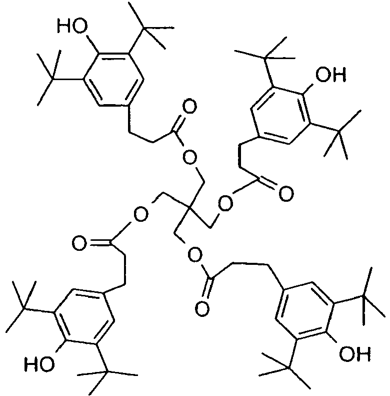 |
| 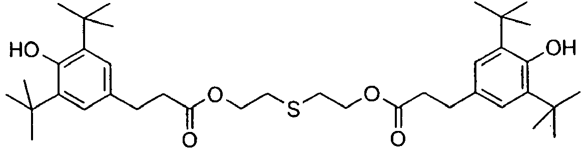 | |
| 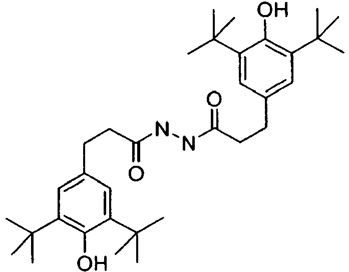 | 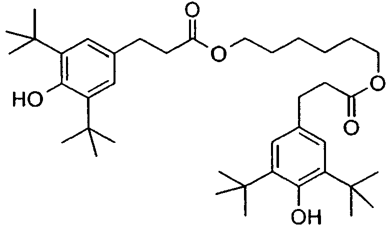 |
| 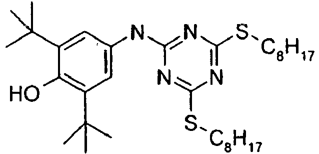 | 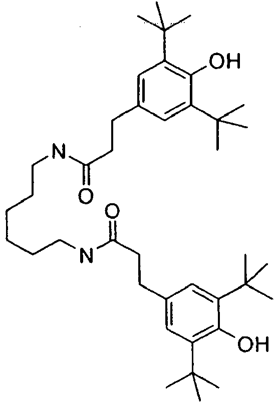 |
| 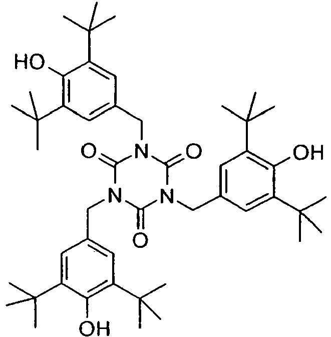 | 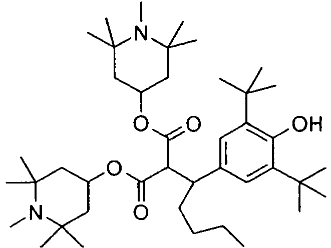 |
| 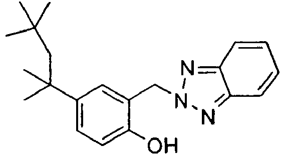 | 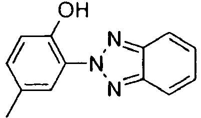 |
| 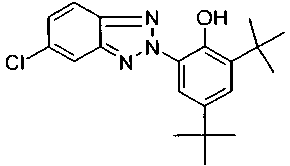 | 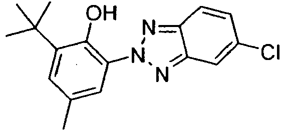 |
| 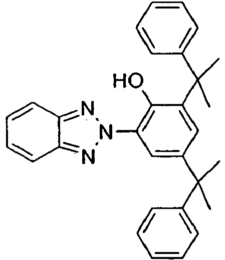 | 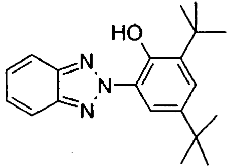 |
| 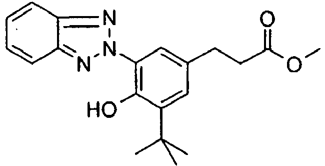 | 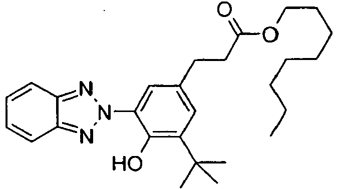 |
| 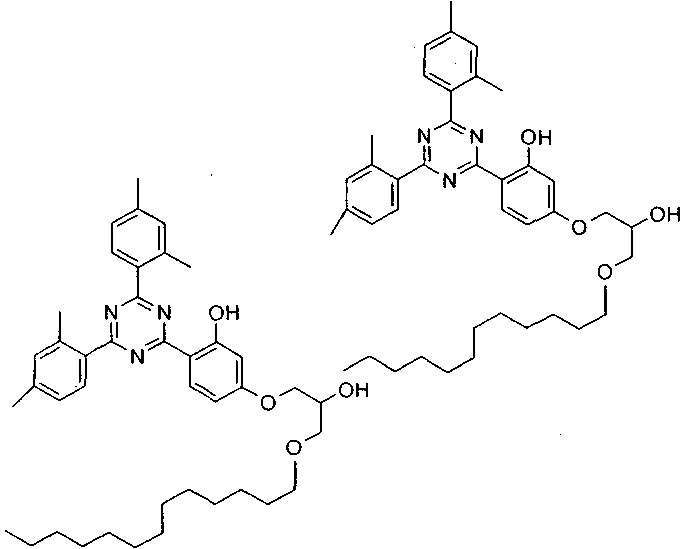 | |
| 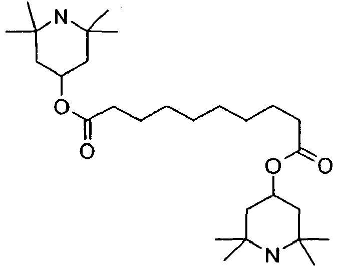 | 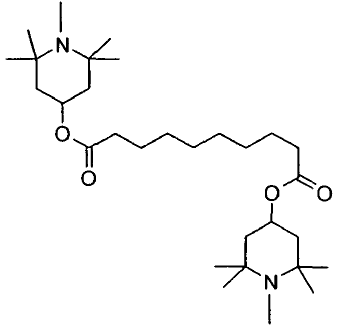 |

Vorzugsweise enthalten die FK-Medien 0 bis 10 Gew.%, insbesondere 1ppm bis 5 Gew.%, besonders bevorzugt 1ppm bis 1 Gew.% an Stabilisatoren. Vorzugsweise enthalten die FK-Medien einen oder mehrere Stabilisatoren ausgewählt aus der Gruppe bestehend aus Verbindungen der Tabelle C.

**Tabelle D**

| In der Tabelle D sind Beispielverbindungen zusammengestellt, die in den FK-Medien gemäß der vorliegenden Erfindung vorzugsweise als reaktive mesogene Verbindungen verwendet werden können. |
|---|
| 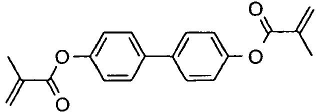 |
| **RM-1** |
| 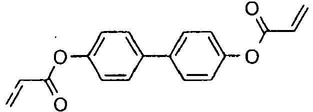 |
| **RM-2** |
| 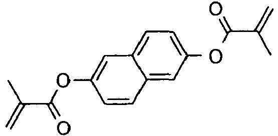 |
| **RM-3** |
| 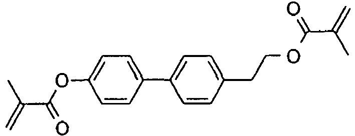 |
| **RM-4** |
| 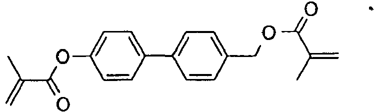 |
| **RM-5** |
| 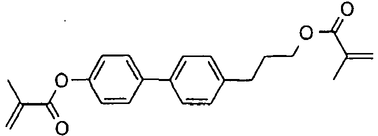 |
| **RM-6** |
| 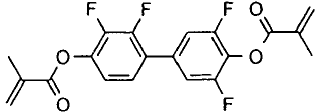 |
| **RM-7** |
| 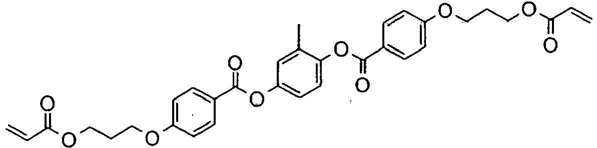 |
| **RM-8** |
| 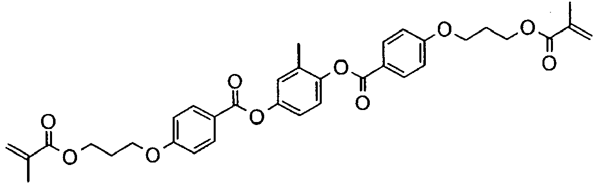 |
| **RM-9** |
| 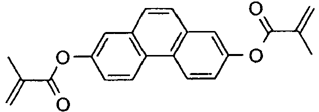 |
| **RM-10** |
| 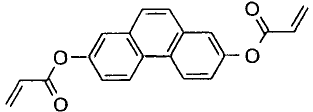 |
| **RM-11** |
| 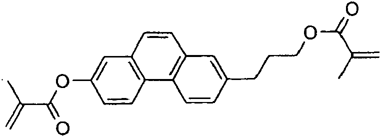 |
| **RM-12** |
| 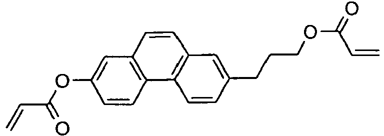 |
| **RM-13** |
| 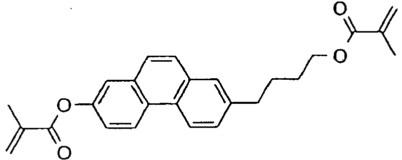 |
| **RM-14** |
| 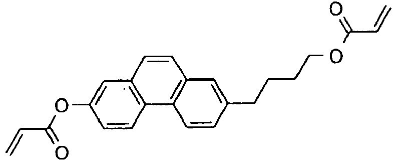 |
| **RM-15** |
| 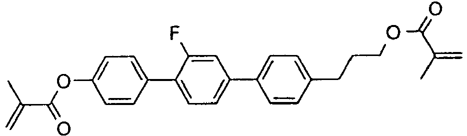 |
| **RM-16** |
| 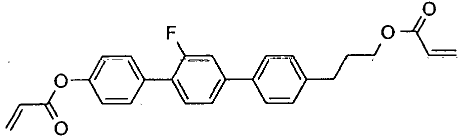 |
| **RM-17** |
| 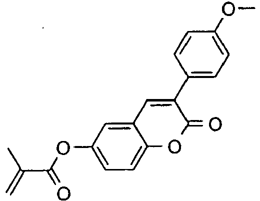 |
| **RM-18** |
| 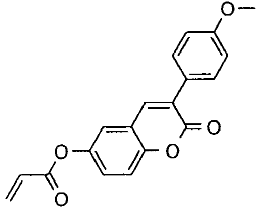 |
| **RM-19** |
| 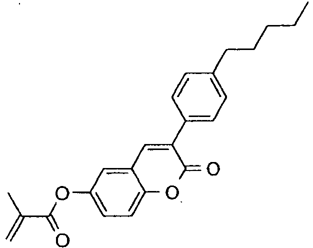 |
| **RM-20** |
| 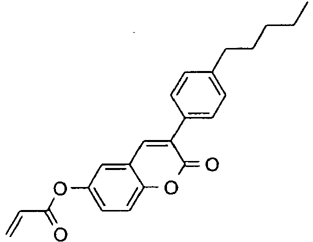 |
| **RM-21** |
| 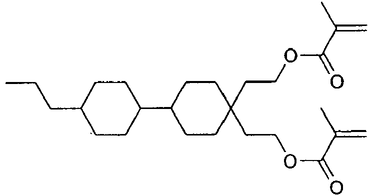 |
| **RM-22** |
| 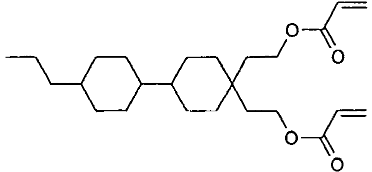 |
| **RM-23** |
| 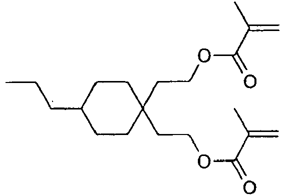 |
| **RM-24** |
| 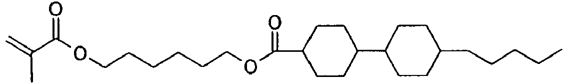 |
| **RM-25** |
| 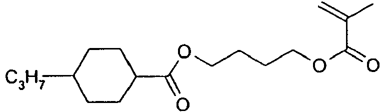 |
| **RM-26** |
| 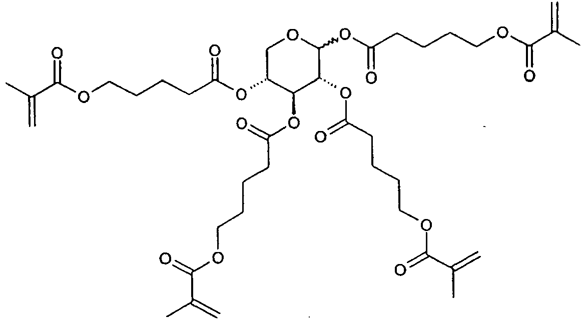 |
| **RM-27** |
| 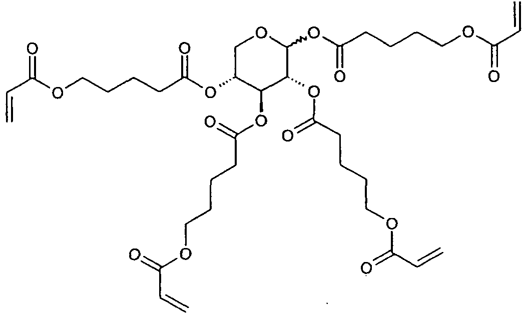 |
| **RM-28** |
| 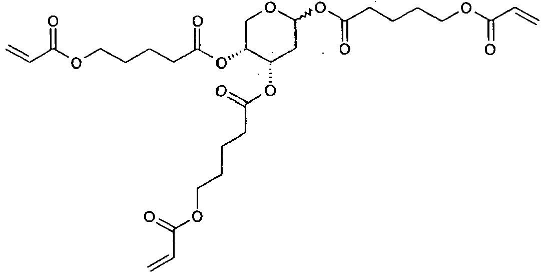 |
| **RM-29** |
| 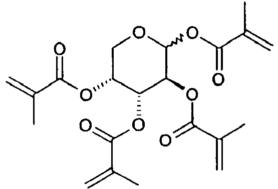 |
| **RM-30** |

In einer bevorzugten Ausführungsform der vorliegenden Erfindung enthalten die mesogenen Medien eine oder mehrere Verbindungen ausgewählt aus der Gruppe der Verbindungen der Tabelle D.

Außerdem werden folgende Abkürzungen und Symbole verwendet:
- Vₒ: Schwellenspannung, kapazitiv [V] bei 20°C,
- nₑ: außerordentlicher Brechungsindex bei 20°C und 589 nm,
- nₒ: ordentlicher Brechungsindex bei 20°C und 589 nm,
- Δn: optische Anisotropie bei 20°C und 589 nm,
- ε_{⊥}: dielektrische Permittivität senkrecht zum Direktor bei 20°C und 1 kHz,
- ε||: dielektrische Permittivität parallel zum Direktor bei 20°C und 1 kHz,
- Δε: dielektrische Anisotropie bei 20°C und 1 kHz,
- Kp., T(N,I): Klärpunkt [°C],
- γ₁: Rotationsviskosität bei 20°C [mPa·s],
- K₁: elastische Konstante, "splay"-Deformation bei 20°C [pN],
- K₂: elastische Konstante, "twist"-Deformation bei 20°C [pN],
- K₃: elastische Konstante, "bend"-Deformation bei 20°C [pN].

Soweit nicht explizit anders vermerkt, sind in der vorliegenden Anmeldung alle Konzentrationen in Gewichtsprozent angegeben und beziehen sich auf die entsprechende Gesamtmischung, enthaltend alle festen oder flüssigkristallinen Komponenten, ohne Lösungsmittel.

Soweit nicht explizit anders vermerkt, sind in der vorliegenden Anmeldung alle angegebenen Werte für Temperaturen, wie z. B. der Schmelzpunkt T(C,N), der Übergang von der smektischen (S) zur nematischen (N) Phase T(S,N) und der Klärpunkt T(N,I), in Grad Celsius (°C) angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar.

Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20°C und Δn wird bei 589 nm und Δε bei 1 kHz bestimmt, sofern nicht jeweils explizit anders angegeben.

Der Begriff "Schwellenspannung" bezieht sich für die vorliegende Erfindung auf die kapazitive Schwelle (V₀), auch Freedericksz-Schwelle genannt, sofern nicht explizit anders angegeben. In den Beispielen kann auch, wie allgemein üblich, die optische Schwelle für 10 % relativen Kontrast (V₁₀) angegeben werden.

Die zur Messung der kapazitiven Schwellenspannung verwendete Anzeige besteht aus zwei planparallelen Glasträgerplatten im Abstand von 20 µm, welche auf den Innenseiten jeweils ein Elektrodenschicht sowie eine darüberliegende, ungeriebene Orientierungsschicht aus Polyimid aufweisen, die eine homöotrope Randorientierung der Flüssigkristallmoleküle bewirken.

Die zur Messung der Tiltwinkel verwendete Anzeige bzw. Testzelle besteht aus zwei planparallelen Glasträgerplatten im Abstand von 4 µm, welche auf den Innenseiten jeweils eine Elektrodenschicht sowie eine darüberliegende Orientierungsschicht aus Polyimid aufweisen, wobei die beiden Polyimidschichten antiparallel zueinander gerieben werden und eine homöotrope Randorientierung der Flüssigkristallmoleküle bewirken.

Die polymerisierbaren Verbindungen werden in der Anzeige bzw. Testzelle durch Bestrahlung mit UVA-Licht (üblicherweise 365nm) einer definierten Intensität für eine vorgegebene Zeit polymerisiert, wobei gleichzeitig eine Spannung an die Anzeige angelegt wird (üblicherweise 10V bis 30V Wechselstrom, 1 kHz). In den Beispielen wird, falls nicht anders angegeben, eine Quecksilberdampflampe mit 28 mW/cm² verwendet, die Intensität wird mit einem Standard-UV-Meter (Fabrikat Ushio UNI meter) gemessen, der mit einem Bandpassfilter bei 365nm ausgerüstet ist.

Der Tiltwinkel wird per Drehkristall-Experiment (Autronic-Melchers TBA-105) bestimmt. Ein niedriger Wert (d.h. eine große Abweichung vom 90°-Winkel) entspricht dabei einem großen Tilt.

Der VHR -Wert wird wie folgt gemessen: Zur FK-Host-Mischung werden 0.3% einer polymerisierbaren monomeren Verbindung zugesetzt, und die dadurch entstandene Mischung in TN-VHR-Testzellen gefüllt (90° gerieben, Orientierungsschicht TN-Polyimid, Schichtdicke d≈4µm). Der HR-Wert wird nach 5min bei 100°C vor und nach 2h UV-Belastung (suntest) bei 1V, 60Hz, 64µs pulse bestimmt (Messgerät: Autronic-Melchers VHRM-105).

### Beispiel 1

### 2-Methylacrylsäure-7-(2-methyl-acryloyloxy)-9,10-dihydro-phenanthren-2-ylester

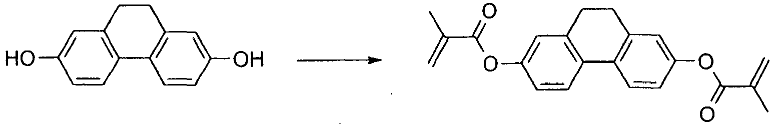

7,50 g (35,3 mmol) 9,10-Dihydro-phenanthren-2,7-diol (CAS-Nr. 99896-39-6) werden in 100 ml Dichlormethan vorgelegt, mit 15 ml Pyridin versetzt und unter Eiskühlung eine Lösung von 13,0 g (0,124 mol) Methacrylsäurechlorid in 100 ml Dichlormethan zutropfen gelassen. Die Kühlung wird entfernt und der Ansatz über Nacht bei Raumtemp. rühren gelassen. Der ausgefallene Niederschlag wird abfiltriert, das Filtrat i. Vak eingeengt und der Rückstand mit Dichlormethan über Kieselgel filtriert. Kristallisation aus Heptan/Toluol liefert 2-Methylacrylsäure-7-(2-methylacryloyloxy)-9,1 0-dihydro-phenanthren-2-ylester als farblosen Feststoff vom Schmp. 116 °C.

### Beispiel 2

### 2-Methyl-acrylsäure-4,7-difluor-8-(2-methyl-acryloyloxy)-6H-benzo[c]chromen-3-ylester

### 2.1 4,7-Difluor-6H-benzo[c]chromen-3,8-diol

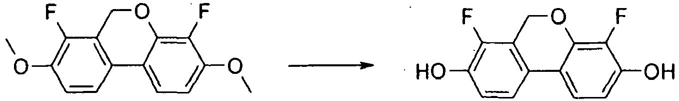

5,23 g (18,8 mmol) **4**,7-Difluor-3,8-dimethoxy-6H-benzo[c]chromen (hergestellt nach: Taugerbeck, Klasen-Memmer, W02004076438) werden in 70 ml Dichlormethan vorgelegt und unter Eiskühlung tropfenweise mit einer Lösung von 5 ml (52,7 mmol) Bortribromid in 10 ml Dichlormethan versetzt. Nach 1 h wird die Kühlung entfernt und der Ansatz 3 h bei Raumtemp. rühren gelassen und auf eiskalte 1 M Natronlauge gegeben. Die wäßrige Phase wird abgetrennt und dreimal mit Essigester extrahiert. Die vereinigten org. Phasen werden mit ges. Natriumchloridlsg. gewaschen und über Natriumsulfat getrocknet. Das Lösungsmittel wird i.Vak. entfernt, der Rückstand mit Toluol/Essigester (3:2) über Kieselgel filtiert, das Rohprodukt mit Heptan/Essigester (2:1) heiß ausgerührt und nach Kühlung auf 8 °C abgesaugt. Man erhält das 4,7-Difluor-6H-benzo[c]chromen-3,8-diol als farblosen Feststoff.
¹H-NMR (300 MHz, DMSO-d₆)
δ = 5,23 ppm (s, 2 H, CH₂O), 6,62 (t, J = 8,4 Hz, 1 H, Ar-H), 6,95 (t, J = 8,8 Hz, 1 H, Ar-H), 7,36 (d, J = 8,4 Hz, 1 H, Ar-H), 7,36 (d, J = 8,8 Hz, 1 H, Ar-H), 10,0 (s, 2 H, Ar-OH).

### 2.2 2-Methyl-acrylsäure-4,7-difluor-8-(2-methyl-acryloyloxy)-6H-benzo[c]chromen-3-ylester

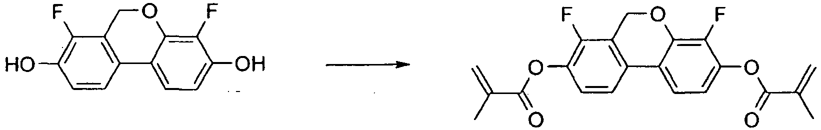

In Analogie zu Beispiel 1 erhält man aus 4,7-Difluor-6H-benzo[c]chromen-3,8-diol den 2-Methyl-acrylsäure-4,7-difluor-8-(2-methyl-acryloyloxy)-6H-benzo[c]chromen-3-ylester als farblosen Feststoff vom Schmp. 202 °C.

### Beispiel 3

### 2-Brom-7-iod-9,10-dihydrophenanthren

### 3.1 2-Iod-9,10-dihydrophenanthren

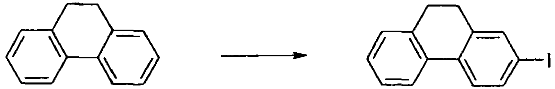

25,0 g (0,139 mol) 9,10-Dihydrophenanthren, 6,30 g (27,6 mmol) Periodsäure und 17,0 g (67,0 mmol) Iod werden in einer Lösung von 4 ml konz. Schwefelsäure und 28 ml Wasser in 140 ml Eisessig 1 h auf 70 °C erhitzt. Anschließend wird die Lösung auf Eiswasser gegeben und dreimal mit Essigester extrahiert. Die vereinigten org. Phasen werden mit ges. Natriumhydrogencarbonatlsg gewaschen und über Natriumsulfat getrocknet. Das Lösungsmittel wird i. Vak. entfernt und der Rückstand bei 170 °C und 0,3 mbar im Kugelrohr destilliert. Man erhält 2-lod-9,10-dihydrophenanthren als gelbes Öl.
¹H-NMR (400 MHz, CDCl₃)
δ = 2,83 ppm (m_{c}, 4 H, -CH₂CH₂-), 7,20 - 7,32 (m, 3 H), 7,46 (d, J = 8,2 Hz, 1 H), 7,59 (d, J = 1,9 Hz, 1 H), 7,62 (dd, J = 1,9 Hz, J = 8,3 HZ, 1 H), 7,70 (d, J = 7,8 Hz, 1 H).
MS (EI) m/z (%) = 306 (98) [M⁺], 178 (100) [M⁺ - HI].

### 3.2 2-Brom-7-iod-9,10-Dihydrophenanthren

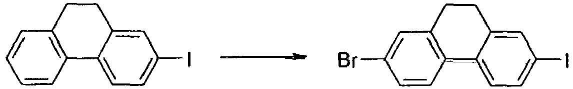

9,5 g (27,9 mmol) 2-Iod,9,10-dihydrophenanthren werden in 40 ml Trimethylphosphat gelöst und bei 20 °C tropfenweise mit einer Lösung von 2 ml (39 mmol) Brom in 10 ml Trimethylphosphat versetzt. Nach 90 min wird der Ansatz auf Eiswasser gegeben, überschüssiges Brom durch Zugabe von Natriumhydrogensulfitlsg. reduziert und das ausgefallene Produkt wird abgesaugt, in Toluol aufenommen, mit ges. Natriumhydrogencarbonatlsg. gewaschen und über Natriumsulfat getrocknet. Das Lösungsmittel wird i. Vak. entfernt und der Rückstand aus Heptan/Toluol umkristallisiert. Man erhält 2-Brom-7-iod-9,10-Dihydrophenanthren als farblose Kristalle.
¹H-NMR (400 MHz, CDCl₃)
δ = 2,81 ppm (s, 4 H, -CH₂CH₂-), 7,37 (d, J = 2,0 Hz, 1 H, Ar-H), 7,40 (d, J = 2,0 Hz, 1 H, Ar-H), 7,43 (d, J = 1,9 Hz, 1 H, Ar-H), 7,55 (d, J = 8,2 Hz, 1 H, Ar-H), 7,59 (d, J = 1,7 Hz, 1 H, Ar-H), 7,62 (dd, J = 1,9 Hz, J = 8,2 Hz, 1 H, Ar-H).
MS (EI) m/z (%) = 384 (83) [M⁺], 178 (100) [M⁺ - HI -HBr].

### Beispiel 4

### 2-Methyl-acrylsäure-7-[4-(2-methyl-acryloyloxy)-butyl]-9, 10-dihydrophenanthren-2-yl ester

Geeignete Vorstufen sind das 2,7-Diiod-9,10-dihydrophenanthren (s. Cho et al., Chemistry of Materials (2008), 20(20), 6289-6291), 2,7-Dibrom-9,10-dihydrophenanthren (s. D. E. Pearson, Synthesis 1976, 621-623) oder 2-Brom-7-iod-9,10-dihydrophenanthren.

### 4.1 7-Iod-9,10-dihydrophenanthren-2-ol

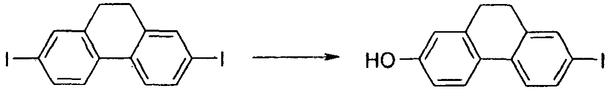

8,8 g (20,4 mmol) 2,7-Diiod-9,10-dihydrophenanthren werden in 250 ml THF gelöst und nach Zugabe von 7,0 ml (30,5 mmol) Triisopropylborat bei -70 °C mit 16,5 ml einer 15proz. Lsg. von n-Butyllithium in Hexan versetzt. Nach 1 h wird der Ansatz mit 2 N Salzsäure hydrolysiert und auf Raumtemperatur erwärmt. Die Lösung wird zweimal mit MTB-Ether extrahiert, über Natriumsulfat getrocknet, das Lösungsmittel i. Vak. entfernt und das erhaltene Rohprodukt in 80 ml Toluol und 30 ml 2 N Natronlauge unter kräftigem Rühren suspendiert. Nach Zugabe von 8 ml 30% Wasserstoffperoxid wird der Ansatz unter leichter Kühlung bei 30-40 °C 30 min gerührt, mit 200 ml Wasser versetzt und mit 2 N Salzsäure angesäuert. Die wäßr. Phase wird abgetrennt und dreimal mit Essigester extrahiert. Die vereinigten org. Phasen werden mit verd. Ammoniumeisen(II) sulfatlsg. gewaschen und über Natriumsulfat getrocknet. Das Lösungsmittel wird. i. Vak. entfernt und der Rückstand mit Dichlormethan über Kieselgel filtriert. Man erhält 7-Iod-9,10-dihydro-phenanthren-2-ol als farblosen Feststoff.
¹H-NMR (400 MHz, DMSO-d₆)
δ = 2,72 ppm (m_{c}, 4 H, -CH₂CH₂-), 6.66 (6, J = 2.4 Hz, 1 H, Ar-H), 6.70 (dd, J = 2.4 Hz, J = 8.5 Hz, 1 H, Ar-H), 7.47 (d, J = 8.0 Hz, 1 H, Ar-H), 7.60 (m_{c}, 2 H, Ar-H), 9.57 (s, br., 1 H, OH).

### 4.2 7-(4-Hydroxybut-1-inyl)-9,10-dihydrophenanthren-2-ol

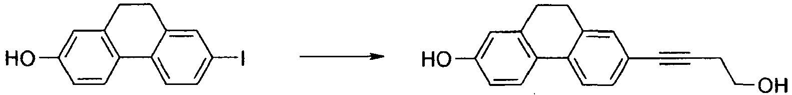

9,0 g (24,6 mmol) 7-Iod-9,10-dihydrophenanthren-2-ol werden in 100 ml THF vorgelegt, 1,0 g (1,43 mmol) Bis(triphenylphosphin) palladium(II) chlorid, 0,3 g (1,58 mmol) Kupfer(I)iodid und 11 ml Diisopropylamin hinzugegeben und anschließend unter leichter Kühlung bei max. 30 °C eine Lösung von 3,0 g (42,8 mmol) But-3-in-1-ol in 20 ml THF zutropfen gelassen. Der Ansatz wird 1,5 h bei Raumtemp. rühren gelassen, auf Wasser gegeben und mit 2 N Salzsäure angesäuert. Die wäßr. Phase wird abgetrennt und dreimal mit Essigester extrahiert. Die vereinigten org. Phasen werden mit Wasser gewaschen, über Natriumsulfat getrocknet und das Lösungsmittel i. Vak. entfernt. Das Rohprodukt wird mit Dichlormethan/Essigester (3:1) über Kieselgel filtriert und aus Toluol umkristallisiert. Man erhält 7-(4-Hydroxybut-1-inyl)-9,10-dihydrophenanthren-2-ol als farblosen Feststoff.

### 4.3 7-(4-Hydroxybutyl)-9,10-dihydrophenanthren-2-ol

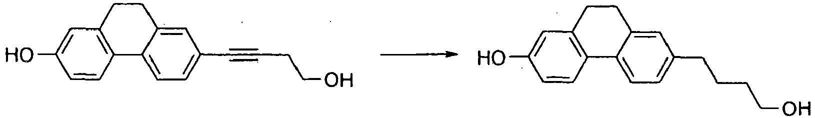

7-(4-Hydroxy-but-1-inyl)-9,10-dihydrophenanthren-2-ol wird in THF gelöst und an Palladium-Aktivkohlekatalysator bis zum Stillstand hydriert. Der Katalysator wird abfiltriert, das Lösungsmittel i. Vak. entfernt und der Rückstand aus Toluol umkristallisiert. Man erhält 7-(4-Hydroxybutyl)-9,10-dihydrophenanthren-2-ol als farblose Kristalle.

### 4.4 2-Methyl-acrylsäure-7-[4-(2-methyl-acryloyloxy)-butyl]-9,10-dihydrophenanthren-2-yl-ester

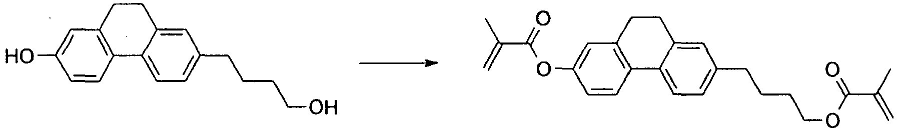

In Analogie zu Beispiel 1 erhält man aus 7-(4-Hydroxybutyl)-9,10-dihydrophenanthren-2-ol den 2-Methyl-acrylsäure-7-[4-(2-methyl-acryloyloxy)-butyl]-9,1 0-dihydrophenanthren-2-ylester als farbloses Öl.

### Beispiel 5-197

Die Synthese für die Verbindungen des Beispiels (32) ist im folgenden konkret beschrieben.

### Beispiel 32: 2-Methyl-acrylsäure-4-[9,9-dimethyl-7-(2-methyl-acryloyloxy)-9H-fluoren-2-yl]-but-3-inylester

### 32.1 7-Brom-9,9-dimethyl-9H-fluoren-2-ol

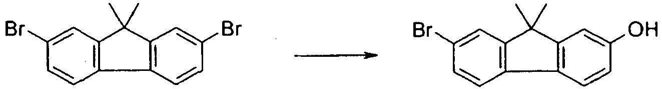

21,2 g (58,6 mmol) 2,7-Dibrom-9,9-dimethyl-9H-fluoren (CAS-Nr. 28320-32-3) und 19,0 ml (82,8 mmol) Triisopropylborat werden in 500 ml THF gelöst und bei -70 °C mit 56 ml (89 mmol) einer 15proz. Lösung von n-Butyllithium in n-Hexan versetzt. Nach 2 h werden 200 ml 2 M Salzsäure hinzugegeben, der Ansatz auf Raumtemp. auftauen gelassen und dreimal mit MTB-Ether extrahiert. Die vereinigten org. Phasen werden über Natriumsulfat getrocknet, das Lösungsmittel i. Vak. entfernt und der Rückstand in 400 ml Toluol aufgenommen. Nach Zugabe von 85 ml 2 M Natronlauge werden unter kräftigem Rühren 15,5 ml 30proz. Wasserstoffperoxid so zugegeben, daß die Temp. 40 °C nicht übersteigt. Nach beendeter Zugabe wird der Ansatz noch 30 min nachgerührt, auf 500 ml Wasser gegeben und mit 2 M Salsäure angesäuert. Die wäßrige Phase wird abgetrennt und dreimal mit Essigester extrahiert. Die vereinigten org. Phasen werden mit verd. Ammoniumeisen(II)sulfatlsg. und Wasser gewaschen, über Natriumsulfat getrocknet und eingeengt. Filtration des Rohproduktes mit Toluol/Essigester (9:1) über Kieselgel liefert 7-Brom-9,9-dimethyl-9H-fluoren-2-ol als farblosen Feststoff.
1 H-NMR (400 MHz, CDCl3)
δ = 1.44 ppm (s, 6 H, Me), 4,81 (s, 1 H, OH), 6,79 (dd, J = 2,4 Hz, J = 8,2 Hz, 1 H, Ar-H), 6,88 (d, J = 2,4 Hz, 1 H, Ar-H), 7,41 (AB-dd, J = 1,7 Hz, 8,1 Hz, 1H, Ar-H), 7,46 (AB-d, J = 8,1 Hz, 1 H, Ar-H), 7,50 (d, J = 1,7 Hz, 1 H, Ar-H), 7,53 (d, J = 8,1 Hz, 1 H, Ar-H).

### 32.2 7-(4-Hydroxy-but-1-inyl)-9,9-dimethyl-9H-fluoren-2-ol

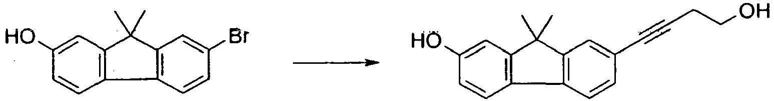

14,7 g (0,048 mol) 7-Brom-9,9-dimethyl-9H-fluoren-2-ol, 1,3 g (1,85 mmol) Bis(triphenylphosphin)palldium(II)chlorid und 0,5 g (2,6 mmol) Kupfer(I)iodid werden in 200 ml THF und 20 ml Diisopropylamin vorgelegt und unter Rückfluß innerhalb von 2 h mit einer Lösung von 9,5 ml (0,13 mmol) But-3-in-1-ol in 50 ml THF versetzt. Nach beendeter Zugabe wird der Ansatz noch 1 h unter Rückfluß erhitzt, auf Wasser gegeben, mit verd. Salzsäure angesäuert und dreimal mit Essigester extrahiert. Die vereinigten org. Phasen werden über Natriumsulfat getrocknet und das Lösungsmittel i. Vak. entfernt. Chromatographie des Rohproduktes mit Toluol/Essigester (4:1) an Kieselgel liefert 7-(4-Hydroxy-but-1-inyl)-9,9-dimethyl-9H-fluoren-2-ol als farbloses Öl.
1H-NMR (400 MHz, CDCl₃): δ = 1,44 ppm (s, 6 H, Me), 2,73 (t, J = 6,2 Hz, 2 H, C≡CC*H*₂), 3,85 (q, J = 6,2 Hz, 2 H, CH₂C*H*₂OH), 5,17 (s, 1 H, OH), 6,80 (dd, J = 2,4 Hz, J = 8,2 Hz, 1 H, Ar-H), 6,89 (d J = 2,4 Hz, 1 H, Ar-H), 7,36 (dd, J = 1,4 Hz, J = 7,8 Hz, 1 H, Ar-H), 7,44 (d, J = 0,8 Hz, 1 H, Ar-H), 7,52 (d, J = 7,8 Hz, 1 H, Ar-H), 7,54 (d, J = 8,2 Hz, 1 H, Ar-H).

### 32.3. 7-(4-Hydroxy-butyl)-9,9-dimethyl-9H-fluoren-2-ol

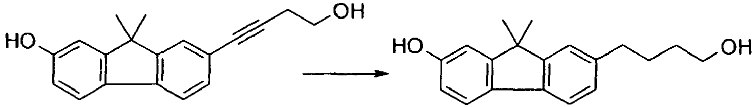

7-(4-Hydroxy-but-1-inyl)-9,9-dimethyl-9H-fluoren-2-ol wird in THF an Palladium-Aktivkohle bis zum Stillstand hydriert. Der Katalysator wird abfiltriert, das Lösungsmittel i.Vak. entfernt und ohne weitere Aufreinigung in der nächsten Stufe eingesetzt.
1H-NMR (400 MHz, CDCl₃): δ = 1,39 ppm (s, br. 1 H, OH), 1,43 (s, 6 H, Me), 1,61-1,79 (m, 4 H, C*H*₂) 2,70 (t, J = 7,3 Hz, 2 H, Ar-C*H*₂-CH₂-), 3,70 (t, J = 6,5 Hz, 2 H, -CH₂C*H*₂OH), 5,29 (s, 1 H, OH), 6,77 (dd, J = 2,4 Hz, J = 8,2 Hz, 1 H, Ar-H), 6,88 (d J = 2,4 Hz, 1 H, Ar-H), 7,11 (dd, J = 1,5 Hz, J = 7,7 Hz, 1 H, Ar-H), 7,19 (d, J = 1,1 Hz, 1 H, Ar-H), 7,51 (d, J = 7,7 Hz, 1 H, Ar-H), 7,51 (d, J = 8,2 Hz, 1 H, Ar-H).

### 32.4 2-Methyl-acrylsäure-9,9-dimethyl-7-[4-(2-methyl-acryloyloxy)-butyl]-9H-fluoren-2-ylester

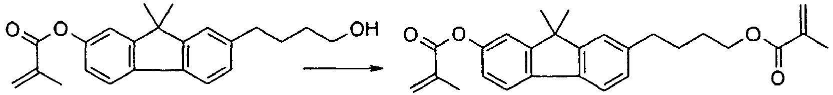

In Analogie zu Beispiel 1 erhält man aus 7-(4-Hydroxy-butyl)-9,9-dimethyl-9H-fluoren-2-ol den 2-Methyl-acrylsäure-9,9-dimethyl-7-[4-(2-methyl-acryloyloxy)-butyl]-9H-fluoren-2-ylester als farbloses Öl. Glasübergang Tg -29 I.

In Analogie zu den in den vorstehenden Beispielen beschriebenen Verfahren werden die folgenden Verbindungen erhalten (Phe = 1,4-Phenylen):

Verbindungen der folgenden Formel (Nr. 5-100):

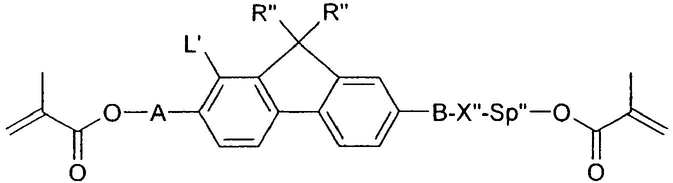

| **Nr.** | **A** | **L'** | **R"** | **B** | **X"** | **Sp"** |
|---|---|---|---|---|---|---|
| 5 | - | H | H | - | - | -CH₂- |
| 6 | - | H | H | - | - | -(CH₂)₂ |
| 7 | - | H | H | - | - | -(CH₂)₃- |
| 8 | - | H | H | - | - | -(CH₂)₄- |
| 9 | - | H | H | - | O | -CH₂- |
| 10 | - | H | H | - | O | -(CH₂)₂ |
| 11 | - | H | H | - | O | -(CH₂)₃- |
| 12 | - | H | H | - | O | -(CH₂)₄- |
| 13 | Phe | H | H | - | - | -CH₂- |
| 14 | Phe | H | H | - | - | -(CH₂)₂ |
| 15 | Phe | H | H | - | - | -(CH₂)₃- |
| 16 | Phe | H | H | - | - | -(CH₂)₄- |
| 17 | Phe | H | H | - | O | -CH₂- |
| 18 | Phe | H | H | - | O | -(CH₂)₂ |
| 19 | Phe | H | H | - | O | -(CH₂)₃- |
| 20 | Phe | H | H | - | O | -(CH₂)₄- |
| 21 | - | H | H | Phe | - | -CH₂- |
| 22 | - | H | H | Phe | - | -(CH₂)₂ |
| 23 | - | H | H | Phe | - | -(CH₂)₃- |
| 24 | - | H | H | Phe | - | -(CH₂)₄- |
| 25 | - | H | H | Phe | O | -CH₂- |
| 26 | - | H | H | Phe | O | -(CH₂)₂ |
| 27 | - | H | H | Phe | O | -(CH₂)₃- |
| 28 | - | H | H | Phe | O | -(CH₂)₄- |
| 29 | - | H | CH₃ | - | - | -CH₂- |
| 30 | - | H | CH₃ | - | - | -(CH₂)₂ |
| 31 | - | H | CH₃ | - | - | -(CH₂)₃- |
| 32 | - | H | CH₃ | - | - | -(CH₂)₄- |
| 33 | - | H | CH₃ | - | O | -CH₂- |
| 34 | - | H | CH₃ | - | O | -(CH₂)₂ |
| 35 | - | H | CH₃ | - | O | -(CH₂)₃- |
| 36 | - | H | CH₃ | - | O | -(CH₂)₄- |
| 37 | Phe | H | CH₃ | - | - | -CH₂- |
| 38 | Phe | H | CH₃ | - | - | -(CH₂)₂ |
| 39 | Phe | H | CH₃ | - | - | -(CH₂)₃- |
| 40 | Phe | H | CH₃ | - | - | -(CH₂)₄- |
| 41 | Phe | H | CH₃ | - | O | -CH₂- |
| 42 | Phe | H | CH₃ | - | O | -(CH₂)₂ |
| 43 | Phe | H | CH₃ | - | O | -(CH₂)₃- |
| 44 | Phe | H | CH₃ | - | O | -(CH₂)₄- |
| 45 | - | H | CH₃ | Phe | - | -CH₂- |
| 46 | - | H | CH₃ | Phe | - | -(CH₂)₂ |
| 47 | - | H | CH₃ | Phe | - | -(CH₂)₃- |
| 48 | - | H | CH₃ | Phe | - | -(CH₂)₄- |
| 49 | - | H | CH₃ | Phe | O | -CH₂- |
| 50 | - | H | CH₃ | Phe | O | -(CH₂)₂ |
| 51 | - | H | CH₃ | Phe | O | -(CH₂)₃- |
| 52 | - | H | CH₃ | Phe | O | -(CH₂)₄- |
| 53 | - | F | H | - | - | -CH₂- |
| 54 | - | F | H | - | - | -(CH₂)₂ |
| 55 | - | F | H | - | - | -(CH₂)₃- |
| 56 | - | F | H | - | - | -(CH₂)₄- |
| 57 | - | F | H | - | O | -CH₂- |
| 58 | - | F | H | - | O | -(CH₂)₂ |
| 59 | - | F | H | - | O | -(CH₂)₃- |
| 60 | - | F | H | - | O | -(CH₂)₄- |
| 61 | Phe | F | H | - | - | -CH₂- |
| 62 | Phe | F | H | - | - | -(CH₂)₂ |
| 63 | Phe | F | H | - | - | -(CH₂)₃- |
| 64 | Phe | F | H | - | - | -(CH₂)₄- |
| 65 | Phe | F | H | - | O | -CH₂- |
| 66 | Phe | F | H | - | O | -(CH₂)₂ |
| 67 | Phe | F | H | - | O | -(CH₂)₃- |
| 68 | Phe | F | H | - | O | -(CH₂)₄- |
| 69 | - | F | H | Phe | - | -CH₂- |
| 70 | - | F | H | Phe | - | -(CH₂)₂ |
| 71 | - | F | H | Phe | - | -(CH₂)₃- |
| 72 | - | F | H | Phe | - | -(CH₂)₄- |
| 73 | - | F | H | Phe | O | -CH₂- |
| 74 | - | F | H | Phe | O | -(CH₂)₂ |
| 75 | - | F | H | Phe | O | -(CH₂)₃- |
| 76 | - | F | H | Phe | O | -(CH₂)₄- |
| 77 | - | F | CH₃ | - | - | -CH₂- |
| 78 | - | F | CH₃ | - | - | -(CH₂)₂ |
| 79 | - | F | CH₃ | - | - | -(CH₂)₃- |
| 80 | - | F | CH₃ | - | - | -(CH₂)₄- |
| 81 | - | F | CH₃ | - | O | -CH₂- |
| 82 | - | F | CH₃ | - | O | -(CH₂)₂ |
| 83 | - | F | CH₃ | - | O | -(CH₂)₃- |
| 84 | - | F | CH₃ | - | O | -(CH₂)₄- |
| 85 | Phe | F | CH₃ | - | - | -CH₂- |
| 86 | Phe | F | CH₃ | - | - | -(CH₂)₂ |
| 87 | Phe | F | CH₃ | - | - | -(CH₂)₃- |
| 88 | Phe | F | CH₃ | - | - | -(CH₂)₄- |
| 89 | Phe | F | CH₃ | - | O | -CH₂- |
| 90 | Phe | F | CH₃ | - | O | -(CH₂)₂ |
| 91 | Phe | F | CH₃ | - | O | -(CH₂)₃- |
| 92 | Phe | F | CH₃ | - | O | -(CH₂)₄- |
| 93 | - | F | CH₃ | Phe | - | -CH₂- |
| 94 | - | F | CH₃ | Phe | - | -(CH₂)₂ |
| 95 | - | F | CH₃ | Phe | - | -(CH₂)₃- |
| 96 | - | F | CH₃ | Phe | - | -(CH₂)₄- |
| 97 | - | F | CH₃ | Phe | O | -CH₂- |
| 98 | - | F | CH₃ | Phe | O | -(CH₂)₂ |
| 99 | - | F | CH₃ | Phe | O | -(CH₂)₃- |
| 100 | - | F | CH₃ | Phe | O | -(CH₂)₄- |

Verbindungen der folgenden Formel (Nr. 101-148):

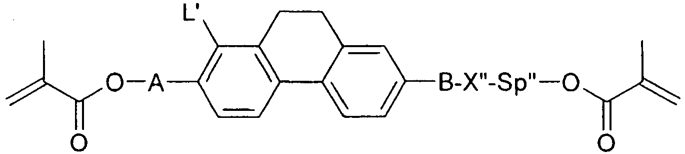

| **Nr.** | **A** | **L'** | **B** | **X"** | **Sp"** |
|---|---|---|---|---|---|
| 101 | - | H | - | - | -CH₂- |
| 102 | - | H | - | - | -(CH₂)₂ |
| 103 | - | H | - | - | -(CH₂)₃- |
| 104 | - | H | - | - | -(CH₂)₄- |
| 105 | - | H | - | O | -CH₂- |
| 106 | - | H | - | O | -(CH-₂)₂ |
| 107 | - | H | - | O | -(CH₂)₃- |
| 108 | - | H | - | O | -(CH₂)₄- |
| 109 | Phe | H | - | - | -CH₂- |
| 110 | Phe | H | - | - | -(CH₂)₂ |
| 111 | Phe | H | - | - | -(CH₂)₃- |
| 112 | Phe | H | - | - | -(CH₂)₄- |
| 113 | Phe | H | - | O | -CH₂- |
| 114 | Phe | H | - | O | -(CH₂)₂ |
| 115 | Phe | H | - | O | -(CH₂)₃- |
| 116 | Phe | H | - | O | -(CH₂)₄- |
| 117 | - | H | Phe | - | -CH₂- |
| 118 | - | H | Phe | - | -(CH₂)₂ |
| 119 | - | H | Phe | - | -(CH₂)₃- |
| 120 | - | H | Phe | - | -(CH₂)₄- |
| 121 | - | H | Phe | O | -CH₂- |
| 122 | - | H | Phe | O | -(CH₂)₂ |
| 123 | - | H | Phe | O | -(CH₂)₃- |
| 124 | - | H | Phe | O | -(CH₂)₄- |
| 125 | - | F | - | - | -CH₂- |
| 126 | - | F | - | - | -(CH₂)₂ |
| 127 | - | F | - | - | -(CH₂)₃- |
| 128 | - | F | - | - | -(CH₂)₄- |
| 129 | - | F | - | O | -CH₂- |
| 130 | - | F | - | O | -(CH₂)₂ |
| 131 | - | F | - | O | -(CH₂)₃- |
| 132 | - | F | - | O | -(CH₂)₄- |
| 133 | Phe | F | - | - | -CH₂- |
| 134 | Phe | F | - | - | -(CH₂)₂ |
| 135 | Phe | F | - | - | -(CH₂)₃- |
| 136 | Phe | F | - | - | -(CH₂)₄- |
| 137 | Phe | F | - | O | -CH₂- |
| 138 | Phe | F | - | O | -(CH₂)₂ |
| 139 | Phe | F | - | O | -(CH₂)₃- |
| 140 | Phe | F | - | O | -(CH₂)₄- |
| 141 | - | F | Phe | - | -CH₂- |
| 142 | - | F | Phe | - | -(CH₂)₂ |
| 143 | - | F | Phe | - | -(CH₂)₃- |
| 144 | - | F | Phe | - | -(CH₂)₄- |
| 145 | - | F | Phe | O | -CH₂- |
| 146 | - | F | Phe | O | -(CH₂)₂ |
| 147 | - | F | Phe | O | -(CH₂)₃- |
| 148 | - | F | Phe | O | -(CH₂)₄- |

Verbindungen der folgenden Formel (Nr. 149-196):

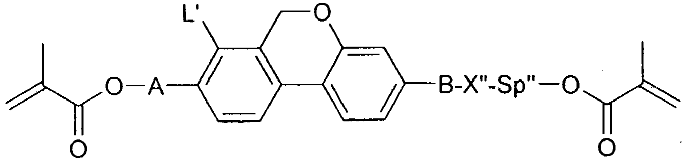

| **Nr.** | **A** | **L'** | **B** | **X"** | **Sp"** |
|---|---|---|---|---|---|
| 149 | - | H | - | - | -CH₂- |
| 150 | - | H | - | - | -(CH₂)₂ |
| 151 | - | H | - | - | -(CH₂)₃- |
| 152 | - | H | - | - | -(CH₂)₄- |
| 153 | - | H | - | O | -CH₂- |
| 154 | - | H | - | O | -(CH₂)₂ |
| 155 | - | H | - | O | -(CH₂)₃- |
| 156 | - | H | - | O | -(CH₂)₄- |
| 157 | Phe | H | - | - | -CH₂- |
| 158 | Phe | H | - | - | -(CH₂)₂ |
| 159 | Phe | H | - | - | -(CH₂)₃- |
| 160 | Phe | H | - | - | -(CH₂)₄- |
| 161 | Phe | H | - | O | -CH₂- |
| 162 | Phe | H | - | O | -(CH₂)₂ |
| 163 | Phe | H | - | O | -(CH₂)₃- |
| 164 | Phe | H | - | O | -(CH₂)₄- |
| 165 | - | H | Phe | - | -CH₂- |
| 166 | - | H | Phe | - | -(CH₂)₂ |
| 167 | - | H | Phe | - | -(CH₂)₃- |
| 168 | - | H | Phe | - | -(CH₂)₄- |
| 169 | - | H | Phe | O | -CH₂- |
| 170 | - | H | Phe | O | -(CH₂)₂ |
| 171 | - | H | Phe | O | -(CH₂)₃- |
| 172 | - | H | Phe | O | -(CH₂)₄- |
| 173 | - | F | - | - | -CH₂- |
| 174 | - | F | - | - | -(CH₂)₂ |
| 175 | - | F | - | - | -(CH₂)₃- |
| 176 | - | F | - | - | -(CH₂)₄- |
| 177 | - | F | - | O | -CH₂- |
| 178 | - | F | - | O | -(CH₂)₂ |
| 179 | - | F | - | O | -(CH₂)₃- |
| 180 | - | F | - | O | -(CH₂)₄- |
| 181 | Phe | F | - | - | -CH₂- |
| 182 | Phe | F | - | - | -(CH₂)₂ |
| 183 | Phe | F | - | - | -(CH₂)₃- |
| 184 | Phe | F | - | - | -(CH₂)₄- |
| 185 | Phe | F | - | O | -CH₂- |
| 186 | Phe | F | - | O | -(CH₂)₂ |
| 187 | Phe | F | - | O | -(CH₂)₃- |
| 188 | Phe | F | - | O | -(CH₂)₄- |
| 189 | - | F | Phe | - | -CH₂- |
| 190 | - | F | Phe | - | -(CH₂)₂ |
| 191 | - | F | Phe | - | -(CH₂)₃- |
| 192 | - | F | Phe | - | -(CH₂)₄- |
| 193 | - | F | Phe | O | -CH₂- |
| 194 | - | F | Phe | O | -(CH₂)₂ |
| 195 | - | F | Phe | O | -(CH₂)₃- |
| 196 | - | F | Phe | O | -(CH₂)₄- |

### Beispiel 197:2-Methyl-acrylsäure-9,9-dimethyl-7-[4-(2-methyl-acryloyloxy)-butyl]-9H-fluoren-2-ylester

### 197.1 2-Methyl-acrylsäure-4-[9,9-dimethyl-7-(2-methyl-acryloyloxy)-9H-fluoren-2-yl]-but-3-inylester

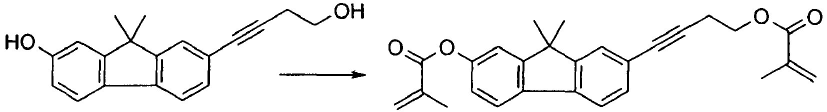

In Analogie zu Beispiel 1 erhält man aus 7-(4-Hydroxy-but-1-inyl)-9,9-dimethyl-9H-fluoren-2-ol (Herstellung siehe Beispiel 32.2) den 2-Methyl-acrylsäure-4-[9,9-dimethyl-7-(2-methyl-acryloyloxy)-9H-fluoren-2-yl]-but-3-inylester als farbloses Öl. Glasübergang Tg -17 I.

### Anwendungsbeispiel A

Die nematische FK-Mischung **N1** wird wie folgt formuliert

| | | | |
|---|---|---|---|
| CCH-501 | 9,00 % | Kp. | + 70,0 |
| CCH-35 | 14,00 % | Δn | 0,0825 |
| PCH-53 | 8,00 % | Δε | - 3,5 |
| CY-3-04 | 14,00 % | ε_{\|\|} | 3,5 |
| CY-5-04 | 13,00 % | K₃/K₁ | 1,00 |
| CCY-3-02 | 8,00 % | γ₁ | 141 |
| CCY-5-02 | 8,00 % | V₀ | 2,06 |
| CCY-2-1 | 9,00 % | | |
| CCY-3-1 | 9,00 % | | |
| CPY-2-02 | 8,00 % | | |

Zur FK-Mischung N1 werden 0.3% einer polymerisierbaren monomeren Verbindung einer der unten aufgeführten Formeln zugesetzt und die dadurch entstanden Mischung in VA-e/o-Testzellen gefüllt (antiparallel gerieben, Orientierungsschicht VA-Polyimid, Schichtdicke d ≈ 4µm). Unter Anlegen einer Spannung von 24 V (Wechselstrom) werden die Zellen in der angegebenen Zeit mit UV-Licht der Intensität 50 mW/cm² bestrahlt, dadurch erfolgt Polymerisation der monomeren Verbindung. Vor und nach der UV-Bestrahlung wird per Drehkristall-Experiment (Autronic-Melchers TBA-105) der Tiltwinkel bestimmt.

Zur Bestimmung der Polymerisationsgeschwindigkeit wird der Restgehalt an unpolymerisiertem RM (in Gew.%) in den Testzellen nach verschiedenen Belichtungszeiten mit der HPLC-Methode gemessen. Dazu wird jede Mischung jeweils unter den angegebenen Bedingungen in der Testzelle polymerisiert. Danach wird die Mischung mit Methylethylketon aus der Testzelle gespült und vermessen.

Zu Vergleichszwecken werden die oben beschriebenen Versuche mit der aus dem Stand der Technik (z.B. US 7,169,449) bekannten strukturanalogen polymerisierbaren Verbindung M1 durchgeführt.

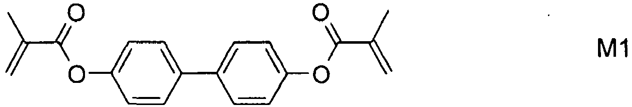

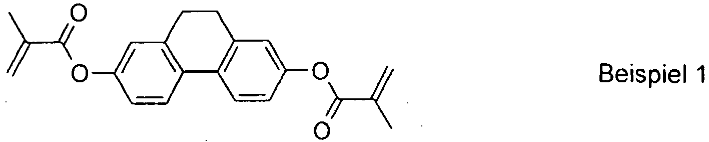

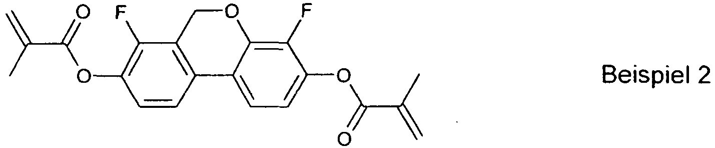

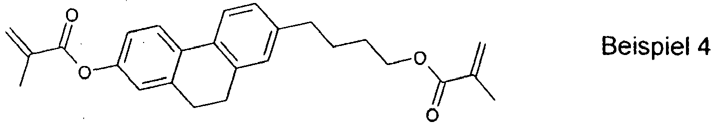

Die Tiltwinkelergebnisse sind in Tabelle 1 zusammengefasst.

**Tabelle 1**

| | M1 | Bsp.1 | Bsp.2 | Bsp.4 |
|---|---|---|---|---|
| Belichtungszeit / s | Tiltwinkel / ° | | | |
| 0 | 89,2 | 89,5 | 88,9 | 89,6 |
| 30 | 89,1 | 88,7 | 88,5 | 89,8 |
| 60 | 88,4 | 83,0 | 86,0 | 88,3 |
| 120 | 83,9 | 77,3 | 81,6 | 83,3 |
| 240 | 76,5 | 74,8 | 76,1 | 73,5 |
| 360 | - | 75,1 | - | 66,1 |

Wie aus Tabelle 1 ersichtlich ist, kann in PSA-Anzeigen mit den erfindungsgemäßen Monomeren aus Beispiel 1-4 schneller ein kleiner Tiltwinkel nach Polymerisation erreicht werden als in PSA-Anzeigen mit dem Monomer M1 aus dem Stand der Technik.

Die RM-Konzentrationen nach verschiedenen Belichtungszeiten sind in Tabelle 2 zusammengefasst.

**Tabelle 2**

| | M1 | Bsp.1 | Bsp.2 | Bsp.4 |
|---|---|---|---|---|
| Belichtungszeit / s | RM-Konzentration (Gew. %) | | | |
| 0 | 0,300 | 0,300 | 0,300 | 0,300 |
| 120 | 0,268 | 0,131 | 0,197 | 0,195 |
| 240 | 0,226 | 0,054 | 0,111 | 0,103 |
| 360 | 0,176 | 0,010 | 0,062 | 0,069 |

Wie aus Tabelle 2 ersichtlich ist, wird in PSA-Anzeigen mit den erfindungsgemäßen Monomeren aus Beispiel 1-4 eine deutlich schnellere Polymerisationsgeschwindigkeit erzielt als in PSA-Anzeigen mit dem Monomer M1 aus dem Stand der Technik.

### Anwendungsbeispiel B

Mit der FK-Mischung N1 und einer polymerisierbaren monomeren Verbindung aus Beispiel 32 bzw. Beispiel 197 werden, wie in Beispiel A beschrieben, Testzellen hergestellt und der Tiltwinkel nach verschiedenen Belichtungszeiten sowie der der Restgehalt an unpolymerisiertem RM gemessen.

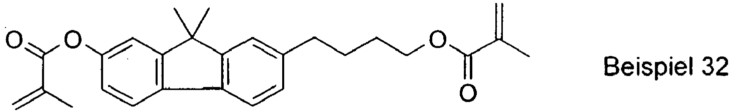

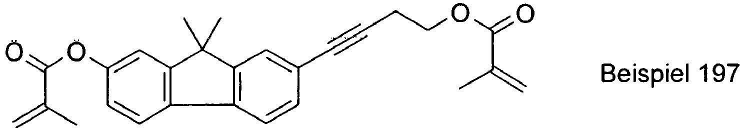

Die Ergebnisse sind in Tabelle 3 und 4 zusammengefasst.

**Tabelle 3**

| | Bsp.32 | Bsp.197 |
|---|---|---|
| Belichtungszeit / s | Tiltwinkel / ° | |
| 0 | 89,7 | 89,2 |
| 30 | 89,1 | 88,7 |
| 60 | 88,6 | 83,2 |
| 120 | 73,8 | 60,5 |
| 240 | 37,6 | 44,6 |
| 360 | 28,8 | 39,2 |

Wie aus Tabelle 3 ersichtlich ist, kann in PSA-Anzeigen mit den erfindungsgemäßen Monomeren schnell ein sehr kleiner Tiltwinkel nach Polymerisation erreicht werden.

**Tabelle 4**

| | Bsp.32 | Bsp.197 |
|---|---|---|
| Belichtungszeit / s | RM-Konzentration (Gew. %) | |
| 0 | 0,300 | 0,300 |
| 120 | 0,110 | 0,013 |
| 240 | 0,000 | 0,000 |
| 360 | 0,000 | 0,000 |

Wie aus Tabelle 4 ersichtlich ist, wird in PSA-Anzeigen mit den erfindungsgemäßen Monomeren eine sehr schnelle und vollständige Polymerisation erzielt.

## Patentansprüche

1. Verwendung von Verbindungen der Formel I 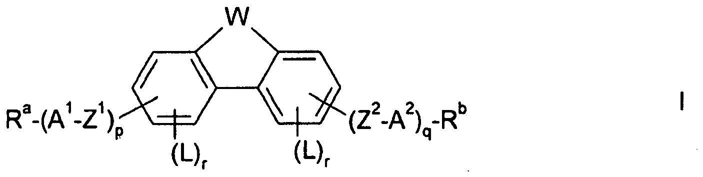 worin die einzelnen Reste folgende Bedeutung besitzen
W -C(R^{c}R^{d})-, -CH₂CH₂-, -CH₂-O-, -O-, -CO-, -CO-O-, -S- oder - N(R^{c})-,
R^{c}, R^{d} jeweils unabhängig voneinander P-Sp-, H, F, Cl, Br, I, -CN, - NO₂, -NCO, -NCS, -OCN, -SCN, SF₅ geradkettiges oder verzweigtes Alkyl mit 1 bis 25 C-Atomen, worin auch eine oder mehrere nicht benachbarte CH₂-Gruppen jeweils unabhängig voneinander durch Arylen, -C(R⁰)=C(R⁰⁰)-, - C≡C-, -N(R⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, Br, I, CN oder P-Sp- ersetzt sein können, oder Aryl oder Heteroaryl, vorzugsweise mit 2 bis 25 C-Atomen, welches auch zwei oder mehr anellierte Ringe enthalten kann und welches optional durch L ein- oder mehrfach substituiert ist,
R^{a}, R^{b} jeweils unabhängig voneinander eine der für R^{c} angegebenen Bedeutungen, wobei mindestens einer der Reste R^{a} und R^{b} eine Gruppe P-Sp- bedeutet oder enthält,
P bei jedem Auftreten gleich oder verschieden eine polymerisierbare Gruppe,
Sp bei jedem Auftreten gleich oder verschieden eine Abstandsgruppe oder eine Einfachbindung,
A¹ und A² jeweils unabhängig voneinander 1,4-Phenylen, Naphthalin-1,4-diyl oder Naphthalin-2,6-diyl, wobei in diesen Gruppen auch eine oder mehrere CH-Gruppen durch N ersetzt sein können, Cyclohexan-1,4-diyl, worin auch eine oder mehrere nicht-benachbarte CH₂-Gruppen durch O und/oder S ersetzt sein können, 1,4-Cyclohexenylen, Bicyclo[1.1.1]pentan-1,3-diyl, Bicyclo[2.2.2]octan-1,4-diyl, Spiro[3.3]heptan-2,6-diyl, Piperidin-1,4-diyl, Decahydronaphthalin-2,6-diyl, 1,2,3,4-Tetrahydronaphthalin-2,6-diyl, Indan-2,5-diyl, Octahydro-4,7-methano-indan-2,5-diyl, Phenanthren-2,7-diyl oder Anthracen-2,7-diyl, wobei alle diese Gruppen unsubstituiert oder durch L ein- oder mehrfach substituiert sein können,
L P, P-Sp-, OH, CH₂OH, F, Cl, Br, I, -CN, -NO₂, -NCO, -NCS, -OCN, -SCN, -C(=O)N(R^{x})₂, -C(=O)Y¹, -C(=O)R^{x}, -N(R^{x})₂, optional substituiertes Silyl, optional substituiertes Aryl mit 6 bis 20 C Atomen, geradkettiges oder verzweigtes Alkyl oder Alkoxy 1 bis 25 C-Atomen, oder geradkettiges oder verzweigtes Alkenyl, Alkinyl, Alkylcarbonyl, Alkoxycarbonyl, Alkylcarbonyloxy oder Alkoxycarbonyloxy mit 2 bis 25 C-Atomen, worin in allen diesen Gruppen auch ein oder mehrere H-Atome durch F, Cl, P oder P-Sp- ersetzt sein können,
Y¹ Halogen,
R^{x} P, P-Sp-, H, Halogen, geradkettiges, verzweigtes oder cyclisches Alkyl mit 1 bis 25 C-Atomen, worin auch eine oder mehrere nicht benachbarte CH₂-Gruppen durch -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl oder P-Sp- ersetzt sein können,
Z¹, Z² jeweils unabhängig voneinander-O-, -S-, -CO-, -CO-O-, -O-CO-, -O-CO-O-, -OCH₂-, -CH₂O-, -SCH₂-, -CH₂S-, -CF₂O-, -OCF₂-, -CF₂S-, -SCF₂-, -(CH₂)ₙ-, -CF₂CH₂-, -CH₂CF₂-, -(CF₂)ₙ-, -CH=CH-, -CF=CF-, -CH=CF-, -CF=CH-, -C≡C-, -CH=CH-CO-O-, -O-CO-CH=CH-, -CH₂-CH₂-CO-O-, -O-CO-CH₂-CH₂-, -C(R⁰R⁰⁰)-, -C(R^{y}R^{z})-, oder eine Einfachbindung,
R⁰, R⁰⁰ jeweils unabhängig voneinander und bei jedem Auftreten gleich oder verschieden H oder Alkyl mit 1 bis 12 C-Atomen,
R^{y}, R^{z} jeweils unabhängig voneinander H, F, CH₃ oder CF₃,
n bei jedem Auftreten gleich oder verschieden 1, 2, 3 oder 4,
p, q jeweils unabhängig voneinander 0, 1, 2 oder 3,
r bei jedem Auftreten gleich oder verschieden 0, 1, 2 oder 3,
in FK-Anzeigen des PS-(polymer stabilized) oder PSA-(polymer sustained alignment) Typs.

2. Verwendung nach Anspruch 1, **dadurch gekennzeichnet, dass** in Formel I R^{a} und R^{b} gleiche oder verschiedene Reste P-Spbedeuten.

3. Verwendung nach Anspruch 1 oder 2, **dadurch gekennzeichnet, dass** die Verbindungen der Formel I ausgewählt sind aus Formel IA 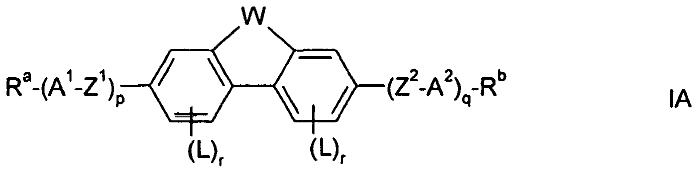 worin W, R^{a,b}, A^{1,2}, Z^{1,2}, L, p, q und r die in Anspruch 1 oder 2 angegebene Bedeutung haben.

4. Verwendung nach einem oder mehreren der Ansprüche 1 bis 3, **dadurch gekennzeichnet, dass** die Verbindungen der Formel I ausgewählt sind aus der Gruppe bestehend aus folgenden Unterformeln 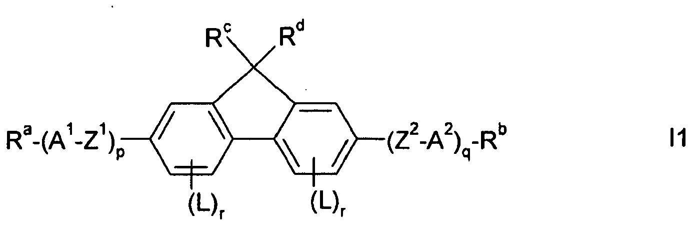 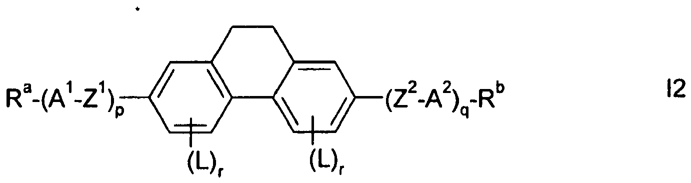 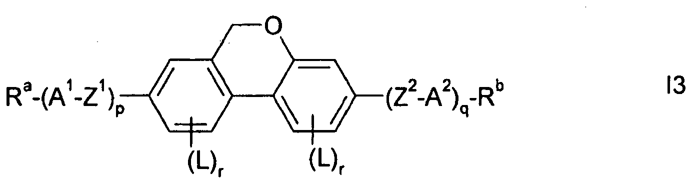 worin die einzelnen Reste die in Anspruch 1 angegebenen Bedeutungen besitzen.

5. Verwendung nach einem oder mehreren der Ansprüche 1 bis 4, **dadurch gekennzeichnet, dass** die Verbindungen der Formel I ausgewählt sind aus der Gruppe bestehend aus folgenden Unterformeln 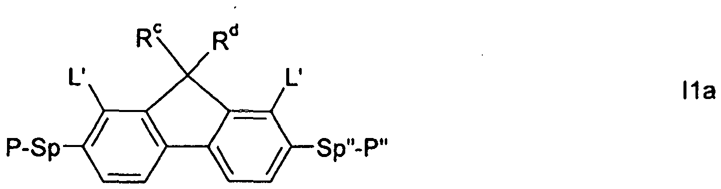 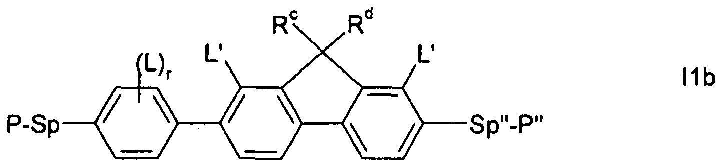 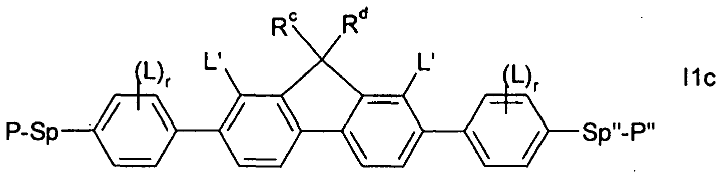 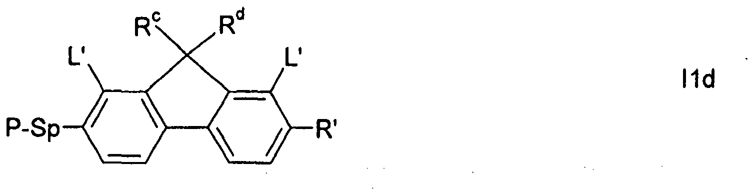 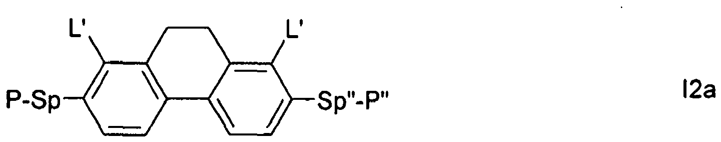 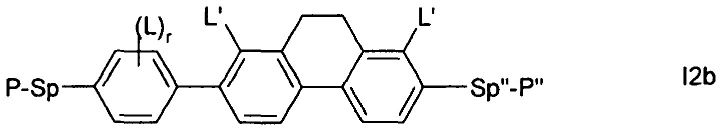 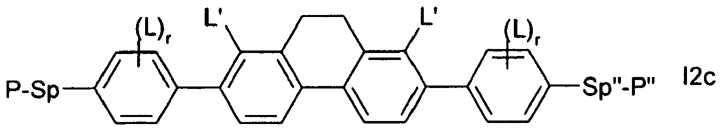 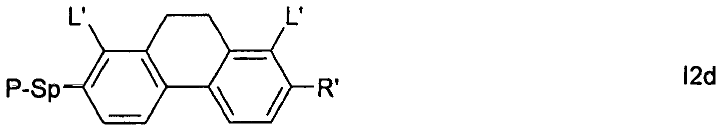 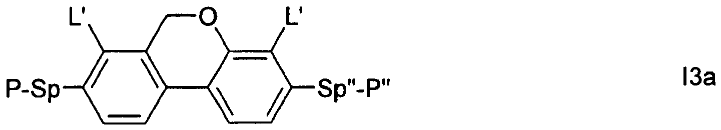 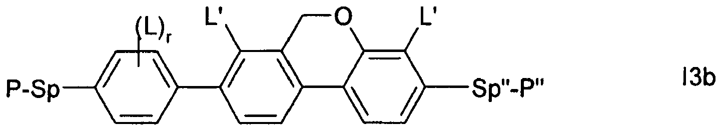 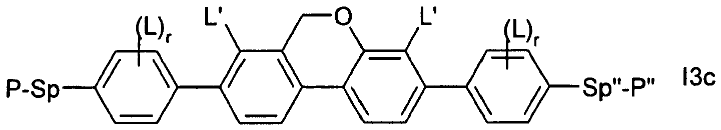 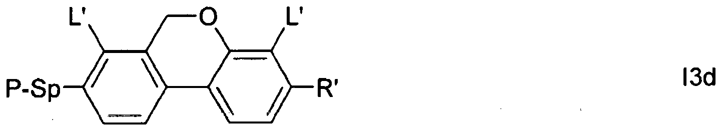 worin L'H oder F bedeutet, P und Sp eine der in Anspruch 1 angegebenen Bedeutungen besitzen, P" eine der in Anspruch 1 für P angegebenen Bedeutungen besitzt, Sp" eine der in Anspruch 1 für Sp angegebenen Bedeutungen besitzt, und R' eine der in Anspruch 1 für R^{a} angegebenen Bedeutungen besitzt, wobei R' von H verschieden ist und keine Gruppe P-Sp- bedeutet oder enthält.

6. Verwendung von Verbindungen nach einem oder mehreren der Ansprüche 1 bis 5 in FK-Anzeigen des PS- oder PSA-Typs, enthaltend eine FK-Zelle mit zwei Substraten, wobei mindestens ein Substrat lichtdurchlässig ist und mindestens ein Substrat eine oder zwei Elektroden aufweist, sowie einer zwischen den Substraten befindlichen Schicht eines FK-Mediums enthaltend eine polymerisierte Komponente und eine niedermolekulare Komponente, wobei die polymerisierte Komponente erhältlich ist durch Polymerisation einer oder mehrerer polymerisierbarer Verbindungen zwischen den Substraten der FK-Zelle im FK-Medium unter Anlegen einer elektrischen Spannung an die Elektroden, wobei mindestens eine der polymerisierbaren Verbindungen aus Formel I, wie in einem oder mehreren der Ansprüche 1 bis 5 definiert, ausgewählt ist.

7. Verwendung nach einem oder mehreren der Ansprüche 1 bis 6, **dadurch gekennzeichnet, dass** die FK-Anzeige ein FK-Medium enthält, welches eine oder mehrere Verbindungen der Formel I sowie eine oder mehrere Verbindungen der Formel CY und/oder PY enthält: 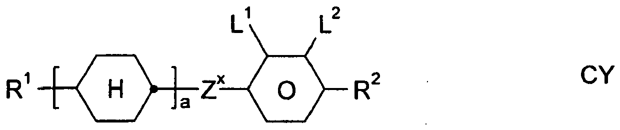 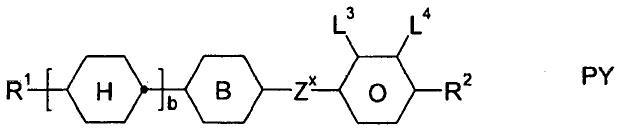 worin die einzelnen Reste folgende Bedeutung besitzen
a 1 oder 2,
b 0 oder 1, 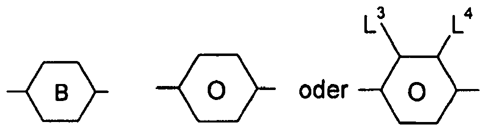
R¹ und R² jeweils unabhängig voneinander Alkyl mit 1 bis 12 C-Atomen, wobei auch eine oder zwei nicht benachbarte CH₂-Gruppen durch -O-, -CH=CH-, -CO-, -O-CO- oder -CO-O- so ersetzt sein können, dass O-Atome nicht direkt miteinander verknüpft sind,
Z^{x} -CH=CH-, -CH₂O-, -OCH₂-, -CF₂O-, -OCF₂-, -O-, -CH₂-, -CH₂CH₂-, oder eine Einfachbindung, vorzugsweise eine Einfachbindung,
L¹⁻⁴ jeweils unabhängig voneinander F, Cl, OCF₃, CF₃, CH₃, CH₂F, CHF₂.

8. Verwendung nach einem oder mehreren der Ansprüche 1 bis 7, **dadurch gekennzeichnet, dass** das FK-Medium eine oder mehrere Verbindungen der folgenden Formel enthält: 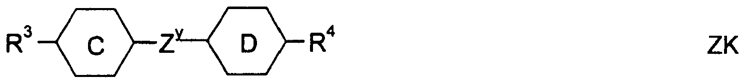 worin die einzelnen Reste folgende Bedeutung besitzen 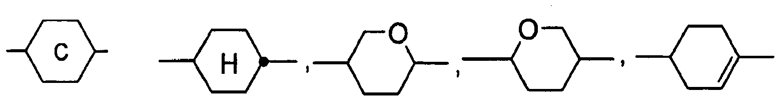 oder 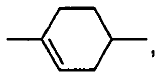 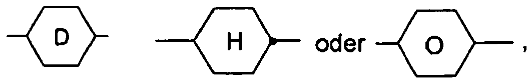
R³ und R⁴ jeweils unabhängig voneinander Alkyl mit 1 bis 12 C-Atomen, worin auch eine oder zwei nicht benachbarte CH₂-Gruppen durch -O-, -CH=CH-, -CO-, -O-CO- oder -CO-O- so ersetzt sein können, dass O-Atome nicht direkt miteinander verknüpft sind,
Z^{y} -CH₂CH₂-, -CH=CH-, -CF₂O-, -OCF₂-, -CH₂O-, -OCH₂-, -COO-, -OCO-, -C₂F₄-, -CF=CF- oder eine Einfachbindung.

9. FK-Anzeige des PS- oder PSA-Typs wie in einem oder mehreren der Ansprüche 1 bis 8 definiert.

10. FK-Anzeige nach Anspruch 9, **dadurch gekennzeichnet, dass** sie eine PSA-VA-, PSA-OCB-, PS-IPS-, PS-FFS- oder PS-TN-Anzeige ist.

11. Verfahren zu Herstellung einer FK-Anzeige des PS- oder PSA-Typs, indem man ein FK-Medium wie in einem oder mehreren der Ansprüche 6 bis 8 definiert in eine FK-Zelle mit zwei Substraten, wobei mindestens ein Substrat lichtdurchlässig ist und mindestens ein Substrat eine oder zwei Elektroden aufweist, füllt, und die polymerisierbaren Verbindungen unter Anlegen einer elektrischen Spannung an die Elektroden polymerisiert.

12. Verbindungen der Formel I1 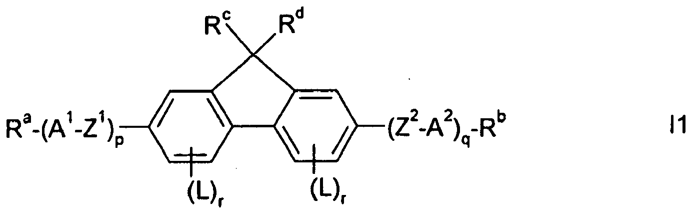 worin R^{c,d}, A^{1,2}, Z^{1,2}, L, p, q und r die in Anspruch 1 oder 2 angegebene Bedeutung besitzen, und R^{a} und R^{b} P-Sp- bedeuten, wobei in einem der Reste R^{a} und R^{b} Sp eine Einfachbindung bedeutet und in dem anderen der Reste R^{a} und R^{b} Sp eine Gruppe der Formel Sp'-X' bedeutet, so dass dieser Rest P-Sp- der Formel P-Sp'-X'- entspricht,
Sp' Alkylen mit 1 bis 20 C-Atomen bedeutet, welches optional durch F, Cl, Br, I oder CN ein- oder mehrfach substituiert ist, und worin auch eine oder mehrere nicht benachbarte CH₂-Gruppen jeweils unabhängig voneinander so durch -O-, -S-, -NH-, -N(R⁰)-, -Si(R⁰⁰R⁰⁰⁰)-, -CO-, -CO-O-, -O-CO-, -O-CO-O-, -S-CO-, -CO-S-, -N(R⁰⁰)-CO-O-, -O-CO-N(R⁰⁰)-, -N(R⁰⁰)-CO-N(R⁰⁰)-, -CH=CH- oder -C≡C- ersetzt sein können, dass O- und/oder S-Atome nicht direkt miteinander verknüpft sind,
X¹ -O-, -S-, -CO-, -CO-O-, -O-CO-, -O-CO-O-, -CO-N(R⁰⁰)-, -N(R⁰⁰)-CO-, -N(R⁰⁰)-CO-N(R⁰⁰)-, -OCHF₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, - CY²=CY³-, -C≡C-, -CH=CH-CO-O-, -O-CO-CH=CH- oder eine Einfachbindung bedeutet,
R⁰⁰, R⁰⁰⁰ jeweils unabhängig voneinander H oder Alkyl mit 1 bis 12 C-Atomen bedeuten,
Y², Y³ jeweils unabhängig voneinander H, F, Cl oder CN bedeuten,
P bei jedem Auftreten gleich oder verschieden ausgewählt ist aus CH₂=CW¹-CO-O-, CH₂=CW¹-CO-, 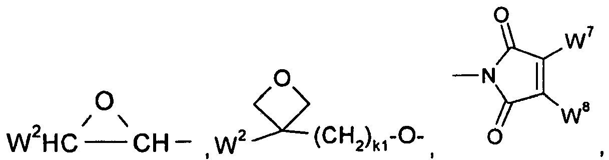 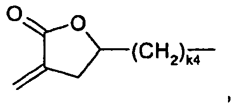
CH₂=CW²-O-, CW¹=CH-CO-(O)ₖ₃-, CW¹=CH-CO-NH-, CH₂=CW¹-CO-NH-, (CH₂=CH)₂CH-OCO-, (CH₁=CH-CH₂)₂CH-OCO-, (CH₂=CH)₂CH-O-, (CH₂=CH-CH₂)₂N-, (CH₂=CH-CH₂)₂N-CO-, CH₂=CW¹-CO-NH-, CH₂=CH-(COO)ₖ₁-Phe-(O)ₖ₂-, CH₂=CH-(CO)ₖ₁-Phe-(O)ₖ₂- und W⁴W⁵W⁶Si-, worin W¹ H, F, Cl, CN, CF₃, Phenyl oder Alkyl mit 1 bis 5 C-Atomen bedeutet, W² und W³ jeweils unabhängig voneinander H oder Alkyl mit 1 bis 5 C-Atomen bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet, k₁, k₂ und k₃ jeweils unabhängig voneinander 0 oder 1 bedeuten und k₄ eine ganze Zahl von 1 bis 10 bedeutet.

13. Verbindungen der Formel I2 oder I3 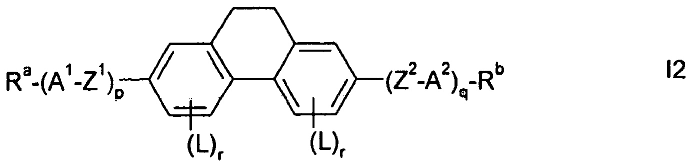 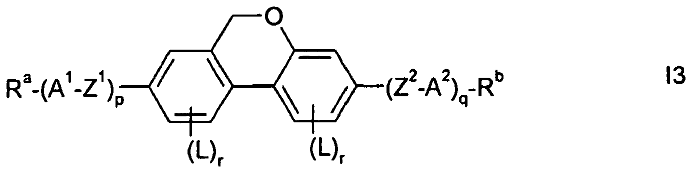 worin die einzelnen Reste die in Anspruch 1 angegebenen Bedeutungen besitzen.

14. Verbindungen nach Anspruch 12 oder 13, ausgewählt aus der Gruppe bestehend aus den folgenden Unterformeln 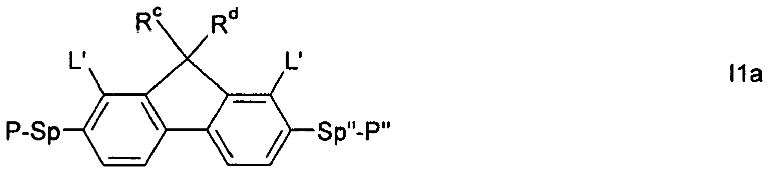 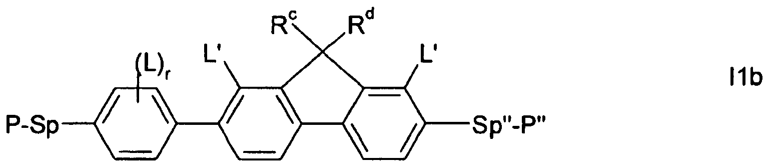 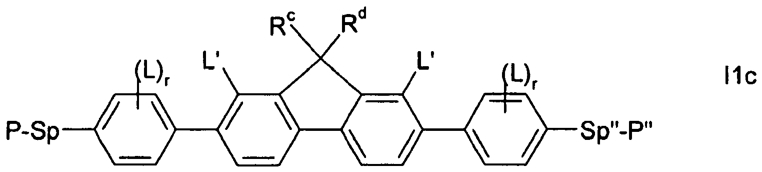 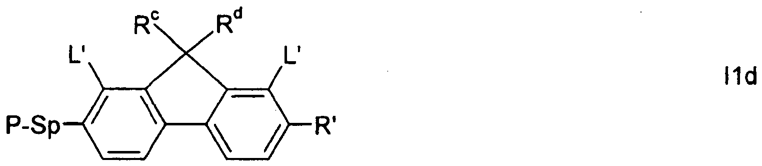 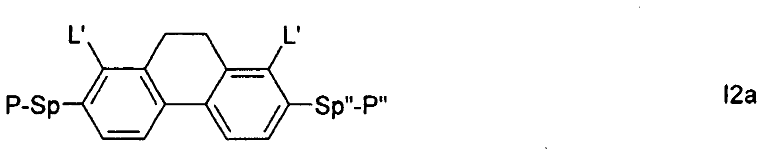 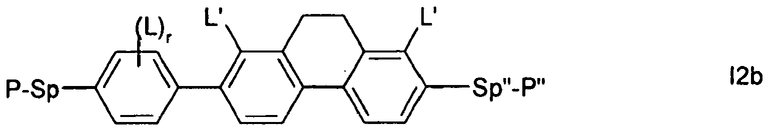 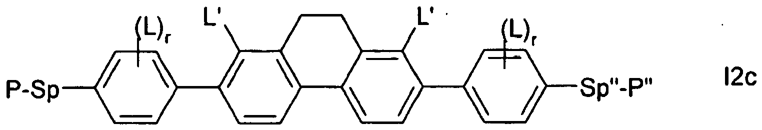 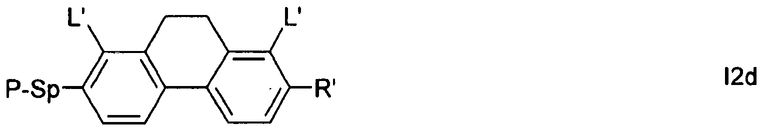 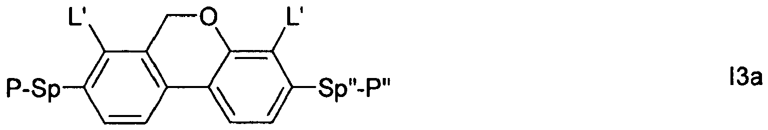 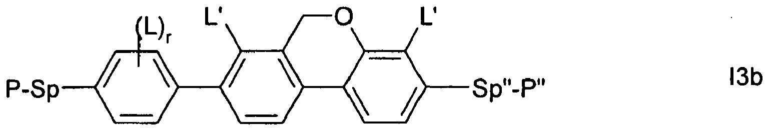 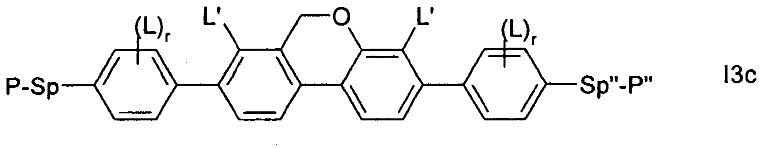 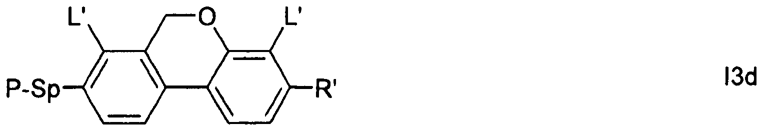 worin L' H oder F bedeutet, P und Sp eine der in Anspruch 12 angegebenen Bedeutungen besitzen, P" eine der für P angegebenen Bedeutungen besitzt, Sp" eine der für Sp angegebenen Bedeutungen besitzt, und R' eine der für R^{a} in Anspruch 12 angegebenen Bedeutungen besitzt, wobei R' von H verschieden ist und keine Gruppe P-Sp- bedeutet oder enthält.

15. Verbindungen nach einem oder mehreren der Ansprüche 12 bis 14, dadurch gekennnzeichnet, dass P und P" eine Acrylat-, Fluoracrylat- oder Methacrylatgruppe bedeuten.

16. Verbindungen nach einem oder mehreren der Ansprüche 12 bis 15, dadurch gekennnzeichnet, dass Sp und Sp" ausgewählt sind aus, - (CH₂)ₚ₁-, -O-(CH₂)ₚ₁-, -(CH₂)ₚ₁-O-, -O-CO-(CH₂)ₚ₁-, -(CH₂)ₚ₁-O-CO-, - O-CO-O-(CH₂)ₚ₁-, -(CH₂)ₚ₁-O-CO-O-, -(CH₂)ᵣ₁-C≡C- oder -C=C-(CH₂)ᵣ₁- oder einer Einfachbindung, worin p1 eine ganze Zahl von 1 bis 12, vorzugsweiswe von 1 bis 6, und r1 eine ganze Zahl von 1 bis 8, vorzugsweise 1, 2 oder 3 bedeuten, wobei diese Gruppen so mit P oder P" verknüpft sind, dass O-Atome nicht direkt miteinander verknüpft sind.

17. FK-Medium enthaltend eine oder mehrere Verbindungen der Formel I1, I2 oder I3 oder deren Unterformeln nach einem oder mehreren der Ansprüche 12 bis 16.

18. FK-Medium nach Anspruch 17, enthaltend
- eine polymerisierbare Komponente A) enthaltend eine oder mehrere polymerisierbare Verbindungen, sowie
- eine flüssigkristalline Komponente B) enthaltend eine oder mehrere niedermolekulare Verbindungen,
**dadurch gekennzeichnet, dass** Komponente A) eine oder mehrere polymerisierbare Verbindungen der Formel I nach einem oder mehreren der Ansprüche 12 bis 16 enthält.

19. FK-Medium nach Anspruch 18, worin Komponente B) eine oder mehrere Verbindungen ausgewählt aus den Formeln CY, PY und ZK nach Anspruch 7 oder 8 enthält.

20. Verbindungen ausgewählt aus der Gruppe bestehend aus folgenden Unterformeln 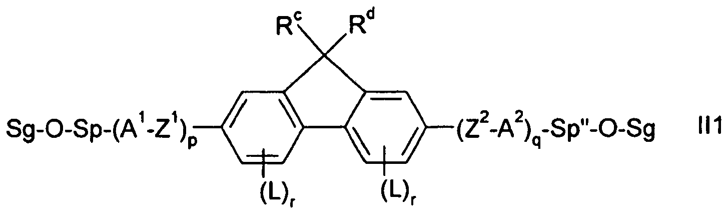 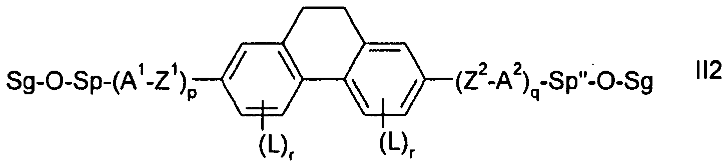 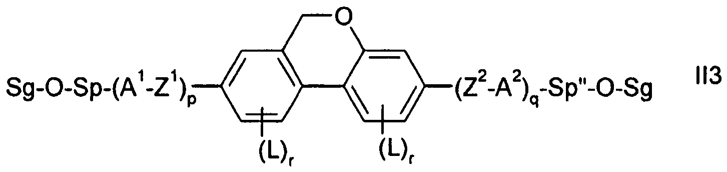 worin R^{c,d}, A^{1,2}, Z^{1,2}, L, p, q, r, Sp und Sp" die in Anspruch 1, 4 und 14 angegebene Bedeutung besitzen, wobei in Formel II1 Sp eine Einfachbindung bedeutet und Sp" von einer Einfachbindung verschieden ist, und Sg ein H-Atom oder eine Schutzgruppe bedeutet.

21. Verbindungen der Formel III 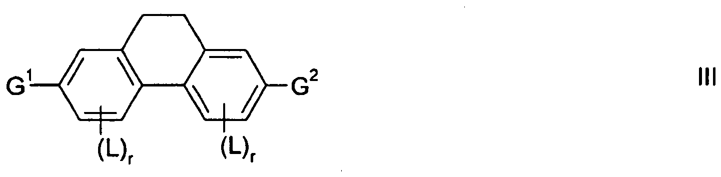 worin L und r die in Anspruch 1 angegebene Bedeutung besitzen und eine der Gruppen G¹ und G² I und die andere Br oder OH bedeutet.

22. Verfahren zur Herstellung einer Verbindung der Formel I oder deren Unterformeln nach einem der Ansprüche 12 bis 16, indem man eine Verbindung nach Anspruch 20 mit entsprechenden Säuren, Säurederivaten, oder halogenierten Verbindungen enthaltend eine Gruppe P, in Gegenwart eines wasserentziehenden Reagens verestert oder verethert.

## Claims

1. Use of compounds of the formula I 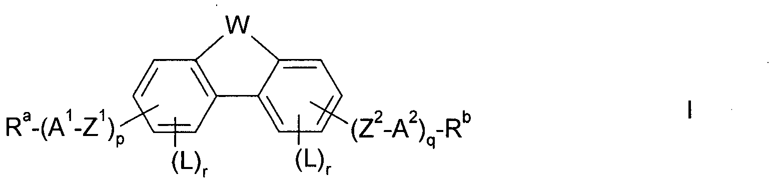 in which the individual radicals have the following meanings:
W denotes -C(R^{c}R^{d})-, -CH₂CH₂-, -CH₂-O-, -O-, -CO-, -CO-O-, -S- or -N(R⁰)-,
R^{c}, R^{d} each, independently of one another, denote P-Sp-, H, F, Cl, Br, I, -CN, -NO₂, -NCO, -NCS, -OCN, -SCN, SF₅, straight-chain or branched alkyl having 1 to 25 C atoms, in which, in addition, one or more non-adjacent CH₂ groups may each be replaced, independently of one another, by arylene, -C(R⁰)=C(R⁰⁰)-, -C≡C-, -N(R⁰)-, -O-, -S-, -CO-, -CO-O-, -O-CO-, -O-CO-O- in such a way that O and/or S atoms are not linked directly to one another, and in which, in addition, one or more H atoms may be replaced by F, Cl, Br, I, CN or P-Sp-, or aryl or heteroaryl, preferably having 2 to 25 C atoms, which may also contain two or more fused rings and which is optionally mono- or poly-substituted by L,
R^{a}, R^{b} each, independently of one another, have one of the meanings indicated for R^{c}, where at least one of the radicals R^{a} and R^{b} denotes or contains a group P-Sp-,
P on each occurrence, identically or differently, denotes a polymerisable group,
Sp on each occurrence, identically or differently, denotes a spacer group or a single bond,
A¹ and A² each, independently of one another, denote 1,4-phenylene, naphthalene-1,4-diyl or naphthalene-2,6-diyl, where, in addition, one or more CH groups in these groups may be replaced by N, cyclohexane-1,4-diyl, in which, in addition, one or more non-adjacent CH₂ groups may be replaced by O and/or S, 1,4-cyclohexenylene, bicyclo-[1.1.1]pentane-1,3-diyl, bicyclo[2.2.2]octane-1,4-diyl, spiro-[3.3]heptane-2,6-diyl, piperidine-1,4-diyl, decahydronaphthalene-2,6-diyl, 1,2,3,4-tetrahydronaphthalene-2,6-diyl, indane-2,5-diyl, octahydro-4,7-methanoindane-2,5-diyl, phenanthrene-2,7-diyl or anthracene-2,7-diyl, where all these groups may be unsubstituted or mono- or polysubstituted by L,
L denotes P, P-Sp-, OH, CH₂OH, F, Cl, Br, I, -CN, -NO₂, -NCO, -NCS, -OCN, -SCN, -C(=O)N(R^{x})₂, -C(=O)Y¹, -C(=O)R^{x}, -N(R^{x})₂, optionally substituted silyl, optionally substituted aryl having 6 to 20 C atoms, straight-chain or branched alkyl or alkoxy having 1 to 25 C atoms, or straight-chain or branched alkenyl, alkynyl, alkylcarbonyl, alkoxycarbonyl, alkylcarbonyloxy or alkoxycarbonyloxy having 2 to 25 C atoms, in which, in addition, one or more H atoms in all these groups may be replaced by F, Cl, P or P-Sp-,
Y¹ denotes halogen,
R^{x} denotes P, P-Sp-, H, halogen, straight-chain, branched or cyclic alkyl having 1 to 25 C atoms, in which, in addition, one or more non-adjacent CH₂ groups may be replaced by -O-, -S-, -CO-, -CO-O-, -O-CO-, -O-CO-O- in such a way that O and/or S atoms are not linked directly to one another, and in which, in addition, one or more H atoms may be replaced by F, Cl or P-Sp-,
Z¹, Z² each, independently of one another, denote -O-, -S-, -CO-, -CO-O-, -O-CO-, -O-CO-O-, -OCH₂-, -CH₂O-, -SCH₂-, -CH₂S-, -CF₂O-, -OCF₂-, -CF₂S-, -SCF₂-, -(CH₂)ₙ-, -CF₂CH₂-, -CH₂CF₂-, -(CF₂)ₙ-, -CH=CH-, -CF=CF-, -CH=CF-, -CF=CH-, -C≡C-, -CH=CH-CO-O-, -O-CO-CH=CH-, -CH₂-CH₂-CO-O-, -O-CO-CH₂-CH₂-, -C(R⁰R⁰⁰)-, -C(R^{y}R^{z})- or a single bond,
R⁰, R⁰⁰ each, independently of one another and identically or differ-ently on each occurrence, denote H or alkyl having 1 to 12 C atoms,
R^{y}, R^{z} each, independently of one another, denote H, F, CH₃ or CF₃,
n on each occurrence, identically or differently, denotes 1, 2, 3 or 4,
p, q each, independently of one another, denote 0, 1, 2 or 3,
r on each occurrence, identically or differently, denotes 0, 1, 2 or 3,
in LC displays of the PS (polymer stabilised) or PSA (polymer sustained alignment) type.

2. Use according to Claim 1, **characterised in that** R^{a} and R^{b} in formula I denote identical or different radicals P-Sp-.

3. Use according to Claim 1 or 2, **characterised in that** the compounds of the formula I are selected from formula IA 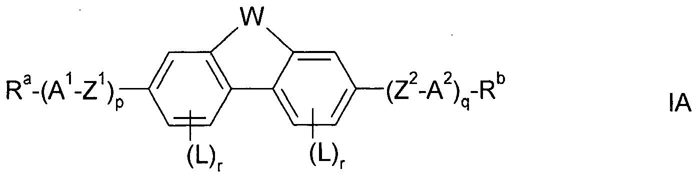 in which W, R^{a,b}, A^{1,2}, Z^{1,2}, L, p, q and r have the meaning indicated in Claim 1 or 2.

4. Use according to one or more of Claims 1 to 3, **characterised in that** the compounds of the formula I are selected from the group consisting of the following sub-formulae: 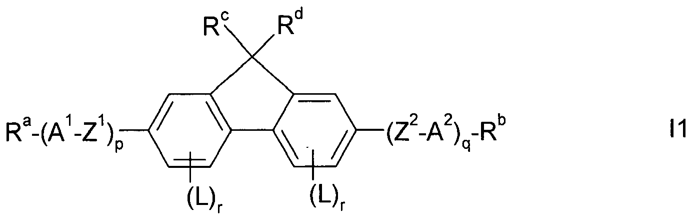 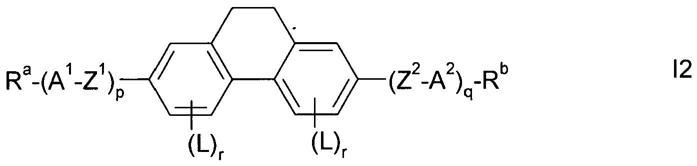 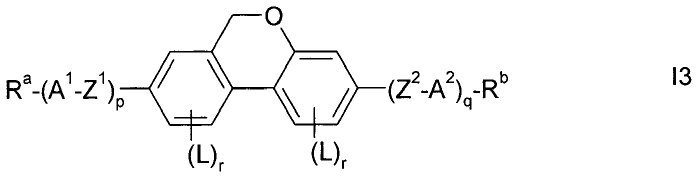 in which the individual radicals have the meanings indicated in Claim 1.

5. Use according to one or more of Claims 1 to 4, **characterised in that** the compounds of the formula I are selected from the group consisting of the following sub-formulae: 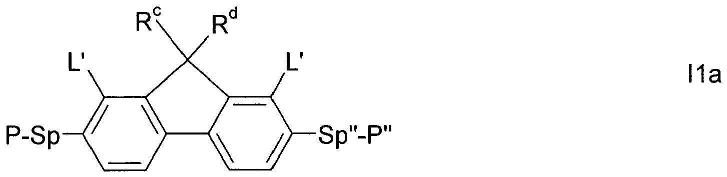 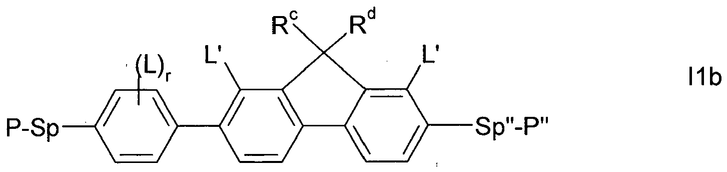 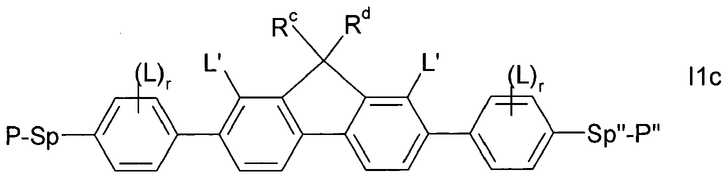 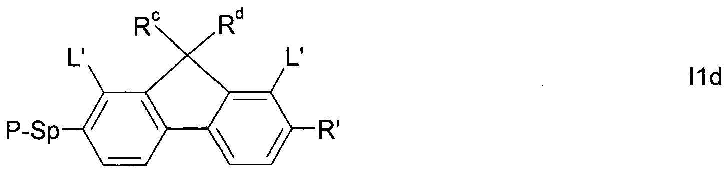 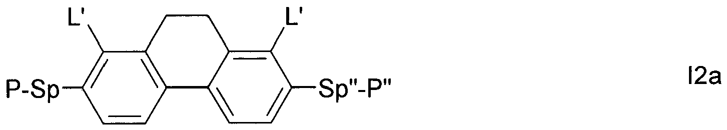 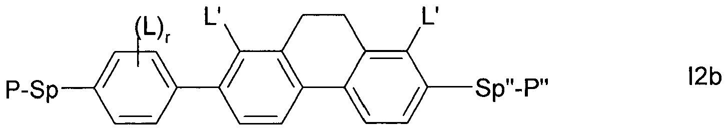 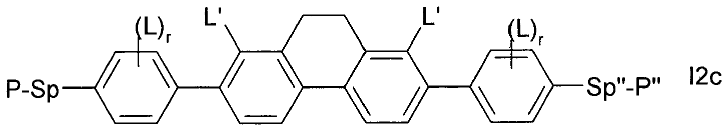 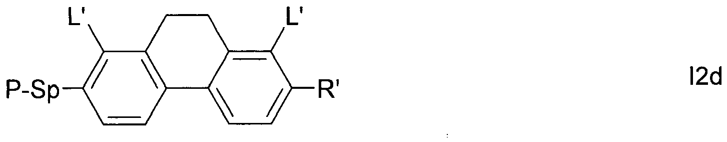 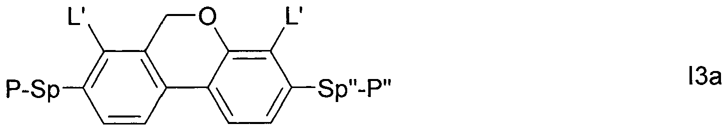 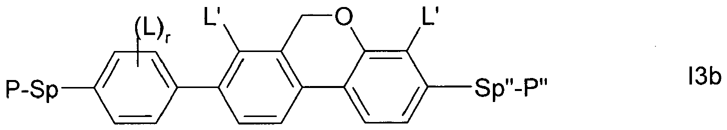 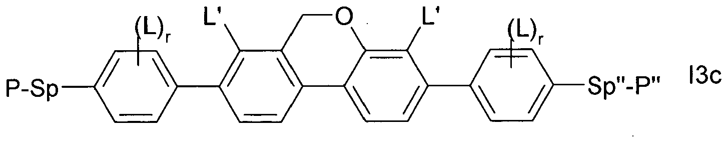 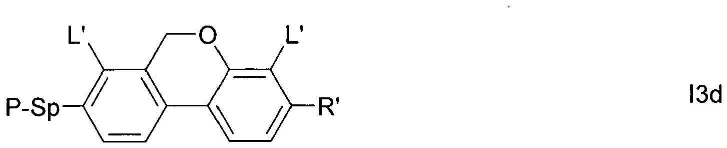 in which L' denotes H or F, P and Sp have one of the meanings indicated in Claim 1, P" has one of the meanings indicated for P in Claim 1, Sp" has one of the meanings indicated for Sp in Claim 1, and R' has one of the meanings indicated for R^{a} in Claim 1, where R' is other than H and does not denote or contain a group P-Sp-.

6. Use of compounds according to one or more of Claims 1 to 5 in LC displays of the PS or PSA type containing an LC cell having two substrates, where at least one substrate is transparent to light and at least one substrate has one or two electrodes, and a layer, located between the substrates, of an LC medium comprising a polymerised component and a low-molecular-weight component, where the polymerised component is obtainable by polymerisation of one or more polymerisable compounds between the substrates of the LC cell in the LC medium with application of an electrical voltage to the electrodes, where at least one of the polymerisable compounds is selected from formula I as defined in one or more of Claims 1 to 5.

7. Use according to one or more of Claims 1 to 6, **characterised in that** the LC display contains an LC medium which comprises one or more compounds of the formulae I and one or more compounds of the formulae CY and/or PY: 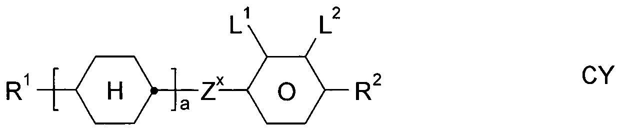 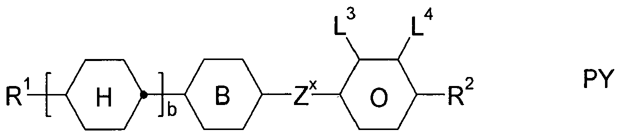 in which the individual radicals have the following meanings:
a denotes 1 or 2,
b denotes 0 or 1, 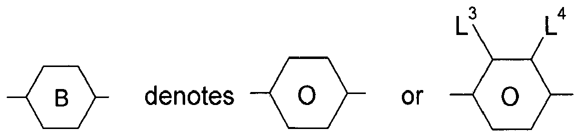
R¹ and R² each, independently of one another, denote alkyl having 1 to 12 C atoms, where, in addition, one or two non-adjacent CH₂ groups may be replaced by -O-, -CH=CH-, -CO-, -O-CO- or -CO-O- in such a way that O atoms are not linked directly to one another,
Z^{x} denotes -CH=CH-, -CH₂O-, -OCH₂-, -CF₂O-, -OCF₂-, -O-, -CH₂-, -CH₂CH₂- or a single bond, preferably a single bond,
L¹⁻⁴ each, independently of one another, denote F, Cl, OCF₃, CF₃, CH₃, CH₂F, CHF₂.

8. Use according to one or more of Claims 1 to 7, **characterised in that** the LC medium comprises one or more compounds of the following formula: 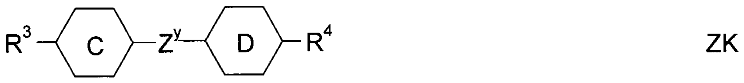 in which the individual radicals have the following meanings: 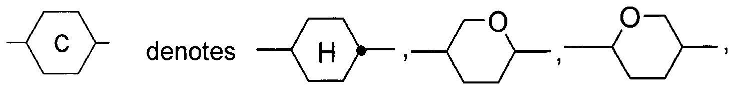 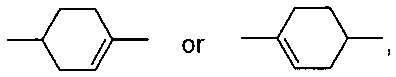 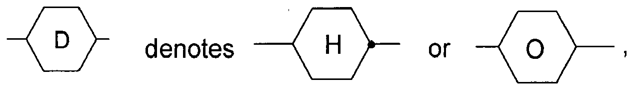
R³ and R⁴ each, independently of one another, denote alkyl having 1 to 12 C atoms, in which, in addition, one or two non-adjacent CH₂ groups may be replaced by -O-, -CH=CH-, -CO-, -O-CO- or -CO-O- in such a way that O atoms are not linked directly to one another,
Z^{y} denotes -CH₂CH₂-, -CH=CH-, -CF₂O-, -OCF₂-, -CH₂O-, -OCH₂-, -COO-, -OCO-, -C₂F₄-, -CF=CF- or a single bond.

9. LC display of the PS or PSA type as defined in one or more of Claims 1 to 8.

10. LC display according to Claim 9, **characterised in that** it is a PSA-VA, PSA-OCB, PS-IPS, PS-FFS or PS-TN display.

11. Process for the production of an LC display of the PS or PSA type, in which an LC medium as defined im one or more of Claims 6 to 8 is introduced into an LC cell having two substrates, where at least one substrate is transparent to light and at least one substrate has one or two electrodes, and the polymerisable compounds are polymerised with application of an electrical voltage to the electrodes.

12. Compounds of the formula I1 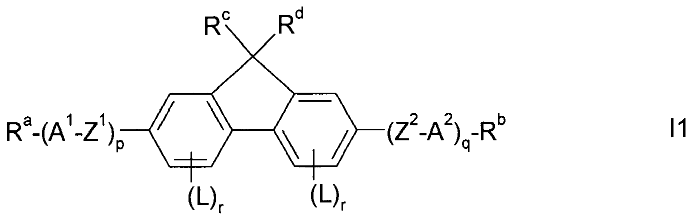 in which R^{c,d}, A^{1,2}, Z^{1,2}, L, p, q and r have the meaning indicated in Claim 1 or 2, and R^{a} and R^{b} denote P-Sp-,
where Sp in one of the radicals R^{a} and R^{b} denotes a single bond and Sp in the other of the radicals R^{a} and R^{b} denotes a group of the formula Sp'-X', so that this radical P-Sp- conforms to the formula P-Sp'-X'-,
Sp' denotes alkylene having 1 to 20 C atoms, which is optionally mono- or polysubstituted by F, Cl, Br, I or CN, and in which, in addition, one or more non-adjacent CH₂ groups may each be replaced, independently of one another, by -O-, -S-, -NH-, -N(R⁰)-, -Si(R⁰⁰R⁰⁰⁰)-, -CO-, -CO-O-, -O-CO-, -O-CO-O-, -S-CO-, -CO-S-, -N(R⁰⁰)-CO-O-, -O-CO-N(R⁰⁰)-, -N(R⁰⁰)-CO-N(R⁰⁰)-, -CH=CH- or -C≡C- in such a way that O and/or S atoms are not linked directly to one another,
X' denotes -O-, -S-, -CO-, -CO-O-, -O-CO-, -O-CO-O-, -CO-N(R⁰⁰)-, -N(R⁰⁰)-CO-, -N(R⁰⁰)-CO-N(R⁰⁰-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=CY³-, -C≡C-, -CH=CH-CO-O-, -O-CO-CH=CH- or a single bond,
R⁰⁰ R⁰⁰⁰ each, independently of one another, denote H or alkyl having 1 to 12 C atoms,
Y², Y³ each, independently of one another, denote H, F, Cl or CN,
P is on each occurrence, identically or differently, selected from CH₂=CW¹-CO-O-, CH₂=CW¹-CO-, 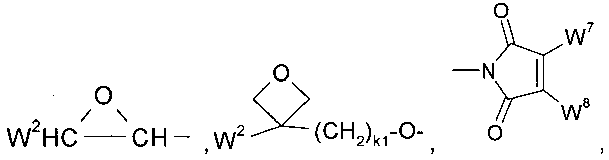 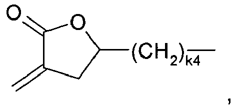 CH₂=CW²-O-, CW¹=CH-CO-(O)ₖ₃-, CW¹=CH-CO-NH-, CH₂=CW¹-CO-NH-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, CH₂=CW¹-CO-NH-, CH₂=CH-(COO)ₖ₁-Phe-(O)ₖ₂-, CH₂=CH-(CO)ₖ₁-Phe-(O)ₖ₂- and W⁴W⁵W⁶Si-, in which W¹ denotes H, F, Cl, CN, CF₃, phenyl or alkyl having 1 to 5 C atoms, W² and W³ each, independently of one another, denote H or alkyl having 1 to 5 C atoms, W⁴, W⁵ and W⁶ each, independently of one another, denote Cl, oxaalkyl or oxacarbonylalkyl having 1 to 5 C atoms, W⁷ and W⁸ each, independently of one another, denote H, Cl or alkyl having 1 to 5 C atoms, Phe denotes 1,4-phenylene, k₁, k₂ and k₃ each, independently of one another, denote 0 or 1, and k₄ denotes an integer from 1 to 10.

13. Compounds of the formula I2 or I3, 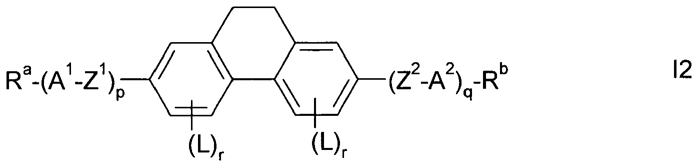 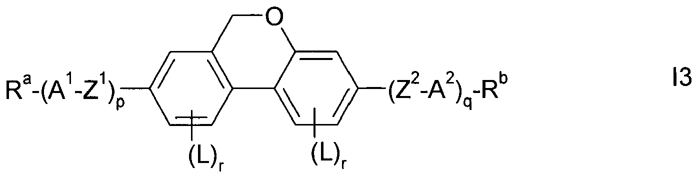 in which the individual radicals have the meanings indicated in Claim 1.

14. Compounds according to Claim 12 or 13, selected from the group consisting of the following sub-formulae: 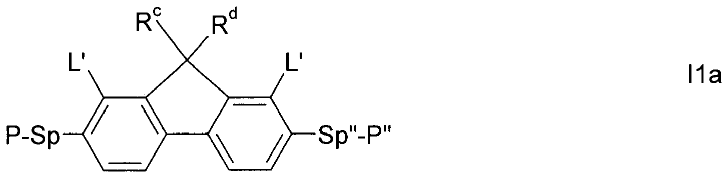 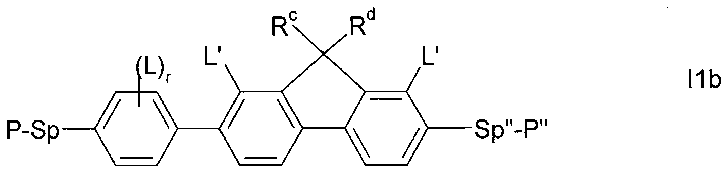 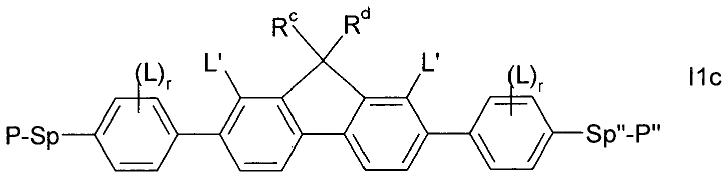 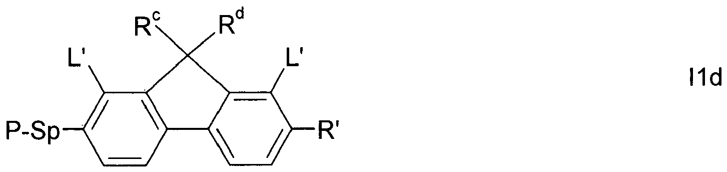 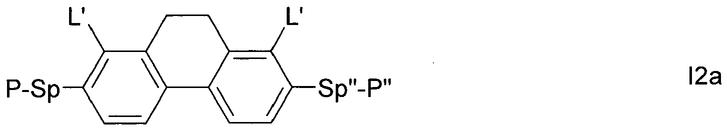 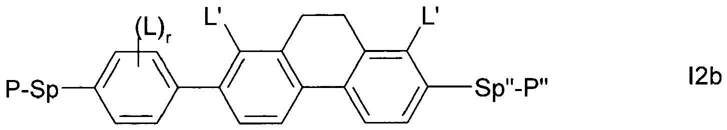 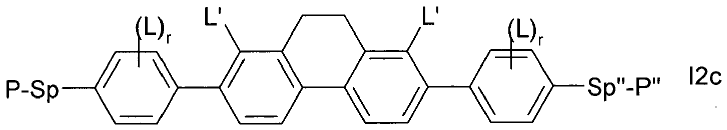 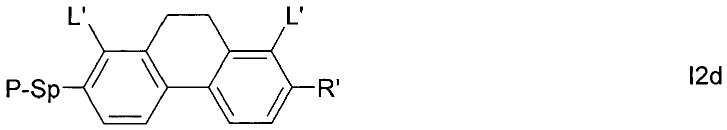 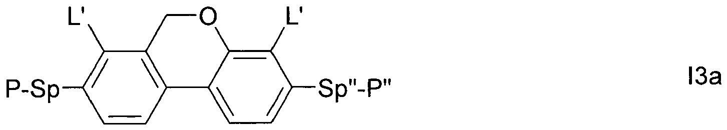 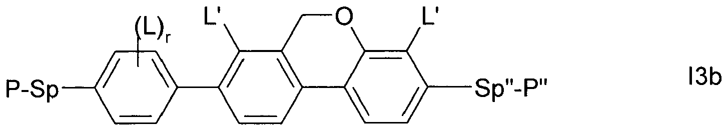 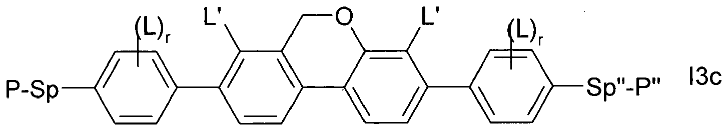 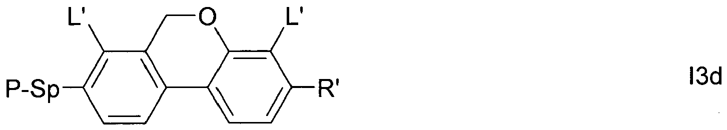 in which L' denotes H or F, P and Sp have one of the meanings indicated in Claim 12, P" has one of the meanings indicated for P, Sp" has one of the meanings indicated for Sp, and R' has one of the meanings indicated for R^{a} in Claim 12, where R' is other than H and does not denote or contain a group P-Sp-.

15. Compounds according to one or more of Claims 12 to 14, **characterised in that** P and P" denote an acrylate, fluoroacrylate or methacrylate group.

16. Compounds according to one or more of Claims 12 to 15, **characterised in that** Sp and Sp" are selected from -(CH₂)ₚ₁-, -O-(CH₂)ₚ₁-, -(CH₂)ₚ₁-O-, -O-CO-(CH₂)ₚ₁-, -(CH₂)ₚ₁-O-CO-, -O-CO-O-(CH₂)ₚ₁-, -(CH₂)ₚ₁-O-CO-O-, -(CH₂)ᵣ₁-C≡C- or -C≡C-(CH₂)ᵣ₁- or a single bond, in which p1 denotes an integer from 1 to 12, preferably from 1 to 6, and r1 denotes an integer from 1 to 8, preferably 1, 2 or 3, where these groups are linked to P or P" in such a way that O atoms are not linked directly to one another.

17. LC medium comprising one or more compounds of the formula I1, I2 or I3 or sub-formulae thereof according to one or more of Claims 12 to 16.

18. LC medium according to Claim 17, comprising
- a polymerisable component A) comprising one or more polymerisable compounds, and
- a liquid-crystalline component B) comprising one or more low-molecular-weight compounds,
**characterised in that** component A) comprises one or more polymerisable compounds of the formula I according to one or more of Claims 12 to 16.

19. LC medium according to Claim 18, in which component B) comprises one or more compounds selected from the formulae CY, PY and ZK according to Claim 7 or 8.

20. Compounds selected from the group consisting of the following sub-formulae: 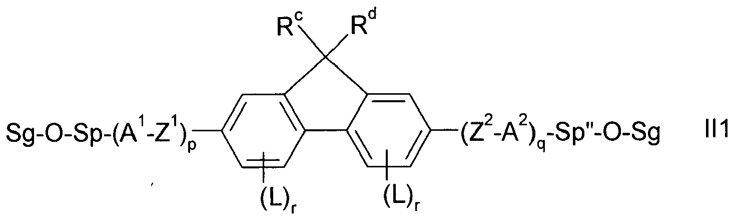 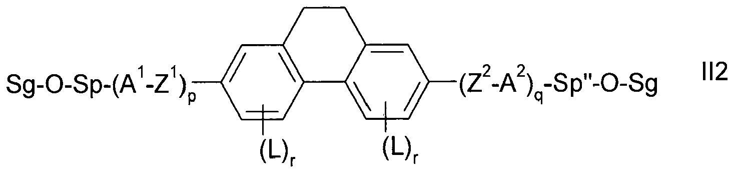 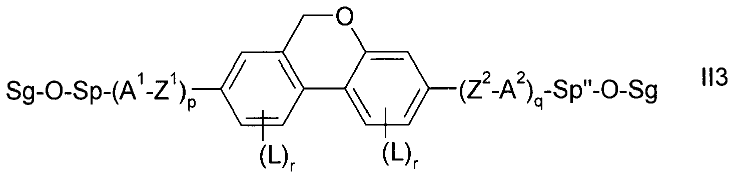 in which R^{c,d}, A^{1,2}, Z^{1,2}, L, p, q, r, Sp and Sp" have the meaning indicated in Claims 1, 4 and 14, where, in formula II1, Sp denotes a single bond and Sp" is other than a single bond, and Sg denotes an H atom or a protecting group.

21. Compounds of the formula III 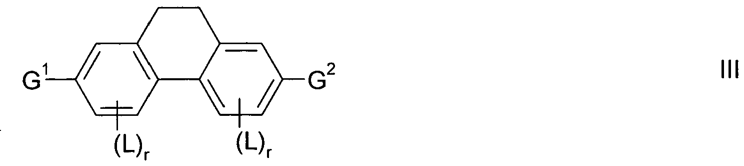 in which L and r have the meanings indicated in Claim 1, and one of the groups G¹ and G² denotes I and the other denotes Br or OH.

22. Process for the preparation of a compound of the formula I or sub-formulae thereof according to one of Claims 12 to 16, in which a compound according to Claim 20 is esterified or etherified using corresponding acids, acid derivatives, or halogenated compounds containing a group P, in the presence of a dehydrating reagent.

## Revendications

1. Utilisation de composés de la formule I 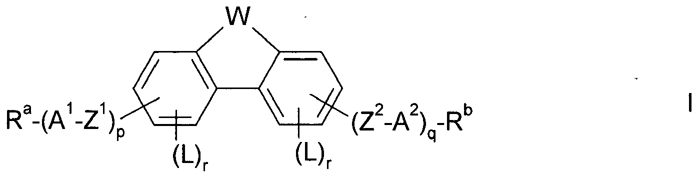 dans laquelle les radicaux individuels présentent les significations qui suivent:
W représente -C(R^{c}R^{d})-, -CH₂CH₂-, -CH₂-O-, -O-, -CO-, -CO-O-, -S- ou -N(R⁰)-,
R^{c}, R^{d} représentent, chacun indépendamment de l'autre, P-Sp-, H, F, CI, Br, I, -CN, -NO₂, -NCO, -NCS, -OCN, -SCN, SF₅, alkyle en chaîne droit ou ramifié comportant de 1 à 25 atomes de C, où, en outre, un ou plusieurs groupes CH₂ non adjacents peuvent chacun être remplacés, indépendamment des autres, par arylène, -C(R⁰)=C(R⁰⁰-, -C≡C-, -N(R⁰)- -O-, -S-, -CO-, -CO-O-, -O-CO-, -O-CO-O- de telle sorte que des atomes de O et/ou de S ne soient pas liés directement les uns aux autres, et où, en outre, un ou plusieurs atomes de H peut/peuvent être remplacé(s) par F, CI, Br, I, CN ou P-Sp-, ou aryle ou hétéroaryle, de préférence comportant de 2 à 25 atomes de C, lequel peut également contenir deux cycles fusionnés ou plus et lequel est en option mono- ou polysubstitué par L,
R^{a}, R^{b} présentent, chacun indépendamment de l'autre, l'une des significations indiquées pour R^{c}, où au moins l'un des radicaux R^{a} et R^{b} représente ou contient un groupe P-Sp-,
P représente, pour chaque occurrence, de manière identique ou différente, un groupe polymérisable,
Sp représente, pour chaque occurrence, de manière identique ou différente, un groupe d'espaceur ou une liaison simple,
A¹ et A² représentent, chacun indépendamment de l'autre, 1,4-phénylène, naphtalène-1 ,4-diyle ou naphtalène-2,6-diyle, où, en outre, un ou plusieurs groupes CH parmi ces groupes peut/peuvent être remplacé(s) par N, cyclohexane-1,4-diyle, où, en outre, un ou plusieurs groupes CH₂ non adjacents peut/peuvent être remplacé(s) par O et/ou S, 1,4-cyclohexénylène, bicyclo[1.1.1]pentane-1,3-diyle, bicyclo[2.2.2]octane-1,4-diyle, spiro[3.3]heptane-2,6-diyle, pipéridine-1,4-diyle, décahydronaphtalène-2,6-diyle, 1,2,3,4-tétrahydronaphtalène-2,6-diyle, indane-2,5-diyle, octahydro-4,7-méthanoindane-2,5-diyle, phénanthrène-2,7-diyle ou anthracène-2,7-diyle, où tous ces groupes peuvent être non substitués ou mono- ou polysubstitués par L,
L représente P, P-Sp-, OH, CH₂OH, F, Cl, Br, I, -CN, -NO₂, -NCO, -NCS, -OCN, -SCN, -C(=O)N(R^{x})₂, -C(=O)Y¹, -C(=O)R^{x}, -N(R^{x})₂, silyle en option substitué, aryle en option substitué comportant de 6 à 20 atomes de C, alkyle ou alcoxy en chaîne droite ou ramifié comportant de 1 à 25 atomes de C, ou alkényle, alkynyle, alkylcarbonyle, alcoxycarbonyle, alkylcarbonyloxy ou alcoxycarbonyloxy en chaîne droite ou ramifié comportant de 2 à 25 atomes de C, où, en outre, un ou plusieurs atomes de H dans tous ces groupes peut/peuvent être remplacé(s) par F, Cl, P ou P-Sp-,
Y¹ représente halogène,
R^{x} représente P, P-Sp-, H, halogène, alkyle en chaîne droite, ramifié ou cyclique comportant de 1 to 25 atomes de C, où, en outre, un ou plusieurs groupes CH₂ non adjacents peut/peuvent être remplacé(s) par -O-, -S-, -CO-, -CO-O-, -O-CO-, -O-CO-O- de telle sorte que des atomes de O et/ou de S ne soient pas liés directement les uns aux autres, et où, en outre, un ou plusieurs atomes de H peut/peuvent être remplacé(s) par F, Cl ou P-Sp-,
Z¹ Z² représentent, chacun indépendamment de l'autre, -O-, -S-, -CO-, -CO-O-, -O-CO-, -O-CO-O-, -OCH₂-, -CH₂O-, -SCH₂-, -CH₂S-, -CF₂O-, -OCF₂-, -CF₂S-, -SCF₂-, -(CH₂)ₙ-, -CF₂CH₂-, -CH₂CF₂-, -(CF₂)ₙ-, -CH=CH-, -CF=CF-, -CH=CF-, -CF=CH-, -C≡C-, -CH=CH-CO-O-, -O-CO-CH=CH-, -CH₂-CH₂-CO-O-, -O-CO-CH₂-CH₂-, -C(R⁰R⁰⁰)-, -C(R^{y}R^{z})- ou une liaison simple,
R⁰, R⁰⁰ représentent, chacun indépendamment de l'autre et de manière identique ou différente pour chaque occurrence, H ou alkyle comportant de 1 à 12 atomes de C,
R^{y}, R^{z} représentent, chacun indépendamment l'autre, H, F, CH₃ ou CF₃,
n représente, pour chaque occurrence, de manière identique ou différente, 1, 2, 3 ou 4,
p, q représentent, chacun indépendamment de l'autre, 0, 1, 2 ou 3,
r représente, pour chaque occurrence, de manière identique ou différente 0, 1, 2 ou 3,
dans des affichages LC du type PS (polymère stabilisé) ou PSA (alignement prolongé par polymère).

2. Utilisation selon la revendication 1, **caractérisée en ce que** R^{a} et R^{b} dans la formule I représentent des radicaux identiques ou différents P-Sp-.

3. Utilisation selon la revendication 1 ou 2, **caractérisée en ce que** les composés de la formule I sont choisis parmi la formule IA 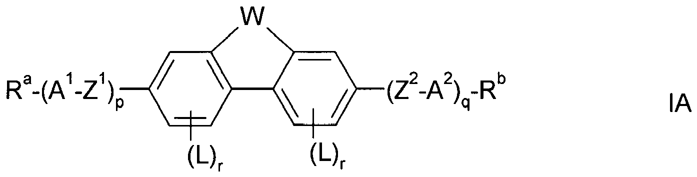 dans laquelle W, R^{a,b}, A^{1,2}, Z^{1,2}, L, p, q et r présentent la signification indiquée selon la revendication 1 ou 2.

4. Utilisation selon une ou plusieurs des revendications 1 à 3, **caractérisée en ce que** les composés de la formule I sont choisis parmi le groupe constitué par les sous-formules qui suivent : 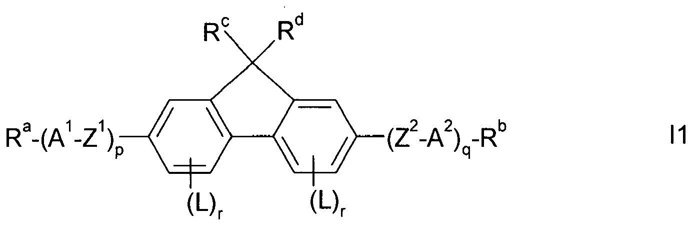 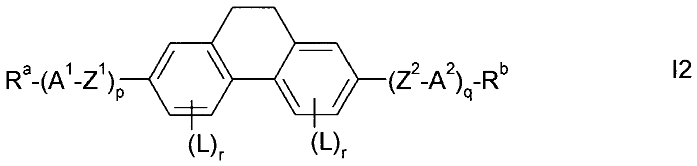 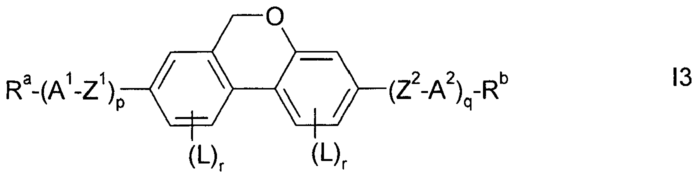 dans lesquelles les radicaux individuels présentent les significations indiquées selon la revendication 1.

5. Utilisation selon une ou plusieurs des revendications 1 à 4, **caractérisée en ce que** les composés de la formule I sont choisis parmi le groupe constitué par les sous-formules qui suivent : 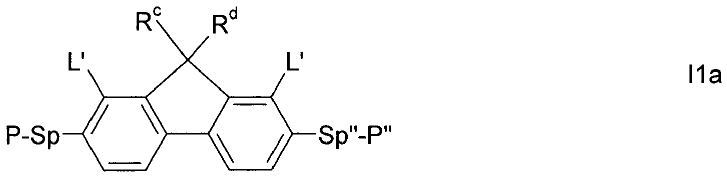 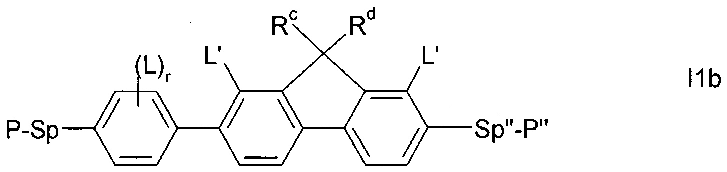 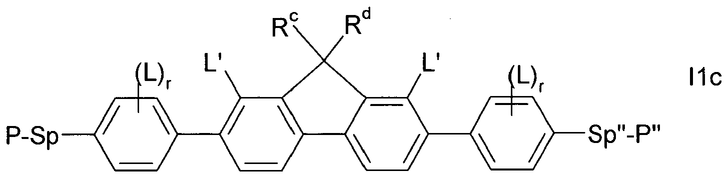 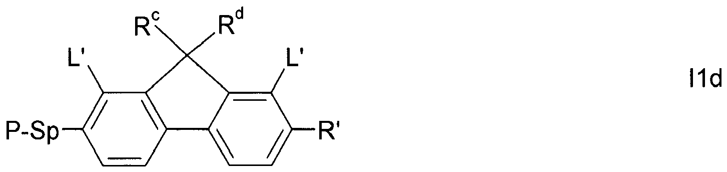 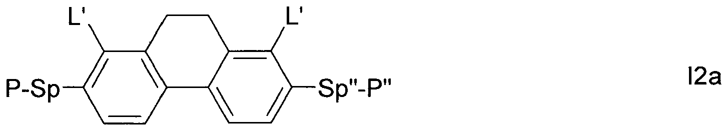 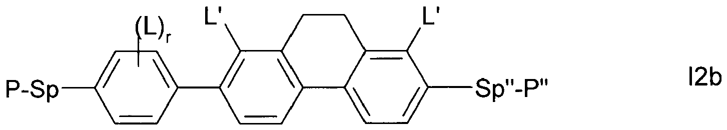 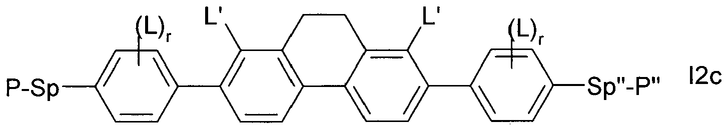 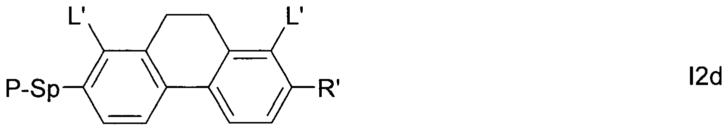 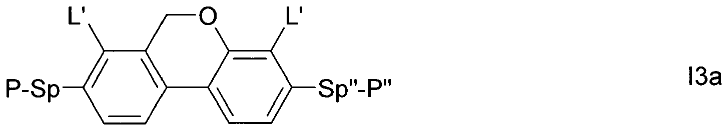 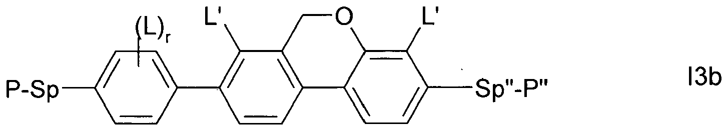 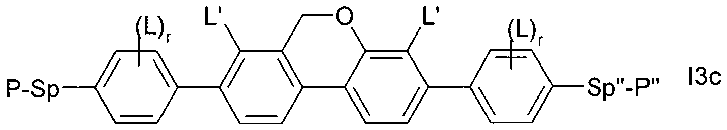 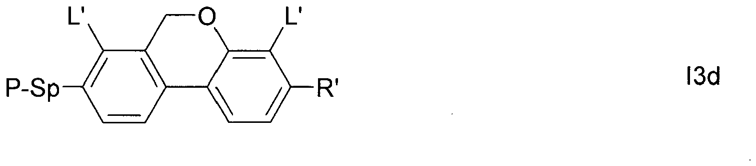 dans lesquelles L' représente H ou F, P et Sp présentent l'une des significations indiquées selon la revendication 1, P" présente l'une des significations indiquées pour P selon la revendication 1, Sp" présente l'une des significations indiquées pour Sp selon la revendication 1, et R' présente l'une des significations indiquées pour R^{a} selon la revendication 1, où R' est autre que H et ni ne représente, ni ne contient un groupe P-Sp-.

6. Utilisation de composés selon une ou plusieurs des revendications 1 à 5 dans des affichages LC du type PS ou PSA contenant une cellule LC comportant deux substrats, où au moins un substrat est transparent vis-à-vis de la lumière et au moins un substrat comporte une ou deux électrodes, et une couche, localisée entre les substrats, d'un milieu LC comprenant un composant polymérisé et un composant de poids moléculaire faible, où le composant polymérisé peut être obtenu par polymérisation d'un ou de plusieurs composés polymérisables entre les substrats de la cellule LC dans le milieu LC en appliquant une tension électrique entre les électrodes, où au moins l'un des composés polymérisables est choisi parmi la formule I comme défini selon une ou plusieurs des revendications 1 à 5.

7. Utilisation selon une ou plusieurs des revendications 1 à 6, **caractérisée en ce que** l'affichage LC contient un milieu LC qui comprend un ou plusieurs composés des formules I et un ou plusieurs composés des formules CY et/ou PY : 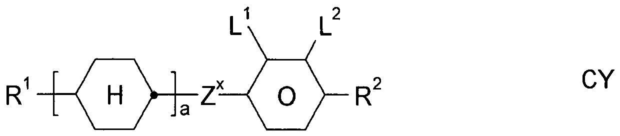 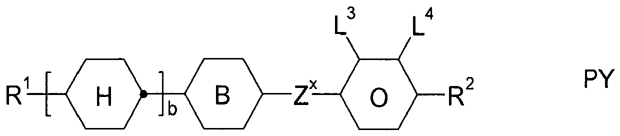 dans lesquelles les radicaux individuels présentent les significations qui suivent :
a représente 1 ou 2,
b représente 0 ou 1, 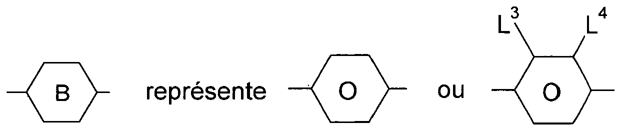
R¹ et R² représentent, chacun indépendamment de l'autre, alkyle comportant de 1 à 12 atomes de C, où, en outre, un ou deux groupes CH₂ non adjacents peut/peuvent être remplacé(s) par -O-, -CH=CH-, -CO-, -O-CO- ou -CO-O- de telle sorte que des atomes de O ne soient pas liés directement les uns aux autres,
Z^{x} représente -CH=CH-, -CH₂O-, -OCH₂-, -CF₂O-, -OCF₂-, -O-, -CH₂-, -CH₂CH₂- ou une liaison simple, de préférence une liaison simple,
L¹⁻⁴ représentent, chacun indépendamment des autres, F, Cl, OCF₃, CF₃, CH₃, CH₂F, CHF₂.

8. Utilisation selon une ou plusieurs des revendications 1 à 7, **caractérisée en ce que** le milieu LC comprend un ou plusieurs composés de la formule qui suit : 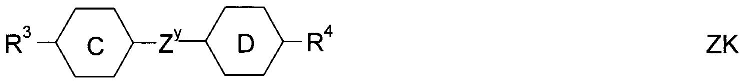 dans laquelle les radicaux individuels présentent les significations qui suivent : 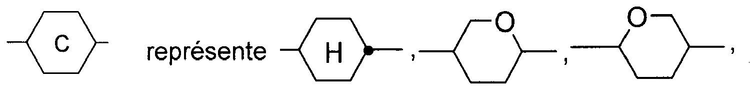 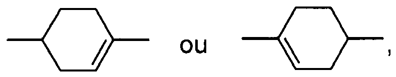 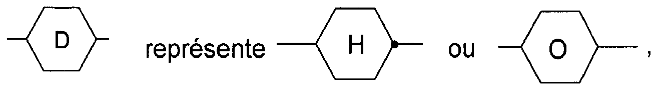
R³ et R⁴ représentent, chacun indépendamment de l'autre, alkyle comportant de 1 à 12 atomes de C, où, en outre, un ou deux groupes CH₂ non adjacents peut/peuvent être remplacé(s) par -O-, -CH=CH-, -CO-, -O-CO- ou -CO-O- de telle sorte que des atomes de O ne soient pas liés directement les uns aux autres,
Z^{y} représente -CH₂CH₂-, -CH=CH-, -CF₂O-, -OCF₂-, -CH₂O-, -OCH₂-, -COO-, -OCO-, -C₂F₄-, -CF=CF- ou une liaison simple.

9. Affichage LC du type PS ou PSA comme défini selon une ou plusieurs des revendications 1 à 8.

10. Affichage LC selon la revendication 9, **caractérisé en ce qu'**il s'agit d'un affichage PSA-VA, PSA-OCB, PS-IPS, PS-FFS ou PS-TN.

11. Procédé pour la fabrication d'un affichage LC du type PS ou PSA, dans lequel un milieu LC comme défini selon une ou plusieurs des revendications 6 à 8 est introduit à l'intérieur d'une cellule LC comportant deux substrats, où au moins un substrat est transparent vis-à-vis de la lumière et au moins un substrat comporte une ou deux électrodes et les composés polymérisables sont polymérisés en appliquant une tension électrique entre les électrodes.

12. Composés de la formule I1 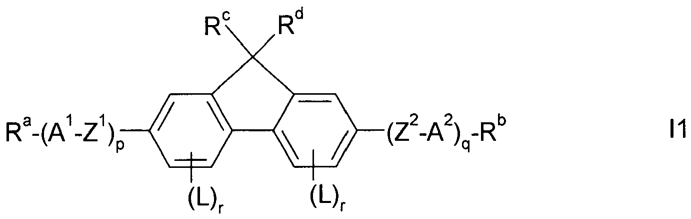 dans laquelle R^{c,d}, A^{1,2}, Z^{1,2}, L, p, q et r présentent la signification indiquée selon la revendication 1 ou 2, et R^{a} et R^{b} représentent P-Sp-,
où Sp dans l'un des radicaux R^{a} et R^{b} représente une liaison simple et Sp dans l'autre des radicaux R^{a} et R^{b} représente un groupe de la formule Sp'-X', de telle sorte que ce radical P-Sp- soit conforme à la formule P-Sp'-X'-,
Sp' représente alkylène comportant de 1 à 20 atomes de C, lequel est en option mono- ou polysubstitué par F, CI, Br, I ou CN, et où, en outre, un ou plusieurs groupes CH₂ non adjacents peuvent chacun être remplacés, indépendamment des autres, par -O-, -S-, -NH-, -N(R⁰)-, -Si(R⁰⁰R⁰⁰⁰)-, -CO-, -CO-O-, -O-CO-, -O-CO-O-, -S-CO-, -CO-S-, -N(R⁰⁰)-CO-O-, -O-CO-N(R⁰⁰)-, -N(R⁰⁰)-CO-N(R⁰⁰)-, -CH=CH- ou -C≡C- de telle sorte que des atomes de O et/ou de S ne soient pas liés directement les uns aux autres,
X' représente -O-, -S-, -CO-, -CO-O-, -O-CO-, -O-CO-O-, -CO-N(R⁰⁰)-, -N(R⁰⁰)-CO-, -N(R⁰⁰)-CO-N(R⁰⁰)-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=CY³-, -C≡C-, -CH=CH-CO-O-, -O-CO-CH=CH- ou une liaison simple,
R⁰⁰, R⁰⁰⁰ représentent, chacun indépendamment de l'autre, H ou alkyle comportant de 1 à 12 atomes de C,
Y², Y³ représentent, chacun indépendamment de l'autre, H, F, CI ou CN,
P est choisi, pour chaque occurrence, de manière identique ou différente, parmi CH₂=CW¹-CO-O-, CH₂=CW¹-CO-, 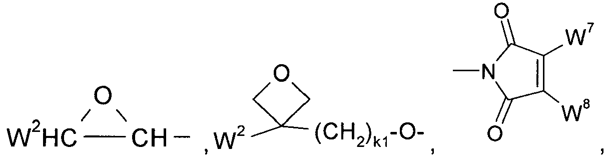 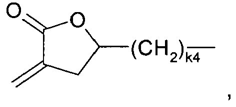
CH₂=CW²-O-, CW¹=CH-CO-(O)ₖ₃-, CW¹=CH-CO-NH-, CH₂=CW¹-CO-NH-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, CH₂=CW¹-CO-NH-, CH₂=CH-(COO)ₖ₁-Phe-(O)ₖ₂-, CH₂=CH-(CO)ₖ₁-Phe-(O)ₖ₂- et W⁴W⁵W⁶Si-, où W¹ représente H, F, CI, CN, CF₃, phényle ou alkyle comportant de 1 à 5 atomes de C, W² et W³ représentent, chacun indépendamment de l'autre, H ou alkyle comportant de 1 à 5 atomes de C, W⁴, W⁵ et W⁶ représentent, chacun indépendamment des autres, CI, oxaalkyle ou oxacarbonylalkyle comportant de 1 à 5 atomes de C, W⁷ et W⁸ représentent, chacun indépendamment de l'autre, H, Cl ou alkyle comportant de 1 à 5 atomes de C, Phe représente 1,4-phénylène, k₁, k₂ et k₃ représentent, chacun indépendamment des autres, 0 ou 1, et k₄ représente un entier de 1 à 10.

13. Composés de la formule I2 ou I3, 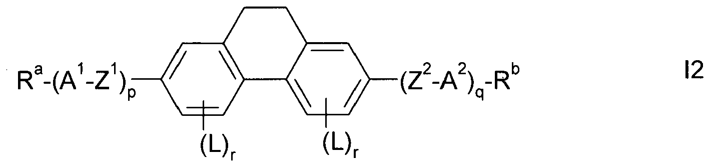 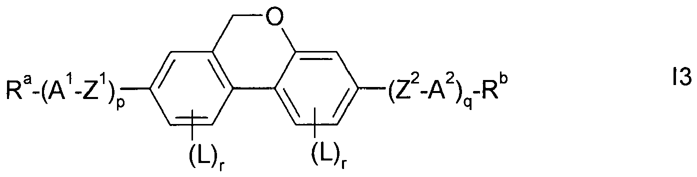 dans lesquelles les radicaux individuels présentent les significations indiquées selon la revendication 1.

14. Composés selon la revendication 12 ou 13, choisis parmi le groupe constitué par les sous-formules qui suivent : 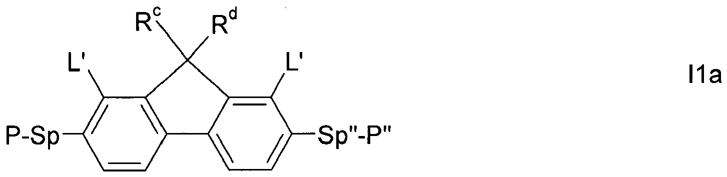 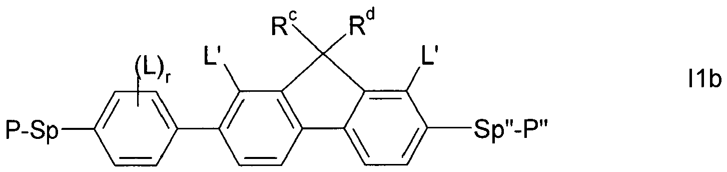 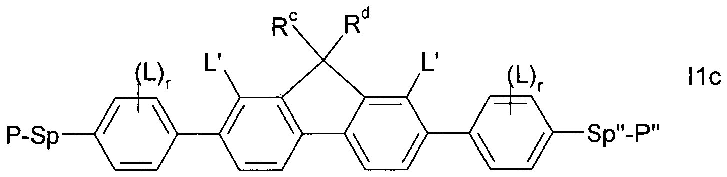 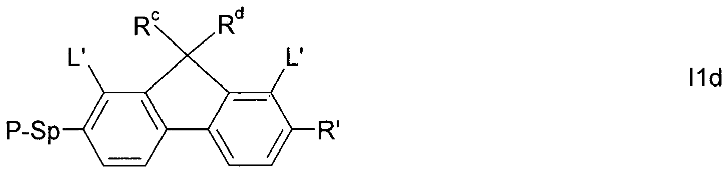 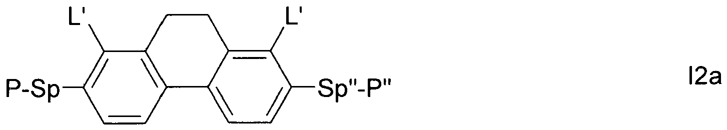 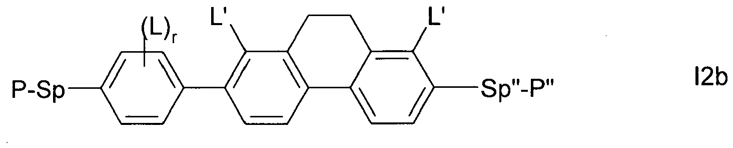 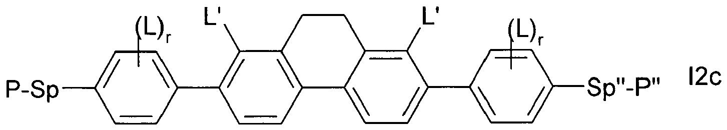 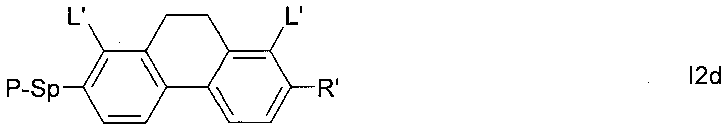 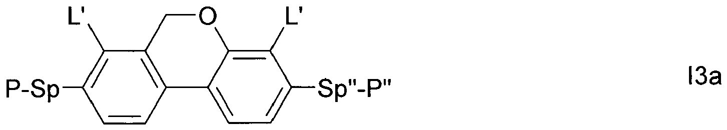 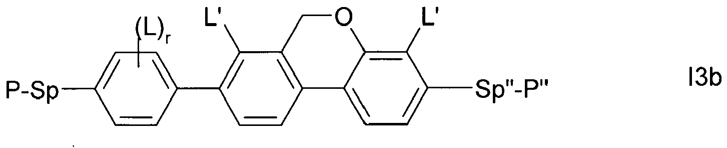 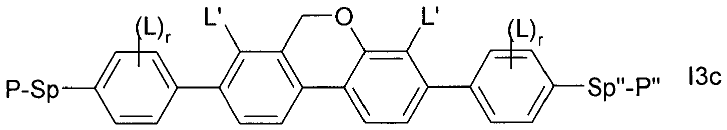 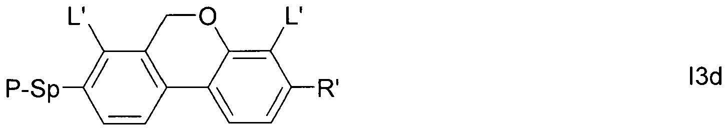 dans lesquelles L' représente H ou F, P et Sp présentent l'une des significations indiquées selon la revendication 12, P" présente l'une des significations indiquées pour P, Sp" présente l'une des significations indiquées pour Sp, et R' présente l'une des significations indiquées pour R^{a} selon la revendication 12, où R' est autre que H et ni ne représente, ni ne contient un groupe P-Sp-.

15. Composés selon une ou plusieurs des revendications 12 à 14, **caractérisés en ce que** P et P" représentent un groupe acrylate, fluoroacrylate ou méthacrylate.

16. Composés selon une ou plusieurs des revendications 12 à 15, **caractérisés en ce que** Sp et Sp" sont choisis parmi -(CH₂)ₚ₁-, -O-(CH₂)ₚ₁-, -(CH₂)ₚ₁-O-, -O-CO-(CH₂)ₚ₁-, -(CH₂)ₚ₁-O-CO-, -O-CO-O-(CH₂)ₚ₁-, -(CH₂)ₚ₁-O-CO-O-, -(CH₂)ᵣ₁-C≡C- ou -C≡C-(CH₂)ᵣ₁- ou une liaison simple, où p1 représente un entier de 1 à 12, de préférence de 1 à 6, et r1 représente un entier de 1 à 8, de préférence 1, 2 ou 3, où ces groupes sont liés à P ou à P" de telle sorte que des atomes de O ne soient pas liés directement les uns aux autres.

17. Milieu LC comprenant un ou plusieurs composé(s) de la formule I1, I2 ou I3 ou de leurs sous-formules selon une ou plusieurs des revendications 12 à 16.

18. Milieu LC selon la revendication 17, comprenant :
- un composant polymérisable A) comprenant un ou plusieurs composés polymérisables, et
- un composant cristallin liquide B) comprenant un ou plusieurs composés de poids moléculaire faible,
**caractérisé en ce que** le composant A) comprend un ou plusieurs composés polymérisables de la formule I selon une ou plusieurs des revendications 12 à 16.

19. Milieu LC selon la revendication 18, dans lequel un composant B) comprend un ou plusieurs composés choisis parmi les formules CY, PY et ZK selon la revendication 7 ou 8.

20. Composés choisis parmi le groupe constitué par les sous-formules qui suivent : 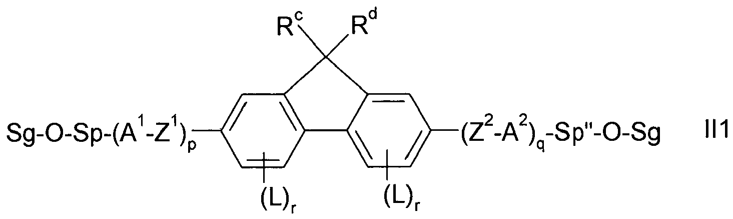 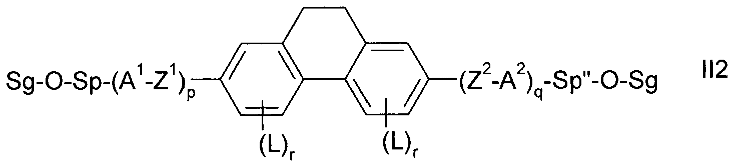 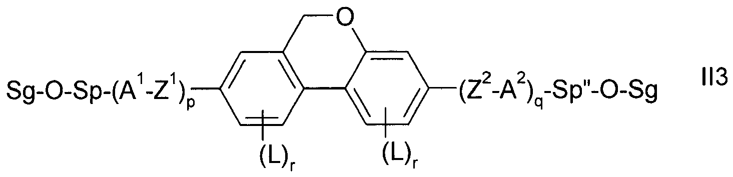 dans lesquelles R^{c,d}, A^{1,2}, Z^{1,2}, L, p, q, r, Sp et Sp" présentent la signification indiquée selon les revendications 1, 4 et 14, où, dans la formule II1, Sp représente une liaison simple et Sp" est autre qu'une liaison simple, et Sg représente un atome de H ou un groupe de protection.

21. Composés de la formule III 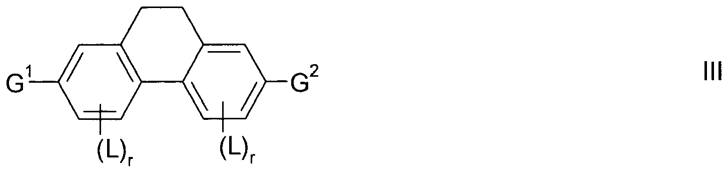 dans laquelle L et r présentent les significations indiquées selon la revendication 1, et l'un des groupes G¹ et G² représente I et l'autre représente Br ou OH.

22. Procédé pour la préparation d'un composé de la formule I ou de ses sous-formules selon l'une des revendications 12 à 16, dans lequel un composé selon la revendication 20 est estérifié ou éthérifié en utilisant des acides correspondants, des dérivés d'acide correspondants, ou des composés halogénés correspondants contenant un groupe P, en présence d'un réactif déshydratant.
